# EUROPÄISCHE PATENTSCHRIFT

(11) **EP 2 730 658 B1**
(45) Veröffentlichungstag und Bekanntmachung des Hinweises auf die Patenterteilung: **10.04.2019**
(21) Anmeldenummer: 13192425.0
(22) Anmeldetag: 12.11.2013
(51) Int. Cl.: C12P 7/62, C12P 7/64, C12N 15/52, C12N 9/02, C12N 9/18

(54) **Verfahren zur Umsetzung eines Carbonsäureesters unter Verwendung BioH-defizienter Zellen**
Process for converting a carboxylic acid ester employing BioH-deficient cells
Procédé de conversion d'un ester d'acide carboxylique en utilisant des cellules déficientes en BioH

(30) Priorität: 12.11.2012 EP 12007663
(43) Veröffentlichungstag der Anmeldung: 14.05.2014
(73) Patentinhaber: Evonik Degussa GmbH, 45128 Essen (DE)
(72) Erfinder: Schaffer, Steffen, 45699 Herten (DE); Andrea, Heiko, 45768 Marl (DE); Wessel, Mirja, 44799 Bochum (DE); Hennemann, Hans-Georg, 45770 Marl (DE); Häger, Harald, 59348 Lüdinghausen (DE)
(74) Vertreter: Evonik Patent Association

(56) Entgegenhaltungen:
- WO-A1-2009/077461
- WO-A2-2007/139871
- LIN, S. ET AL.: "Biotin Synthesis Begins by Hijacking the Fatty Acid Synthetic Pathway", NATURE CHEMICAL BIOLOGY, Bd. 6, Nr. 9, September 2010 (2010-09), Seiten 682-688, XP002699295, -& LIN, S. ET AL.: "Supplemental Information to: Biotin Synthesis Begins by Hijacking the Fatty Acid Synthetic Pathway", , 2010, XP002699296, Gefunden im Internet: URL:http://www.nature.com/nchembio/journal /v6/n9/extref/nchembio.420-S1.pdf [gefunden am 2013-06-21]
- SCHNEIKER, S. ET AL.: "cytochrome P450 family protein [Alcanivorax borkumensis SK2]", GENPEPT, 10. Juni 2013 (2013-06-10), XP002713949,
- KOK, M. ET AL.: "alkane-1 monooxygenase [Pseudomonas putida]", GENBANK, 23. Oktober 2008 (2008-10-23), XP002713950,

### SEQUENCE LISTING

<110> Evonik Industries AG
<120> Verfahren zur Umsetzung eines Carbonsäureesters
<130> 201200172
<160> 14
<170> PatentIn version 3.5
<210> 1
   <211> 771
   <212> DNA
   <213> Escherichia coli
<220>
   <221> CDS
   <222> (1)..(771)
<400> 1
   atg aat aac atc tgg tgg cag acc aaa ggt cag ggg aat gtt cat ctt          48
   Met Asn Asn Ile Trp Trp Gln Thr Lys Gly Gln Gly Asn Val His Leu
   1               5                   10                  15
   gtg ctg ctg cac gga tgg gga ctg aat gcc gaa gtg tgg cgt tgc att          96
   Val Leu Leu His Gly Trp Gly Leu Asn Ala Glu Val Trp Arg Cys Ile
               20                  25                  30
   gac gag gaa ctt agc tcg cat ttt acg ctg cac ctt gtt gac ctg ccc         144
   Asp Glu Glu Leu Ser Ser His Phe Thr Leu His Leu Val Asp Leu Pro
           35                  40                  45
   ggc ttc ggg cgt agc cgg gga ttt ggt gcg ctg tca ctt gct gat atg         192
   Gly Phe Gly Arg Ser Arg Gly Phe Gly Ala Leu Ser Leu Ala Asp Met
       50                  55                  60
   gcc gaa gcc gtg ctg caa cag gca cct gat aaa gcc att tgg tta ggc         240
   Ala Glu Ala Val Leu Gln Gln Ala Pro Asp Lys Ala Ile Trp Leu Gly
   65                  70                  75                  80
   tgg agt ctg ggc ggg ctg gtg gca agc cag att gcg tta acc cat ccc         288
   Trp Ser Leu Gly Gly Leu Val Ala Ser Gln Ile Ala Leu Thr His Pro
                   85                  90                  95
   gag cgt gtt cag gcg ctg gtc acc gtg gcg tcg tca cct tgt ttt agt         336
   Glu Arg Val Gln Ala Leu Val Thr Val Ala Ser Ser Pro Cys Phe Ser
               100                 105                 110
   gct cgt gac gag tgg ccg ggg ata aaa ccg gac gtg ctg gcg gga ttt         384
   Ala Arg Asp Glu Trp Pro Gly Ile Lys Pro Asp Val Leu Ala Gly Phe
           115                 120                 125
   cag cag caa ctc agt gat gat ttt cag cgt aca gtg gag cgg ttc ctg         432
   Gln Gln Gln Leu Ser Asp Asp Phe Gln Arg Thr Val Glu Arg Phe Leu
       130                 135                 140
   gcg tta caa acc atg ggg act gaa acg gcg cgc cag gat gcg cgg gcg         480
   Ala Leu Gln Thr Met Gly Thr Glu Thr Ala Arg Gln Asp Ala Arg Ala
   145                 150                 155                 160
   ttg aag aaa acc gtt ctg gcg tta ccg atg ccg gag gtt gac gtg ctt         528
   Leu Lys Lys Thr Val Leu Ala Leu Pro Met Pro Glu Val Asp Val Leu
                   165                 170                 175
   aat ggc ggg ctg gaa atc ctg aaa acg gtc gat ctc cgt cag ccg ctg         576
   Asn Gly Gly Leu Glu Ile Leu Lys Thr Val Asp Leu Arg Gln Pro Leu
               180                 185                 190
   caa aac gtg tcc atg ccg ttt ttg cga ttg tat ggc tat ctc gac ggt         624
   Gln Asn Val Ser Met Pro Phe Leu Arg Leu Tyr Gly Tyr Leu Asp Gly
           195                 200                 205
   ctg gtg ccg cgc aaa gtg gtg ccg atg ctg gat aaa ctt tgg cct cac         672
   Leu Val Pro Arg Lys Val Val Pro Met Leu Asp Lys Leu Trp Pro His
       210                 215                 220
   agc gaa tca tat atc ttc gcc aaa gcg gcc cat gcg cca ttt att tcg         720
   Ser Glu Ser Tyr Ile Phe Ala Lys Ala Ala His Ala Pro Phe Ile Ser
   225                 230                 235                 240
   cat ccg gcc gag ttt tgt cac ctg ctg gtg gcg ttg aag cag agg gtg         768
   His Pro Ala Glu Phe Cys His Leu Leu Val Ala Leu Lys Gln Arg Val
                   245                 250                 255
   tag                                                                     771
<210> 2
   <211> 256
   <212> PRT
   <213> Escherichia coli
<400> 2
   Met Asn Asn Ile Trp Trp Gln Thr Lys Gly Gln Gly Asn Val His Leu
   1               5                   10                  15
   Val Leu Leu His Gly Trp Gly Leu Asn Ala Glu Val Trp Arg Cys Ile
               20                  25                  30
   Asp Glu Glu Leu Ser Ser His Phe Thr Leu His Leu Val Asp Leu Pro
           35                  40                  45
   Gly Phe Gly Arg Ser Arg Gly Phe Gly Ala Leu Ser Leu Ala Asp Met
       50                  55                  60
   Ala Glu Ala Val Leu Gln Gln Ala Pro Asp Lys Ala Ile Trp Leu Gly
   65                  70                  75                  80
   Trp Ser Leu Gly Gly Leu Val Ala Ser Gln Ile Ala Leu Thr His Pro
                   85                  90                  95
   Glu Arg Val Gln Ala Leu Val Thr Val Ala Ser Ser Pro Cys Phe Ser
               100                 105                 110
   Ala Arg Asp Glu Trp Pro Gly Ile Lys Pro Asp Val Leu Ala Gly Phe
   115 120 125
   Gln Gln Gln Leu Ser Asp Asp Phe Gln Arg Thr Val Glu Arg Phe Leu
   130 135 140
   Ala Leu Gln Thr Met Gly Thr Glu Thr Ala Arg Gln Asp Ala Arg Ala
   145 150 155 160
   Leu Lys Lys Thr Val Leu Ala Leu Pro Met Pro Glu Val Asp Val Leu
   165 170 175
   Asn Gly Gly Leu Glu Ile Leu Lys Thr Val Asp Leu Arg Gln Pro Leu
   180 185 190
   Gln Asn Val Ser Met Pro Phe Leu Arg Leu Tyr Gly Tyr Leu Asp Gly
   195 200 205
   Leu Val Pro Arg Lys Val Val Pro Met Leu Asp Lys Leu Trp Pro His
   210 215 220
   Ser Glu Ser Tyr Ile Phe Ala Lys Ala Ala His Ala Pro Phe Ile Ser
   225 230 235 240
   His Pro Ala Glu Phe Cys His Leu Leu Val Ala Leu Lys Gln Arg Val
   245 250 255
<210> 3
   <211> 500
   <212> DNA
   <213> Escherichia coli
<400> 3
   cggcttcgct atcccattgg cagtgcaacc agcgtgataa cggctgacac agcaaatcgc 60
   tctgattaaa tccccgacgc cagtgacgcc gctgccataa cggaacgctg acgatgcgat 120
   ccggcaattg caacccggtg gtgcgacgag cgtgtaagac ttccaatagt aacagacgtg 180
   acagggcgct ggcgatttca ctgcgccggg aaaatttaag ctggtggata agcggactta 240
   acggcggcgc atagtcggca accgtgacca gtctttgcca gggcggcggt ttttgcaggc 300
   agcgaccgca gggaagatgg gagtgtgtgg cgggtaatcc acattgtggg cataacgttt 360
   tatctgtgcg ggtggcgcgt gaacagaccg aacaaatccc ccaatgacct aacgccagtg 420
   gcattcggca tagccagcat aatcccggta ctgttagcat atgttcatcc ttgtaagtca 480
   aaagagaaca atagcggatg 500
<210> 4
   <211> 500
   <212> DNA
   <213> Escherichia coli
<400> 4
   gcgttgaagc agagggtgta ggtggctttt gaaatggcga gacagaactt tactgactcg      60
   ccgcagccaa ctcttcttct gacagcccgg taaagcgcat gatgtctgca agaggaactc     120
   cggattcaag cattattttg gctatatgca gggctttgga ttgttccccc tcctgccgca     180
   atctttccgc aatagtcatt aaactctcct tgtgtttcgg tgaacgttcg gcaacgccgt     240
   cgataaaatc gttaaaacgt acagcgtcgc cagtttgcag tatgtaatta aacagccctt     300
   tgatttgtct gtcattagcg tatccactac ttaataagca ggccatttgc tctaccagcc     360
   ccatcaggtc gcgttgacga atatgttttt gaattaactc cagcagcgcc atacgtcggt     420
   gctgcatgat ttcatcatca ggcatgacgg tgacatcaat cagcggaaat gcggaggcat     480
   aaagctgcct tgccagtttg                                                 500
<210> 5
   <211> 6654
   <212> DNA
   <213> Artificial Sequence
<220>
   <223> vector
<400> 5
   gagtcgaccg gtggcgaatg ggacgcgccc tgtagcggcg cattaagcgc ggcgggtgtg      60
   gtggttacgc gcagcgtgac cgctacactt gccagcgccc tagcgcccgc tcctttcgct     120
   ttcttccctt cctttctcgc cacgttcgcc ggctttcccc gtcaagctct aaatcggggg     180
   ctccctttag ggttccgatt tagtgcttta cggcacctcg accccaaaaa acttgattag     240
   ggtgatggtt cacgtagtgg gccatcgccc tgatagacgg tttttcgccc tttgacgttg     300
   gagtccacgt tctttaatag tggactcttg ttccaaactg gaacaacact caaccctatc     360
   tcggtctatt cttttgattt ataagggatt ttgccgattt cggcctattg gttaaaaaat     420
   gagctgattt aacaaaaatt taacgcgaat tttaacaaaa tattaacgct tacaatttag     480
   gtggcacttt tcggggaaat gtgcgcggaa cccctatttg tttatttttc taaatacatt     540
   caaatatgta tccgctcacc gcgatccttt ttaacccatc acatatacct gccgttcact     600
   attatttagt gaaatgagat attatgatat tttctgaatt gtgattaaaa aggcaacttt     660
   atgcccatgc aacagaaact ataaaaaata cagagaatga aaagaaacag atagattttt     720
   tagttcttta ggcccgtagt ctgcaaatcc ttttatgatt ttctatcaaa caaaagagga     780
   aaatagacca gttgcaatcc aaacgagagt ctaatagaat gaggtcgaaa agtaaatcgc     840
   gcgggtttgt tactgataaa gcaggcaaga cctaaaatgt gtaaagggca aagtgtatac     900
   tttggcgtca ccccttacat attttaggtc tttttttatt gtgcgtaact aacttgccat    960
   cttcaaacag gagggctgga agaagcagac cgctaacaca gtacataaaa aaggagacat 1020
   gaacgatgaa catcaaaaag tttgcaaaac aagcaacagt attaaccttt actaccgcac 1080
   tgctggcagg aggcgcaact caagcgtttg cgaaagaaac gaaccaaaag ccatataagg 1140
   aaacatacgg catttcccat attacacgcc atgatatgct gcaaatccct gaacagcaaa 1200
   aaaatgaaaa atatcaagtt cctgagttcg attcgtccac aattaaaaat atctcttctg 1260
   caaaaggcct ggacgtttgg gacagctggc cattacaaaa cgctgacggc actgtcgcaa 1320
   actatcacgg ctaccacatc gtctttgcat tagccggaga tcctaaaaat gcggatgaca 1380
   catcgattta catgttctat caaaaagtcg gcgaaacttc tattgacagc tggaaaaacg 1440
   ctggccgcgt ctttaaagac agcgacaaat tcgatgcaaa tgattctatc ctaaaagacc 1500
   aaacacaaga atggtcaggt tcagccacat ttacatctga cggaaaaatc cgtttattct 1560
   acactgattt ctccggtaaa cattacggca aacaaacact gacaactgca caagttaacg 1620
   tatcagcatc agacagctct ttgaacatca acggtgtaga ggattataaa tcaatctttg 1680
   acggtgacgg aaaaacgtat caaaatgtac agcagttcat cgatgaaggc aactacagct 1740
   caggcgacaa ccatacgctg agagatcctc actacgtaga agataaaggc cacaaatact 1800
   tagtatttga agcaaacact ggaactgaag atggctacca aggcgaagaa tctttattta 1860
   acaaagcata ctatggcaaa agcacatcat tcttccgtca agaaagtcaa aaacttctgc 1920
   aaagcgataa aaaacgcacg gctgagttag caaacggcgc tctcggtatg attgagctaa 1980
   acgatgatta cacactgaaa aaagtgatga aaccgctgat tgcatctaac acagtaacag 2040
   atgaaattga acgcgcgaac gtctttaaaa tgaacggcaa atggtacctg ttcactgact 2100
   cccgcggatc aaaaatgacg attgacggca ttacgtctaa cgatatttac atgcttggtt 2160
   atgtttctaa ttctttaact ggcccataca agccgctgaa caaaactggc cttgtgttaa 2220
   aaatggatct tgatcctaac gatgtaacct ttacttactc acacttcgct gtacctcaag 2280
   cgaaaggaaa caatgtcgtg attacaagct atatgacaaa cagaggattc tacgcagaca 2340
   aacaatcaac gtttgcgcca agcttcctgc tgaacatcaa aggcaagaaa acatctgttg 2400
   tcaaagacag catccttgaa caaggacaat taacagttaa caaataaaaa cgcaaaagaa 2460
   aatgccgata ttgactaccg gaagcagtgt gaccgtgtgc ttctcaaatg cctgattcag 2520
   gctgtctatg tgtgactgtt gagctgtaac aagttgtctc aggtgttcaa tttcatgttc 2580
   tagttgcttt gttttactgg tttcacctgt tctattaggt gttacatgct gttcatctgt 2640
   tacattgtcg atctgttcat ggtgaacagc tttaaatgca ccaaaaactc gtaaaagctc 2700
   tgatgtatct atctttttta caccgttttc atctgtgcat atggacagtt ttccctttga 2760
   tatgtaacgg tgaacagttg ttctactttt gtttgttagt cttgatgctt cactgataga 2820
   tacaagagcc ataagaacct cagatccttc cgtatttagc cagtatgttc tctagtgtgg 2880
   ttcgttgttt ttgcgtgagc catgagaacg aaccattgag atcatactta ctttgcatgt 2940
   cactcaaaaa ttttgcctca aaactggtga gctgaatttt tgcagttaaa gcatcgtgta 3000
   gtgtttttct tagtccgtta tgtaggtagg aatctgatgt aatggttgtt ggtattttgt 3060
   caccattcat ttttatctgg ttgttctcaa gttcggttac gagatccatt tgtctatcta 3120
   gttcaacttg gaaaatcaac gtatcagtcg ggcggcctcg cttatcaacc accaatttca 3180
   tattgctgta agtgtttaaa tctttactta ttggtttcaa aacccattgg ttaagccttt 3240
   taaactcatg gtagttattt tcaagcatta acatgaactt aaattcatca aggctaatct 3300
   ctatatttgc cttgtgagtt ttcttttgtg ttagttcttt taataaccac tcataaatcc 3360
   tcatagagta tttgttttca aaagacttaa catgttccag attatatttt atgaattttt 3420
   ttaactggaa aagataaggc aatatctctt cactaaaaac taattctaat ttttcgcttg 3480
   agaacttggc atagtttgtc cactggaaaa tctcaaagcc tttaaccaaa ggattcctga 3540
   tttccacagt tctcgtcatc agctctctgg ttgctttagc taatacacca taagcatttt 3600
   ccctactgat gttcatcatc tgaacgtatt ggttataagt gaacgatacc gtccgttctt 3660
   tccttgtagg gttttcaatc gtggggttga gtagtgccac acagcataaa attagcttgg 3720
   tttcatgctc cgttaagtca tagcgactaa tcgctagttc atttgctttg aaaacaacta 3780
   attcagacat acatctcaat tggtctaggt gattttaatc actataccaa ttgagatggg 3840
   ctagtcaatg ataattacta gtccttttcc tttgagttgt gggtatctgt aaattctgct 3900
   agacctttgc tggaaaactt gtaaattctg ctagaccctc tgtaaattcc gctagacctt 3960
   tgtgtgtttt ttttgtttat attcaagtgg ttataattta tagaataaag aaagaataaa 4020
   aaaagataaa aagaatagat cccagccctg tgtataactc actactttag tcagttccgc 4080
   agtattacaa aaggatgtcg caaacgctgt ttgctcctct acaaaacaga ccttaaaacc 4140
   ctaaaggctt aagtagcacc ctcgcaagct cgggcaaatc gctgaatatt ccttttgtct 4200
   ccgaccatca ggcacctgag tcgctgtctt tttcgtgaca ttcagttcgc tgcgctcacg 4260
   gctctggcag tgaatggggg taaatggcac tacaggcgcc ttttatggat tcatgcaagg 4320
   aaactaccca taatacaaga aaagcccgtc acgggcttct cagggcgttt tatggcgggt 4380
   ctgctatgtg gtgctatctg actttttgct gttcagcagt tcctgccctc tgattttcca 4440
   gtctgaccac ttcggattat cccgtgacag gtcattcaga ctggctaatg cacccagtaa 4500
   ggcagcggta tcatcaacag gcttacccgt cttactgtcg ggatcgacgc ctctccctta 4560
   tgcgactcct gcacctttcg tcttcgaata aatacctgtg acggaagatc acttcgcaga 4620
   ataaataaat cctggtgtcc ctgttgatac cgggaagccc tgggccaact tttggcgaaa 4680
   atgagacgtt gatcggcacg taagaggttc caactttcac cataatgaaa taagatcact 4740
   accgggcgta ttttttgagt tatcgagatt ttcaggagct aaggaagcta aaatggagaa 4800
   aaaaatcact ggatatacca ccgttgatat atcccaatgg catcgtaaag aacattttga 4860
   ggcatttcag tcagttgctc aatgtaccta taaccagacc gttcagctgg atattacggc 4920
   ctttttaaag accgtaaaga aaaataagca caagttttat ccggccttta ttcacattct 4980
   tgcccgcctg atgaatgctc atccggaatt ccgtatggca atgaaagacg gtgagctggt 5040
   gatatgggat agtgttcacc cttgttacac cgttttccat gagcaaactg aaacgttttc 5100
   atcgctctgg agtgaatacc acgacgattt ccggcagttt ctacacatat attcgcaaga 5160
   tgtggcgtgt tacggtgaaa acctggccta tttccctaaa gggtttattg agaatatgtt 5220
   tttcgtctca gccaatccct gggtgagttt caccagtttt gatttaaacg tggccaatat 5280
   ggacaacttc ttcgcccccg ttttcaccat gggcaaatat tatacgcaag gcgacaaggt 5340
   gctgatgccg ctggcgattc aggttcatca tgccgtttgt gatggcttcc atgtcggcag 5400
   aatgcttaat gaattacaac agtactgcga tgagtggcag ggcggggcgt aattttttta 5460
   aggcagttat tggtgccctt aaacgcctgg ttgctacgcc tgaataagtg ataataagcg 5520
   gatgaatggc agaaattcga aagcaaattc gacccggtcg tcggttcagg gcagggtcgt 5580
   taaatagccg cttatgtcta ttgctggtct cggtacccgg ggatcgcggc ccggcttcgc 5640
   tatcccattg gcagtgcaac cagcgtgata acggctgaca cagcaaatcg ctctgattaa 5700
   atccccgacg ccagtgacgc cgctgccata acggaacgct gacgatgcga tccggcaatt 5760
   gcaacccggt ggtgcgacga gcgtgtaaga cttccaatag taacagacgt gacagggcgc 5820
   tggcgatttc actgcgccgg gaaaatttaa gctggtggat aagcggactt aacggcggcg 5880
   catagtcggc aaccgtgacc agtctttgcc agggcggcgg tttttgcagg cagcgaccgc 5940
   agggaagatg ggagtgtgtg gcgggtaatc cacattgtgg gcataacgtt ttatctgtgc 6000
   gggtggcgcg tgaacagacc gaacaaatcc cccaatgacc taacgccagt ggcattcggc 6060
   atagccagca taatcccggt actgttagca tatgttcatc cttgtaagtc aaaagagaac 6120
   aatagcggat gcctcgaggg cgttgaagca gagggtgtag gtggcttttg aaatggcgag 6180
   acagaacttt actgactcgc cgcagccaac tcttcttctg acagcccggt aaagcgcatg 6240
   atgtctgcaa gaggaactcc ggattcaagc attattttgg ctatatgcag ggctttggat 6300
   tgttccccct cctgccgcaa tctttccgca atagtcatta aactctcctt gtgtttcggt 6360
   gaacgttcgg caacgccgtc gataaaatcg ttaaaacgta cagcgtcgcc agtttgcagt 6420
   atgtaattaa acagcccttt gatttgtctg tcattagcgt atccactact taataagcag 6480
   gccatttgct ctaccagccc catcaggtcg cgttgacgaa tatgtttttg aattaactcc 6540
   agcagcgcca tacgtcggtg ctgcatgatt tcatcatcag gcatgacggt gacatcaatc 6600
   agcggaaatg cggaggcata aagctgcctt gccagtttgg gccgcgatcc tcta 6654
<210> 6
   <211> 39
   <212> DNA
   <213> Artificial Sequence
<220>
   <223> primer
<400> 6
   ccggggatcg cggcccggct tcgctatccc attggcagt 39
<210> 7
   <211> 58
   <212> DNA
   <213> Artificial Sequence
<220>
   <223> primer
<400> 7
   cctctgcttc aacgccctcg aggcatccgc tattgttctc ttttgactta caaggatg 58
<210> 8
   <211> 24
   <212> DNA
   <213> Artificial Sequence
<220>
   <223> primer
<400> 8
   gcgttgaagc agagggtgta ggtg 24
<210> 9
   <211> 39
   <212> DNA
   <213> Artificial Sequence
<220>
   <223> primer
<400> 9
   tagaggatcg cggcccaaac tggcaaggca gctttatgc 39
<210> 10
   <211> 1008
   <212> DNA
   <213> Artificial Sequence
<220>
   <223> resulting k/o gene
<400> 10
   cggcttcgct atcccattgg cagtgcaacc agcgtgataa cggctgacac agcaaatcgc 60
   tctgattaaa tccccgacgc cagtgacgcc gctgccataa cggaacgctg acgatgcgat 120
   ccggcaattg caacccggtg gtgcgacgag cgtgtaagac ttccaatagt aacagacgtg      180
   acagggcgct ggcgatttca ctgcgccggg aaaatttaag ctggtggata agcggactta      240
   acggcggcgc atagtcggca accgtgacca gtctttgcca gggcggcggt ttttgcaggc      300
   agcgaccgca gggaagatgg gagtgtgtgg cgggtaatcc acattgtggg cataacgttt      360
   tatctgtgcg ggtggcgcgt gaacagaccg aacaaatccc ccaatgacct aacgccagtg      420
   gcattcggca tagccagcat aatcccggta ctgttagcat atgttcatcc ttgtaagtca      480
   aaagagaaca atagcggatg cctcgagggc gttgaagcag agggtgtagg tggcttttga      540
   aatggcgaga cagaacttta ctgactcgcc gcagccaact cttcttctga cagcccggta      600
   aagcgcatga tgtctgcaag aggaactccg gattcaagca ttattttggc tatatgcagg      660
   gctttggatt gttccccctc ctgccgcaat ctttccgcaa tagtcattaa actctccttg      720
   tgtttcggtg aacgttcggc aacgccgtcg ataaaatcgt taaaacgtac agcgtcgcca      780
   gtttgcagta tgtaattaaa cagccctttg atttgtctgt cattagcgta tccactactt      840
   aataagcagg ccatttgctc taccagcccc atcaggtcgc gttgacgaat atgtttttga      900
   attaactcca gcagcgccat acgtcggtgc tgcatgattt catcatcagg catgacggtg      960
   acatcaatca gcggaaatgc ggaggcataa agctgccttg ccagtttg                  1008
<210> 11
   <211> 12348
   <212> DNA
   <213> Artificial Sequence
<220>
   <223> vector
<400> 11
   gggaagccct gcaaagtaaa ctggatggct ttcttgccgc caaggatctg atggcgcagg      60
   ggatcaagat ctgatcaaga gacaggatga ggatcgtttc gcatgattga acaagatgga     120
   ttgcacgcag gttctccggc cgcttgggtg gagaggctat tcggctatga ctgggcacaa     180
   cagacaatcg gctgctctga tgccgccgtg ttccggctgt cagcgcaggg gcgcccggtt     240
   ctttttgtca agaccgacct gtccggtgcc ctgaatgaac tgcaggacga ggcagcgcgg     300
   ctatcgtggc tggccacgac gggcgttcct tgcgcagctg tgctcgacgt tgtcactgaa     360
   gcgggaaggg actggctgct attgggcgaa gtgccggggc aggatctcct gtcatctcac     420
   cttgctcctg ccgagaaagt atccatcatg gctgatgcaa tgcggcggct gcatacgctt     480
   gatccggcta cctgcccatt cgaccaccaa gcgaaacatc gcatcgagcg agcacgtact     540
   cggatggaag ccggtcttgt cgatcaggat gatctggacg aagagcatca ggggctcgcg     600
   ccagccgaac tgttcgccag gctcaaggcg cgcatgcccg acggcgagga tctcgtcgtg     660
   acccatggcg atgcctgctt gccgaatatc atggtggaaa atggccgctt ttctggattc     720
   atcgactgtg gccggctggg tgtggcggac cgctatcagg acatagcgtt ggctacccgt 780
   gatattgctg aagagcttgg cggcgaatgg gctgaccgct tcctcgtgct ttacggtatc 840
   gccgctcccg attcgcagcg catcgccttc tatcgccttc ttgacgagtt cttctgagcg 900
   ggactctggg gttcgaaatg accgaccaat cgattggtaa ctgtcagacc aagtttactc 960
   atatatactt tagattgatt taaaacttca tttttaattt aaaaggatct aggtgaagat 1020
   cctttttgat aatctcatga ccaaaatccc ttaacgtgag ttttcgttcc actgagcgtc 1080
   agaccccgta gaaaagatca aaggatcttc ttgagatcct ttttttctgc gcgtaatctg 1140
   ctgcttgcaa acaaaaaaac caccgctacc agcggtggtt tgtttgccgg atcaagagct 1200
   accaactctt tttccgaagg taactggctt cagcagagcg cagataccaa atactgtcct 1260
   tctagtgtag ccgtagttag gccaccactt caagaactct gtagcaccgc ctacatacct 1320
   cgctctgcta atcctgttac cagtggctgc tgccagtggc gataagtcgt gtcttaccgg 1380
   gttggactca agacgatagt taccggataa ggcgcagcgg tcgggctgaa cggggggttc 1440
   gtgcacacag cccagcttgg agcgaacgat ctacaccgaa ctgagatacc tacagcgtga 1500
   gctatgagaa agcgccacgc ttcccgaagg gagaaaggcg gacaggtatc cggtaagcgg 1560
   cagagtcgga acaggagagc gcacgaggga gcttccaggg ggaaacgcct ggtatcttta 1620
   tagtcctgtc gggtttcgcc acctctgact tgagcgtcga tttttgtgat gctcgtcagg 1680
   ggggcggagc ctatggaaaa acgccagcaa cgcggccttt ttacggttcc tggccttttg 1740
   ctggcctttt gctcacatgt tctttcctgc gttatcccct gattctgtgg ataaccgtat 1800
   taccgccttt gagtgagctg ataccgctcg ccgcagccga acgaccgagc gcagcgagtc 1860
   agtgagcgag gaagcggaag agcgcctgat gcggtatttt ctccttacgc atctgtgcgg 1920
   tatttcacac cgcatagggg atctccaatc gtgccttggc gcagcgacag ccctcggtcc 1980
   cccagatagc cattgatctt ctctcgcctg tcccctcagt tcagtaattt cctgcatttg 2040
   cctgtttcca gtcggtagat attccacaaa acagcaggga agcagcgctt ttccgctgca 2100
   taaccctgct tcggggtcat tatagcgatt ttttcggtat atccatcctt tttcgcacga 2160
   tatacaggat tttgccaaag ggttcgtgta gactttcctt ggtgtatcca acggcgtcag 2220
   ccgggcagga taggtgaagt aggcccaccc gcgagcgggt gttccttctt cactgtccct 2280
   tattcgcacc tggcggtgct caacgggaat cctgctctgc gaggctggcc ggctaccgcc 2340
   ggcgtaacag atgagggcaa gcggatggct gatgaaacca agccaaccag gaagggcagc 2400
   ccacctatca aggtgtactg ccttccagac gaacgaagag cgattgagga aaaggcggcg 2460
   gcggccggca tgagcctgtc ggcctacctg ctggccgtcg gccagggcta caaaatcacg 2520
   ggcgtcgtgg actatgagct cgagaacgct taccgccaac acagcagtgt atttgaataa 2580
   gagctcgaac atgatcggat ctcccatttc agcaagggaa atcccaccat agccaccacc 2640
   gatttatgtg gcgtagaaaa cggtcatcac caaatatggc aatacttccc cgcccaagcg 2700
   ccacataaca gcctgggcca cattttacgc ggtagttccg cacgtacgga gtgcgggggg 2760
   cggcaggtaa ctgccgtccc tatcgcgacc gttggttcga gtggctgagc atcattgcta 2820
   atcaggtaat tttatactcc ctgcaagcgc accttgacgt ttaggcaaat ttattgtctc 2880
   agttgcaatc agtcgctcct gcttgtacgc aaggacttct agttcaagag tttcgttatt 2940
   aattgcaaca acgagtttat cgtagtcctt tagagcacca agtccttgca gcgccatccc 3000
   tttaagatca gaccagaacc gtggtggggt tggtgctggt gttgatgtgc cacagatgct 3060
   acttgcgaca atttgagcgt gtgtaaccgc attatgaatt gtctctaaac gtaccatcgt 3120
   tccccaaaaa ggatttctag ccattgcgca gtcgccgatt gcatatatac ttgtatccga 3180
   tgtacacatc tgatcatcga ccacaacacc attactcact tcaagggccg cctcagttgc 3240
   cagctctagc tctgggatag caccgattcc aactacaatc agatccgcct gaatttcttc 3300
   tccactttca agtacgcatt gttcaacatg gccattcctg ccctttatag acgttaattt 3360
   cgcattcagc ttgaactcaa ttccttcagc ctccaggcgg gctctgacta agtttgctgc 3420
   tgccggcgta accacgcgcg ccattacacg cggggcggct tctatcactg tgaccctctt 3480
   ccctaagccc accgcagctg aggcgacttc aagcccgatt actccgccgc ccaacacaac 3540
   aacagacgca ctctccacaa gtttcctacg taaatttttg gcgtcttcca tactgcgtaa 3600
   atagcagacc ccagacagtt cagacccctc gcaggttaac ctacgtgcgc tagcacctgt 3660
   tgcaagaatc aatttttcat acgcgtattc ttttccatct ttagaagaaa ctatcttacg 3720
   ccccacgtcg attgatacaa tcggtgtatt taacgaaatg gtaatattgt tattcgtata 3780
   aaaaccttct ggctttaatg gcactgcgga ttctgcaatc tcacttgtca gaaaagcctt 3840
   ggatagagga ggccgctgat aaggcgccac agactccctg ctaaaaatcc taatttcccc 3900
   tttataacca tattgacgaa gccagaacgc agcatttact ccagctgtac cagcgccaac 3960
   aacaacgatt gccataattc tctctccggt atacttttca ctatatcact taatgccgat 4020
   tattttagat aattccttga cgctcagctt caattgttgc ttgcgtgcga ttcactacat 4080
   tcaaggtggc aaatattttc ctcatatgcc actttatagc atcttcggtg acatgcatat 4140
   ttgttgctat ttgtttgttt gagcacccct cttttacaag cctcaagaca gcaatctgct 4200
   tccgtgtcaa taaagcgtca gctttattct ctgcggactt tccaatctca actattcgcg 4260
   gaagactaaa agccccaatc gcttgatcta aattaactgc tgtgaaggct tcacatgaag 4320
   ccggtattat tcgctcaatt aaacatactt catcaagaac tgtttgaaag cattgaagct 4380
   gttttgctat ctccactgca taaacaatgt taagctgagc cttttttaaa tcaccggcac 4440
   ctgcctgcgc tccggccaaa cacaataatc cacggacttc cagctggccc gcgttaattt 4500
   tacgggcttg ctgaatagcc aataacgctc tgtgcgcggc actatgaaag ttccgatctc 4560
   gggaaagcac tagtgattga acaagcagca ggcgtgcttt taggggggct gagtgctgtc 4620
   cggagaaaat cttatgatct tcaagagttt ttaaattatt tatgcccgtt atgccttgac 4680
   agactaagcg ctgatagatc tcaatttggc tcataacttc caatcttggt agattttttt 4740
   caaccgcatg cgccttcgcc cactccaata tctcaatgga gccatttagg tcactccttc 4800
   caagccgcca agctgacaca gcacggcata cggaaaaaaa cacgtctgtc accccgtgat 4860
   tggaaatgaa ctctaaaatt ttggagagct tttcttctga ggtgtccaag cagcgcaatt 4920
   cataatgtaa ctcaagctct agagcgtcaa acattttcga agtaaactcg gattccatca 4980
   tctgcgcgcg actgtctgtg cgtgcttgag ttataatctg cctcgcccag cccatttttc 5040
   cgcttgctag ggcttgttga aacctcgcga catacagcca accaaaagca aaattttgtt 5100
   ttgcaaattt attcacggct tgggcctgag ccagcacctt ctccaactct gcaaatctat 5160
   actcactggc aaaaataaaa gccaaacagg ttagcgcggc ccttttccca actgcgtttg 5220
   aatccccaaa taaactaatc cacttattac agagctcctc actcgaaagc atttcatctt 5280
   tcgttgcttt acctattgca agcacaagct gcagccattc cttttcttgc catttatttt 5340
   ttttatcgga ttgtgaagat aggtctttaa ttaacttctc tgctcgcgcg ccttgctgac 5400
   tgaaatacaa tacccacgcg taactaataa gcactatggg ttttttgtgc caggcctgct 5460
   tcggcagctc taacagccac tgtctcagcg catctatttc gccctgacga aatgacaaat 5520
   ctaaaattat tctctcagac atgctgactg cccagcgaca gtcattcgcc cgtagggata 5580
   ttcgtattgc atactggtat tcacctctac gccaatgcca gaaagctgca cgcttaagca 5640
   ggtaggatct tttagcagga ttttcagtcc aagtaatttc tcgtagaaaa ttacgcagta 5700
   ctggatgcag tgtaaactgc gctggctcac cgctcacatg gcgaagcaac atgtaattag 5760
   tgcttaaata cttaatacat gagaccccat tgacgcattt gaatacataa ttgtattgat 5820
   caggcgtcac gaaatcgagc aatgaagaat ttgcaagaaa aacacgatag cgctcgggaa 5880
   tcgcctcaaa tatttcatcc ctaaagtaat tgtctacttc aactactgct gaaatatgct 5940
   tggccggcaa ctcacgcttt aacaaaaaaa ctacaagagc aggccacccc tcaacttctt 6000
   gcaccaaggt ctctatctgt tcttcaggaa ctccaagaac agactctgcc tccgctaacg 6060
   ccaccgcctc ttctgcgcta aaggccaagt ctttctcggt gtactcccgc atagcgcctg 6120
   caagtttaag ctgcgagaac ccttttattg tattgcctgc aactgcaaac ctgatatttt 6180
   ttggtgtatt taacataaac tccataagtg cgtgcaacaa cggcaagtct aagtcatgat 6240
   taatattatc caaacaaact agcgtttcta tctcgttatt cgaggtgctc tgccaaagac 6300
   tagatgcaag gtctcgcaag agcgcaggct tgctcacacc ctctctcaca cggctgaatt 6360
   ttaccatttc gaaagtttca agctgctcaa taatctctgc gcagatatca aattcactgt 6420
   aagaactggc tcttaaagaa agccacactg caggacgtcc ggctgttctg tggcgtagcc 6480
   actcgaacgc aagagcaacg gttttcccat atccaggtgg ggctctgtaa aggcatactc 6540
   tgggagcggc tccatccgcg atactcaatc ttggccgata tatgcaacta tgaactttgg 6600
   cacttactag agtcgtaatt tgatccgctc cgaccttagc gaccgggaaa tcattattta 6660
   ttattatttt cattatgcta ttctcgcgcc agctgactgg aaattttcac cataggttac 6720
   ggtgttaaat attaaaacta cacttaagtg tagtcggcat gatcggtggt gcaaaatatt 6780
   tactagggaa ggtctgaagt aggccgctat ttctggccga cttcggcctt cgccgatttt 6840
   gaagacgggc accgggtcaa aatcgaccag atagctcgct catttcggtg ctttcagccg 6900
   tcgcgagtag ctcgcggtac ctggcatgct tgcggccagc tcgtgttttt ccagcagacg 6960
   acggagcaaa aactacccgt aggtgtagtt ggcgcaagcg tccgattagc tcaggtttaa 7020
   gatgtcgaga gtgagagtgg gcggcttaac tttctcagtt aggcataaaa ttacgtctta 7080
   aatctcgtag cgactaattt aataaaaatt ggagaattcc atatgcttga gaaacacaga 7140
   gttctggatt ccgctccaga gtacgtagat aaaaagaaat atctctggat actatcaact 7200
   ttgtggccgg ctactccgat gatcggaatc tggcttgcaa atgaaactgg ttgggggatt 7260
   ttttatgggc tggtattgct cgtatggtac ggcgcacttc cattgcttga tgcgatgttt 7320
   ggtgaggact ttaataatcc gcctgaagaa gtggtgccga aactagagaa ggagcggtac 7380
   tatcgagttt tgacatatct aacagttcct atgcattacg ctgcattaat tgtgtcagca 7440
   tggtgggtcg gaactcagcc aatgtcttgg cttgaaattg gtgcgcttgc cttgtcactg 7500
   ggtatcgtga acggactagc gctcaataca ggacacgaac tcggtcacaa gaaggagact 7560
   tttgatcgtt ggatggccaa aattgtgttg gctgtcgtag ggtacggtca cttctttatt 7620
   gagcataata agggtcatca ccgtgatgtc gctacaccga tggatcctgc aacatcccgg 7680
   atgggagaaa gcatttataa gttttcaatc cgtgagatcc caggagcatt tattcgtgct 7740
   tgggggcttg aggaacaacg cctttcgcgc cgtggccaaa gcgtttggag tttcgataat 7800
   gaaatcctcc aaccaatgat catcacagtt attctttacg ccgttctcct tgccttgttt 7860
   ggacctaaga tgctggtgtt cctgccgatt caaatggctt tcggttggtg gcagctgacc 7920
   agtgcgaact atattgaaca ttacggcttg ctccgtcaaa aaatggagga cggtcgatat 7980
   gagcatcaaa agccgcacca ttcttggaat agtaatcaca tcgtctctaa tctagtgctg 8040
   ttccaccttc agcggcactc ggatcaccac gcgcatccaa cacgttctta tcagtcactt 8100
   cgggattttc ccggcctgcc ggctcttccg acgggttacc ctggtgcatt tttgatggcg 8160
   atgattcctc agtggtttag atcagttatg gatcccaagg tagtagattg ggctggtggt 8220
   gaccttaata agatccaaat tgatgattcg atgcgagaaa cctatttgaa aaaatttggc 8280
   actagtagtg ctggtcatag ttcgagtacc tctgcggtag catcgtagtt atgtgagcac 8340
   gcagagcccg gcggtcgata tttacaataa gtgcttcaat tttatgtgcg gcgttgaaag 8400
   ctctcacaaa gagtgcactt cgctaaagtg ctgagggttg attgcctctc tgtaattgct 8460
   ttgaaggcga cctgctccga tagttacact ctgatgaagt tgtcggagca gcgactaacg 8520
   ctgagttaat aggagagtgg gagaatgtca aggtaccagt gtccagattg tcagtatatc 8580
   tatgatgaaa ataaggggga gccgcacgaa ggtttccacc cgaacaccag ctggaatgat 8640
   atccccaaag attgggcatg cccggactgc gcagttcgag acaaggtgga ctttatcttt 8700
   ctcgcggatt ctccctcgaa agaaacacag ctaggggtga atagtcagct tgccaactcg 8760
   gaaagtggta tttcagatgc tactccaact ggaatggcag ttttggccgc agaattagtg 8820
   atcccactta atcaagaaaa taaaaatgag ggctgtgcgg ctaagactga agttcttgat 8880
   caggcgagca ccccacaggt tgtaagaaaa tcttccacaa ggaagaagat gagaaataaa 8940
   taacgcaaat ttgccgcaac gcaaaataac aatttgacat ggtgatgagt atggctagct 9000
   ataaatgccc ggattgtaat tatgtttatg atgagagtgc gggtaatgtg catgaggggt 9060
   tttctccagg tacgccttgg caccttattc ctgaggattg gtgctgcccc gattgcgccg 9120
   ttcgagacaa gcttgacttc atgttaattg agagcggcgt aggtgaaaag ggcgtcacct 9180
   caacccatac ttcgccaaat ttatccgagg ttagtggcac aagtttaact gctgaagcag 9240
   tggttgcgcc gacaagctta gagaaattgc ctagtgccga cgttaaaggc caagatctat 9300
   ataaaactca acctccaagg tctgatgccc aaggcgggaa agcatacttg aagtggatat 9360
   gtattacttg tggccatata tatgatgagg cgttgggcga tgaggccgag ggttttactc 9420
   caggtactcg ctttgaggat attcctgatg actggtgctg tccggattgc ggggctacga 9480
   aagaagacta tgtgctctac gaggaaaagt gaagattaaa acttcaagtc attctaggta 9540
   attcaggaca aaataaaaat gaccatacca attagcctag ccaagttaaa ctctagtgcc 9600
   gatacccatt cagcgcttgt cgacctgtaa cgacaacaaa acgagggtag cacaatgagt 9660
   ttttctaatt ataaagtaat cgcgatgccg gtgttggttg ctaattttgt tttgggggcg 9720
   gccactgcat gggcgaatga aaattatccg gcgaaatctg ctggctataa tcagggtgac 9780
   tgggtcgcta gcttcaattt ttctaaggtc tatgtgggtg aggagcttgg cgatctaaat 9840
   gttggagggg gggctttgcc aaatgctgat gtaagtattg gtaatgatac aacacttacg 9900
   tttgatatcg cctattttgt tagctcaaat atagcggtgg atttttttgt tggggtgcca 9960
   gctagggcta aatttcaagg tgagaaatca atctcctcgc tgggaagagt cagtgaagtt 10020
   gattacggcc ctgcaattct ttcgcttcaa tatcattacg atagctttga gcgactttat 10080
   ccatatgttg gggttggtgt tggtcgggtg ctattttttg ataaaaccga cggtgctttg 10140
   agttcgtttg atattaagga taaatgggcg cctgcttttc aggttggcct tagatatgac 10200
   cttggtaact catggatgct aaattcagat gtgcgttata ttcctttcaa aacggacgtc 10260
   acaggtactc ttggcccggt tcctgtttct actaaaattg aggttgatcc tttcattctc 10320
   agtcttggtg cgtcatatgt tttctaagta atcaggtctg tcactgtcgc aggtcgacct 10380
   gcagccaagc ttctgttttg gcggatgaga gaagattttc agcctgatac agattaaatc 10440
   agaacgcaga agcggtctga taaaacagaa tttgcctggc ggcagtagcg cggtggtccc 10500
   acctgacccc atgccgaact cagaagtgaa acgccgtagc gccgatggta gtgtggggtc 10560
   tccccatgcg agagtaggga actgccaggc atcaaataaa acgaaaggct cagtcgaaag 10620
   actgggcctt tcgttttatc tgttgtttgt cggtgaacgc tctcctgagt aggacaaatc 10680
   cgccgggagc ggatttgaac gttgcgaagc aacggcccgg agggtggcgg gcaggacgcc 10740
   cgccataaac tgccaggcat caaattaagc agaaggccat cctgacggat ggcctttttg 10800
   cgtttctaca aactcttttg tttatttttc taaatacatt caaatatgta tccgctcatg 10860
   agacaataac cctgataaat gcttcaataa tgcagcctga aaggcaggcc gggccgtggt 10920
   ggccacggcc tctaggccag atccagcggc atctgggtta gtcgagcgcg ggccgcttcc 10980
   catgtctcac cagggcgagc ctgtttcgcg atctcagcat ctgaaatctt cccggccttg 11040
   cgcttcgctg gggccttacc caccgccttg gcgggcttct tcggtccaaa actgaacaac 11100
   agatgtgtga ccttgcgccc ggtctttcgc tgcgcccact ccacctgtag cgggctgtgc 11160
   tcgttgatct gcgtcacggc tggatcaagc actcgcaact tgaagtcctt gatcgaggga 11220
   taccggcctt ccagttgaaa ccactttcgc agctggtcaa tttctatttc gcgctggccg 11280
   atgctgtccc attgcatgag cagctcgtaa agcctgatcg cgtgggtgct gtccatcttg 11340
   gccacgtcag ccaaggcgta tttggtgaac tgtttggtga gttccgtcag gtacggcagc 11400
   atgtctttgg tgaacctgag ttctacacgg ccctcaccct cccggtagat gattgtttgc 11460
   acccagccgg taatcatcac actcggtctt ttccccttgc cattgggctc ttgggttaac 11520
   cggacttccc gccgtttcag gcgcagggcc gcttctttga gctggttgta ggaagattcg 11580
   atagggacac ccgccatcgt cgctatgtcc tccgccgtca ctgaatacat cacttcatcg 11640
   gtgacaggct cgctcctctt cacctggcta atacaggcca gaacgatccg ctgttcctga 11700
   acactgaggc gatacgcggc ctcgaccagg gcattgcttt tgtaaaccat tgggggtgag 11760
   gccacgttcg acattccttg tgtataaggg gacactgtat ctgcgtccca caatacaaca 11820
   aatccgtccc tttacaacaa caaatccgtc ccttcttaac aacaaatccg tcccttaatg 11880
   gcaacaaatc cgtccctttt taaactctac aggccacgga ttacgtggcc tgtagacgtc 11940
   ctaaaaggtt taaaagggaa aaggaagaaa agggtggaaa cgcaaaaaac gcaccactac 12000
   gtggccccgt tggggccgca tttgtgcccc tgaaggggcg ggggaggcgt ctgggcaatc 12060
   cccgttttac cagtccccta tcgccgcctg agagggcgca ggaagcgagt aatcagggta 12120
   tcgaggcgga ttcacccttg gcgtccaacc agcggcacca gcggcgcctg agaggcgaat 12180
   tgacataagc ctgttcggtt cgtaaactgt aatgcaagta gcgtatgcgc tcacgcaact 12240
   ggtccagaac cttgaccgaa cgcagcggtg gtaacggcgc agtggcggtt ttcatggctt 12300
   gttatgactg tttttttgta cagtctatgc ctcgggcatc caatcgat 12348
<210> 12
   <211> 6834
   <212> DNA
   <213> Artificial Sequence
<220>
   <223> vector
<400> 12
   accaatgctt aatcagtgag gcacctatct cagcgatctg tctatttcgt tcatccatag      60
   ttgcctgact ccccgtcgtg tagataacta cgatacggga gggcttacca tctggcccca     120
   gcgctgcgat gataccgcga gaaccacgct caccggctcc ggatttatca gcaataaacc     180
   agccagccgg aagggccgag cgcagaagtg gtcctgcaac tttatccgcc tccatccagt     240
   ctattaattg ttgccgggaa gctagagtaa gtagttcgcc agttaatagt ttgcgcaacg     300
   ttgttgccat cgctacaggc atcgtggtgt cacgctcgtc gtttggtatg gcttcattca     360
   gctccggttc ccaacgatca aggcgagtta catgatcccc catgttgtgc aaaaaagcgg     420
   ttagctcctt cggtcctccg atcgttgtca gaagtaagtt ggccgcagtg ttatcactca     480
   tggttatggc agcactgcat aattctctta ctgtcatgcc atccgtaaga tgcttttctg     540
   tgactggtga gtactcaacc aagtcattct gagaatagtg tatgcggcga ccgagttgct     600
   cttgcccggc gtcaatacgg gataataccg cgccacatag cagaacttta aaagtgctca     660
   tcattggaaa acgttcttcg gggcgaaaac tctcaaggat cttaccgctg ttgagatcca     720
   gttcgatgta acccactcgt gcacccaact gatcttcagc atcttttact ttcaccagcg     780
   tttctgggtg agcaaaaaca ggaaggcaaa atgccgcaaa aaagggaata agggcgacac     840
   ggaaatgttg aatactcata ttcttccttt ttcaatatta ttgaagcatt tatcagggtt     900
   attgtctcat gagcggatac atatttgaat gtatttagaa aaataaacaa ataggggtca     960
   gtgttacaac caattaacca attctgaaca ttatcgcgag cccatttata cctgaatatg    1020
   gctcataaca ccccttgttt gcctggcggc agtagcgcgg tggtcccacc tgaccccatg    1080
   ccgaactcag aagtgaaacg ccgtagcgcc gatggtagtg tggggactcc ccatgcgaga    1140
   gtagggaact gccaggcatc aaataaaacg aaaggctcag tcgaaagact gggcctttcg    1200
   cccgggctaa ttatggggtg tcgcccttat tcgactctat agtgaagttc ctattctcta    1260
   gaaagtatag gaacttctga agtggggatc cggccggcct gcagccccgc agggcctgtc 1320
   tcggtcgatc attcagcccg gctcatagat atgcgggcag tgagcgcaac gcaattaatg 1380
   taagttagct cactcattag gcaccccagg cttgacactt tatgcttccg gctcgtataa 1440
   tgtgtggaat tgtgagcgga taacaataac aatttcacac aggatctagg aaccaaggag 1500
   agtggcatat gatcatcggc gtccctaaag aaatcaaaaa caacgaaaac cgcgtggcac 1560
   tgaccccggg tggtgtgtcc caattgatca gcaacggcca ccgtgtcttg gtcgaaaccg 1620
   gcgctggtct gggtagcggc tttgagaacg aggcatacga gtcggcaggt gcggagatta 1680
   ttgccgatcc taagcaggtg tgggacgccg agatggttat gaaagtgaaa gaaccgctgc 1740
   cggaagaata cgtttacttt cgcaaaggtc tggttctgtt cacctatctg cacttggccg 1800
   ctgagccgga gctggcacaa gcgctgaagg ataagggcgt tacggcgatc gcgtatgaaa 1860
   cggtgtctga gggccgtacc ctgccgctgc tgaccccgat gagcgaggtt gccggtcgta 1920
   tggcagccca gatcggtgcg cagttcctgg agaaaccgaa aggtggcaag ggcattctgc 1980
   tggcgggtgt cccgggtgtt tctcgtggta aggtcactat cattggcggt ggcgtggtcg 2040
   gtaccaacgc ggcgaagatg gcggttggcc tgggtgctga cgttacgatt atcgacttga 2100
   acgctgatcg cctgcgtcaa ttggacgaca tctttggcca ccagatcaag accttgatct 2160
   ccaatccggt gaatatcgcg gacgcggtgg cggaggcgga tctgctgatt tgcgcagttc 2220
   tgattcctgg cgcgaaggcg ccgaccctgg tcacggaaga aatggtgaaa caaatgaaac 2280
   cgggtagcgt gattgttgac gtagcgattg atcagggtgg tatcgtggaa actgttgacc 2340
   acatcacgac tcatgatcag ccgacgtacg agaaacatgg tgttgttcac tatgcagttg 2400
   caaatatgcc gggtgcggtc ccgcgtacta gcacgattgc cctgaccaat gtgaccgttc 2460
   cgtatgcact gcaaattgca aataagggtg cggtgaaggc tttggcggac aacaccgcgc 2520
   tgcgtgctgg tctgaatacc gcgaacggtc atgtgaccta tgaggcggtc gcacgtgacc 2580
   tgggttatga gtacgtgcct gcagagaagg cactgcagga cgagagctcc gtggcaggtg 2640
   cgtaataagc tttaaggaga tataatatgc agaaacagcg taccacctct cagtggcgtg 2700
   aactggatgc ggcgcatcat ctgcatccgt ttaccgatac cgcgagcctg aatcaggcgg 2760
   gtgcgcgtgt gatgacccgt ggcgaaggcg tgtatctgtg ggatagcgaa ggcaacaaaa 2820
   ttattgatgg catggcgggc ctgtggtgcg tgaacgtggg ctatggccgt aaagattttg 2880
   cggaagcggc gcgtcgtcag atggaagaac tgccgtttta taacaccttc tttaaaacca 2940
   cccatccggc ggtggtggaa ctgagcagcc tgctggccga agttaccccg gcaggttttg 3000
   atcgtgtgtt ttataccaac agcggcagcg aaagcgtgga taccatgatt cgtatggtgc 3060
   gtcgttattg ggatgtgcag ggcaaaccgg aaaaaaaaac cctgattggc cgttggaacg 3120
   gctatcacgg cagcaccatt ggcggtgcga gcctgggcgg catgaaatat atgcatgaac 3180
   agggcgatct gccgattccg ggcatggcgc atattgaaca gccgtggtgg tataaacatg 3240
   gcaaagatat gaccccggat gaatttggcg tggttgcggc gcgttggctg gaagaaaaaa 3300
   ttctggaaat cggcgcggat aaagtggcgg cgtttgtggg cgaaccgatt cagggtgcgg 3360
   gcggtgtgat tgttccgccg gcaacctatt ggccggaaat tgaacgtatt tgccgcaaat 3420
   atgatgtgct gctggttgcg gatgaagtga tttgcggctt tggccgtacc ggcgaatggt 3480
   ttggccatca gcattttggc tttcagccgg acctgtttac cgcggcgaaa ggcctgagca 3540
   gcggctatct gccgattggc gcggtgtttg tgggcaaacg tgttgcggaa ggtctgattg 3600
   cgggcggtga ttttaaccat ggctttacct atagcggcca tccggtgtgt gcggcggtgg 3660
   cgcatgcgaa tgttgcggcg ctgcgtgatg aaggcattgt gcagcgtgtg aaagatgata 3720
   ttggcccgta tatgcagaaa cgttggcgtg aaacctttag ccgttttgaa catgtggatg 3780
   atgtgcgtgg cgtgggcatg gtgcaggcgt ttaccctggt gaaaaacaaa gcgaaacgtg 3840
   aactgtttcc ggattttggc gaaattggca ccctgtgccg cgatattttt tttcgcaaca 3900
   acctgattat gcgtgcgtgc ggcgatcaca ttgtgtctgc accgccgctg gttatgaccc 3960
   gtgcggaagt ggatgaaatg ctggccgtgg cggaacgttg cctggaagaa tttgaacaga 4020
   ccctgaaagc gcgtggcctg gcctaaacta gttcaacaac tctcctggcg caccatcgtc 4080
   ggctacagcc tcgggaattg ctgcaagtcg acggatcgcc ggaattaatt ctcatgtttg 4140
   acagcttatc actgatcagt gaattaatgg cgatgacgca tcctcacgat aatatccggg 4200
   taggcgcaat cactttcgtc tctactccgt tacaaagcga ggctgggtat ttcccggcct 4260
   ttttgggccg gccggatcca aaatgaaggg aagttcctat actttctaga gaataggaac 4320
   ttctataggg agtcgaataa gggcgacaca aaaggtattc taaatgcata ataaatactg 4380
   ataacatctt atagtttgta ttatattttg tattatcgtt gacatgtata attttgatat 4440
   caaaaactga ttttcccttt attattttcg agatttattt tcttaattct ctttaacaaa 4500
   ctagaaatat tgtatataca aaaaatcata aataatagat gaatagttta attataggtg 4560
   ttcatcaatc gaaaaagcaa cgtatcttat ttaaagtgcg ttgctttttt ctcatttata 4620
   aggttaaata attctcatat atcaagcaaa gtgacaggcg cccttaaata ttctgacaaa 4680
   tgctctttcc ctaaactccc cccataaaaa aacccgccga agcgggtttt tacgttattt 4740
   gcggattaac gattactcgt tatcagaacc gcccaggatg cctggcagtt ccctactctc 4800
   gccgctgcgc tcggtcgttc ggctgcggga cctcagcgct agcggagtgt atactggctt 4860
   actatgttgg cactgatgag ggtgtcagtg aagtgcttca tgtggcagga gaaaaaaggc 4920
   tgcaccggtg cgtcagcaga atatgtgata caggatatat tccgcttcct cgctcactga 4980
   ctcgctacgc tcggtcgttc gactgcggcg agcggaaatg gcttacgaac ggggcggaga 5040
   tttcctggaa gatgccagga agatacttaa cagggaagtg agagggccgc ggcaaagccg 5100
   tttttccata ggctccgccc ccctgacaag catcacgaaa tctgacgctc aaatcagtgg 5160
   tggcgaaacc cgacaggact ataaagatac caggcgtttc cccctggcgg ctccctcgtg 5220
   cgctctcctg ttcctgcctt tcggtttacc ggtgtcattc cgctgttatg gccgcgtttg 5280
   tctcattcca cgcctgacac tcagttccgg gtaggcagtt cgctccaagc tggactgtat 5340
   gcacgaaccc cccgttcagt ccgaccgctg cgccttatcc ggtaactatc gtcttgagtc 5400
   caacccggaa agacatgcaa aagcaccact ggcagcagcc actggtaatt gatttagagg 5460
   agttagtctt gaagtcatgc gccggttaag gctaaactga aaggacaagt tttggtgact 5520
   gcgctcctcc aagccagtta cctcggttca aagagttggt agctcagaga accttcgaaa 5580
   aaccgccctg caaggcggtt ttttcgtttt cagagcaaga gattacgcgc agaccaaaac 5640
   gatctcaaga agatcatctt attaatcact gcccgctttc cagtcgggaa acctgtcgtg 5700
   ccagctgcat taatgaatcg gccaacgcgc ggggagaggc ggtttgcgta ttgggcgcca 5760
   gggtggtttt tcttttcacc agtgagactg gcaacagctg attgcccttc accgcctggc 5820
   cctgagagag ttgcagcaag cggtccacgc tggtttgccc cagcaggcga aaatcctgtt 5880
   tgatggtggt taacggcggg atataacatg agctatcttc ggtatcgtcg tatcccacta 5940
   ccgagatatc cgcaccaacg cgcagcccgg actcggtaat ggcgcgcatt gcgcccagcg 6000
   ccatctgatc gttggcaacc agcatcgcag tgggaacgat gccctcattc agcatttgca 6060
   tggtttgttg aaaaccggac atggcactcc agtcgccttc ccgttccgct atcggctgaa 6120
   tttgattgcg agtgagatat ttatgccagc cagccagacg cagacgcgcc gagacagaac 6180
   ttaatgggcc cgctaacagc gcgatttgct ggtgacccaa tgcgaccaga tgctccacgc 6240
   ccagtcgcgt accgtcctca tgggagaaaa taatactgtt gatgggtgtc tggtcagaga 6300
   catcaagaaa taacgccgga acattagtgc aggcagcttc cacagcaatg gcatcctggt 6360
   catccagcgg atagttaatg atcagcccac tgacgcgttg cgcgagaaga ttgtgcaccg 6420
   ccgctttaca ggcttcgacg ccgcttcgtt ctaccatcga caccaccacg ctggcaccca 6480
   gttgatcggc gcgagattta atcgccgcga caatttgcga cggcgcgtgc agggccagac 6540
   tggaggtggc aacgccaatc agcaacgact gtttgcccgc cagttgttgt gccacgcggt 6600
   tgggaatgta attcagctcc gccatcgccg cttccacttt ttcccgcgtt ttcgcagaaa 6660
   cgtggctggc ctggttcacc acgcgggaaa cggtctgata agagacaccg gcatactctg 6720
   cgacatcgta taacgttact ggtttcatat tcaccaccct gaattgactc tcttccgggc 6780
   gctatcatgc cataccgcga aaggttttgc gccattcgat ggcgcgccgc tttt 6834
<210> 13
   <211> 14403
   <212> DNA
   <213> Artificial Sequence
<220>
   <223> vector
<400> 13
   cggccccggg ctaattaggg ggtgtcgccc ttcttagtcg ctgaaaacgt tgaattccct 60
   tgggactcta gagatccgcg ggggcccccc gggggcgcgc caagtatagg aacttccctt 120
   cattttggat ccggccggcc caaaaaggcc gggaaatacc cagcctcgct ttgtaacgga 180
   gtagagacga aagtgattgc gcctacccgg atattatcgt gaggatgcgt catcgccatt 240
   aattcactga tcagtgataa gctgtcaaac atgagaatta attccggcga tccgtcgact 300
   tgcagcaatt cccgaggctg tagccgacga tggtgcgcca ggagagttgt tgaactagtt 360
   agtataacag gcgtgagtac caacgctcta gattattagg cgaggccacg cgctttgagg 420
   gtctgttcaa attcttcgag gcaacgttcc gccacggcga gcatttcatc cacttccgca 480
   cgggtcataa cgagcggcgg tgcagacaca atgtgatcgc cgcacgcacg cataatgagg 540
   ttgttgcgaa aaaaaatatc gcggcagagg gtgccaattt cgccaaaatc cggaaagagt 600
   tcacgtttcg ctttgttttt cacgagggta aacgcctgca ccatgcccac gccacgcaca 660
   tcatccacat gttcaaaacg gctaaaggtt tcacgccaac gtttctgcat atacgggcca 720
   atatcatctt tcacacgctg cacaatgcct tcatcacgga gcgccgcaac attcgcatgc 780
   gccaccgccg cacacaccgg atggccgcta taggtaaagc catggttaaa atcaccgccc 840
   gcaatgagac cttccgcaac acgtttgccc acaaacaccg cgccaatcgg gagatagccg 900
   ctgctgaggc ctttcgccgc ggtaaagagg tccggctgaa agccaaaatg ctgatggcca 960
   aaccattcgc cggtacggcc aaagccgcaa atcacttcat ccgcaacgag gagcacatca 1020
   tatttgcggc aaatacgttc aatttccggc caataggttg ccggcggaac aatcacaccg 1080
   cccgcaccct gaatcggttc gcccacaaac gccgccactt tatccgcgcc gatttcgaga 1140
   attttttctt cgagccaacg cgccgcaacc acgccaaatt catccggggt catatctttg 1200
   ccatgtttat accaccacgg ctgttcaata tgcgccatgc ccggaatcgg gagatcgccc 1260
   tgttcatgca tatatttcat gccgccgagg ctcgcaccgc caatggtgct gccgtgatag 1320
   ccgttccaac ggccaatgag ggtttttttt tccggtttgc cctgcacatc ccaataacga 1380
   cgcaccatac gaatcatggt atccacgctt tcgctgccgc tgttggtata aaacacacga 1440
   tcaaaacctg ccggggtaac ttcggcgagg aggctgctga gttccaccac cgccggatgg 1500
   gtggttttaa agaaggtgtt ataaaacggg agttcttcca tctgacgacg cgccgcttcc 1560
   gcaaaatctt tacggccata gcccacgttc acgcaccaga ggcccgccat gccatcaata 1620
   attttgttgc cttcgctatc ccagagatac acgccttcgc cacgggtcat cacacgcgca 1680
   cccgcctgat tgaggctcgc ggtatcggta aacggatgga gatgatgcgc cgcatcgagt 1740
   tcacgccact gagaggtggt acgctgtttc tgcatattat atctccttaa ctcgagttat 1800
   tacgcacctg ccacggagct ctcgtcctgc agtgccttct ctgcaggcac gtactcataa 1860
   cccaggtcac gtgcgaccgc ctcataggtc acatgaccgt tcgcggtatt cagaccagca 1920
   cgcagcgcgg tgttgtccgc caaagccttc accgcaccct tatttgcaat ttgcagtgca 1980
   tacggaacgg tcacattggt cagggcaatc gtgctagtac gcgggaccgc acccggcata 2040
   tttgcaactg catagtgaac aacaccatgt ttctcgtacg tcggctgatc atgagtcgtg 2100
   atgtggtcaa cagtttccac gataccaccc tgatcaatcg ctacgtcaac aatcacgcta 2160
   cccggtttca tttgtttcac catttcttcc gtgaccaggg tcggcgcctt cgcgccagga 2220
   atcagaactg cgcaaatcag cagatccgcc tccgccaccg cgtccgcgat attcaccgga 2280
   ttggagatca aggtcttgat ctggtggcca aagatgtcgt ccaattgacg caggcgatca 2340
   gcgttcaagt cgataatcgt aacgtcagca cccaggccaa ccgccatctt cgccgcgttg 2400
   gtaccgacca cgccaccgcc aatgatagtg accttaccac gagaaacacc cgggacaccc 2460
   gccagcagaa tgcccttgcc acctttcggt ttctccagga actgcgcacc gatctgggct 2520
   gccatacgac cggcaacctc gctcatcggg gtcagcagcg gcagggtacg gccctcagac 2580
   accgtttcat acgcgatcgc cgtaacgccc ttatccttca gcgcttgtgc cagctccggc 2640
   tcagcggcca agtgcagata ggtgaacaga accagacctt tgcgaaagta aacgtattct 2700
   tccggcagcg gttctttcac tttcataacc atctcggcgt cccacacctg cttaggatcg 2760
   gcaataatct ccgcacctgc cgactcgtat gcctcgttct caaagccgct acccagacca 2820
   gcgccggttt cgaccaagac acggtggccg ttgctgatca attgggacac accacccggg 2880
   gtcagtgcca cgcggttttc gttgtttttg atttctttag ggacgccgat gatcatatgc 2940
   cactctcctt ggttcctaga tcctgtgtga aattgttatt gttatccgct cacaattcca 3000
   cacattatac gagccggaag cataaagtgt caagcctggg gtgcctaatg agtgagctaa 3060
   cttacattaa ttgcgttgcg ctcactgccc ctgcaggggc cggccgttta aacaccaggt 3120
   gcgatcgcgc ggccgcgctc gagaacgctt accgccaaca cagcagtgta tttgaataag 3180
   agctcgaaca tgatcggatc tcccatttca gcaagggaaa tcccaccata gccaccaccg 3240
   atttatgtgg cgtagaaaac ggtcatcacc aaatatggca atacttcccc gcccaagcgc 3300
   cacataacag cctgggccac attttacgcg gtagttccgc acgtacggag tgcggggggc 3360
   ggcaggtaac tgccgtccct atcgcgaccg ttggttcgag tggctgagca tcattgctaa 3420
   tcaggtaatt ttatactccc tgcaagcgca ccttgacgtt taggcaaatt tattgtctca 3480
   gttgcaatca gtcgctcctg cttgtacgca aggacttcta gttcaagagt ttcgttatta 3540
   attgcaacaa cgagtttatc gtagtccttt agagcaccaa gtccttgcag cgccatccct 3600
   ttaagatcag accagaaccg tggtggggtt ggtgctggtg ttgatgtgcc acagatgcta 3660
   cttgcgacaa tttgagcgtg tgtaaccgca ttatgaattg tctctaaacg taccatcgtt 3720
   ccccaaaaag gatttctagc cattgcgcag tcgccgattg catatatact tgtatccgat 3780
   gtacacatct gatcatcgac cacaacacca ttactcactt caagggccgc ctcagttgcc 3840
   agctctagct ctgggatagc accgattcca actacaatca gatccgcctg aatttcttct 3900
   ccactttcaa gtacgcattg ttcaacatgg ccattcctgc cctttataga cgttaatttc 3960
   gcattcagct tgaactcaat tccttcagcc tccaggcggg ctctgactaa gtttgctgct 4020
   gccggcgtaa ccacgcgcgc cattacacgc ggggtggctt ctatcactgt gaccctcttc 4080
   cctaagccca ccgcagctga ggcgacttca agcccgatta ctccgccgcc caacacaaca 4140
   acagacgcac tctccacaag tttcctacgt aaatttttgg cgtcttccat actgcgtaaa 4200
   tagcagaccc cagacagttc agacccctcg caggttaacc tacgtgcgct agcaggtgtt 4260
   gcaagaatca atttttcata cgcgtattct tttccatctt tagaagaaac tatcttacgc 4320
   cccacgtcga ttgatacaat cggtgtattt aacgaaatgg taatattgtt attcgtataa 4380
   aaaccttctg gctttaatgg cactgcggat tctgcaatct cacttgtcag aaaagccttg 4440
   gatagaggag gccgctgata aggcgccaca gactccctgc taaaaatcct aatttcccct 4500
   ttataaccat attgacgaag ccagaacgca gcatttactc cagctgtacc agcgccaaca 4560
   acaacgattg ccataattct ctctccggta tacttttcac tatatcactt aatgccgatt 4620
   attttagata attccttgac gctcagcttc aattgttgct tgcgtgcgat tcactacatt 4680
   caaggtggca aatattttcc tcatatgcca ctttatagca tcttcggtga catgcatatt 4740
   tgttgctatt tgtttgtttg agcacccctc ttttacaagc ctcaagacag caatctgctt 4800
   ccgtgtcaat aaagcgtcag ctttattctc tgcggacttt ccaatctcaa ctattcgcgg 4860
   aagactaaaa gccccaatcg cttgatctaa attaactgct gtgaaggctt cacatgaagc 4920
   cggtattatt cgctcaatta aacatacttc atcaagaact gtttgaaagc attgaagctg 4980
   ttttgctatc tccactgcat aaacaatgtt aagctgagcc ttttttaaat caccggcacc 5040
   tgcctgcgct ccggccaaac acaataatcc acggacttcc agctggcccg cgttaatttt 5100
   acgggcttgc tgaatagcca ataacgctct gtgcgcggca ctatgaaagt tccgatctcg 5160
   ggaaagcact agtgattgaa caagcagcag gcgtgctttt aggggggctg agtgctgtcc 5220
   ggagaaaatc ttatgatctt caagagtttt taaattattt atgcccgtta tgccttgaca 5280
   gactaagcgc tgatagatct caatttggct cataacttcc aatcttggta gatttttttc 5340
   aaccgcatgc gccttcgccc actccaatat ctcaatggag ccatttaggt cactccttcc 5400
   aagccgccaa gctgacacag cacggcatac ggaaaaaaac acgtctgtca ccccgtgatt 5460
   ggaaatgaac tctaaaattt tggagagctt ttcttctgag gtgtccaagc agcgcaattc 5520
   ataatgtaac tcaagctcta gagcgtcaaa cattttcgaa gtaaactcgg attccatcat 5580
   ctgcgcgcga ctgtctgtgc gtgcttgagt tataatctgc ctcgcccagc ccatttttcc 5640
   gcttgctagg gcttgttgaa acctcgcgac atacagccaa ccaaaagcaa aattttgttt 5700
   tgcaaattta ttcacggctt gggcctgagc cagcaccttc tccaactctg caaatctata 5760
   ctcactggca aaaataaaag ccaaacaggt tagcgcggcc ccttttccaa ctgcgtttga 5820
   atccccaaat aaactaatcc acttattaca gagctcctca ctcgaaagca tttcatcttt 5880
   ggttgcttta cctattgcaa gcacaagctg cagccattcc ttttcttgcc atttattttt 5940
   tttatcggat tgtgaagata ggtctttaat taacttctct gctcgcgcgc cttgctgact 6000
   gaaatacaat acccacgcgt aactaataag cactatgggt ttttggtgcc aggcctgctt 6060
   cggcagctct aacagccact gtctcagcgc atctatttcg ccctgacgaa atgacaaatc 6120
   taaaattatt ctctcagaca tgctgactgc ccagcgacag tcattcgccc gtagggatat 6180
   tcgtattgca tactggtatt cacctctacg ccaatgccag aaagctgcac gcttaagcag 6240
   gtaggatctt ttagcaggat tttcagtcca agtaatttct cgtagaaaat tacgcagtac 6300
   tggatgcagt gtaaactgcg ctggctcacc gctcacatgg cgaagcaaca tgtaattagt 6360
   gcttaaatac ttaatacatg agaccccatt gacgcatttg aatacataat tgtattgatc 6420
   aggcgtcacg aaatcgagca atgaagaatt tgcaagaaaa acacgatagc gctcgggaat 6480
   cgcctcaaat atttcatccc taaagtaatt gtctacttca actactgctg aaatatgctt 6540
   ggccggcaac tcacgcttta acaaaaaaac tacaagagca ggccacccct caacttcttg 6600
   caccaaggtc tctatctgtt cttcaggaac tccaagaaca gactctgcct ccgctaacgc 6660
   caccgcctct tctgcgctaa aggccaagtc tttctcggtg tactcccgca tagcgcctgc 6720
   aagtttaagc tgcgagaacc cttttattgt attgcctgca actgcaaacc tgatattttt 6780
   tggtgtattt aacataaact ccataagtgc gtgcaacaac ggcaagtcta agtcatgatt 6840
   aatattatcc aaacaaacta gcgtttctat ctcgttattc gaggtgctct gccaaagact 6900
   agatgcaagg tctcgcaaga gcgcaggctt gctcacaccc tctctcacac ggctgaattt 6960
   taccatttcg aaagtttcaa gctgctcaat aatctctgcg cagatatcaa attcactgta 7020
   agaactggct cttaaagaaa gccacactgc aggacgtccg gctgttctgt ggcgtagcca 7080
   ctcgaacgca agagcaacgg ttttcccata tccaggtggg gctctgtaaa ggcatactct 7140
   gggagcggct ccatccgcga tactcaatct tggccgatat atgcaactat gaactttggc 7200
   acttactaga gtcgtaattt gatccgctcc gaccttagcg accgggaaat cattatttat 7260
   tattattttc attatgctat tctcgcgcca gctgactgga aattttcacc ataggttacg 7320
   gtgttaaata ttaaaactac acttaagtgt agtcggcatg atcggtggtg caaaatattt 7380
   actagggaag gtctgaagta ggccgctatt tctggccgac ttcggccttc gccgattttg 7440
   aagacgggca ccgggtcaaa atcgaccaga tagctcgctc atttcggtgc tttcagccgt 7500
   cgcgagtagc tcgcggtacc tggcatgctt gcggccagct cgtgtttttc cagcagacga 7560
   cggagcaaaa actacccgta ggtgtagttg gcgcaagcgt ccgattagct caggtttaag 7620
   atgtcgagag tgagagtggg cggcttaact ttctcagtta ggcataaaat tacgtcttaa 7680
   atctcgtagc gactaattta ataaaaattg gagaattcca tatgcttgag aaacacagag 7740
   ttctggattc cgctccagag tacgtagata aaaagaaata tctctggata ctatcaactt 7800
   tgtggccggc tactccgatg atcggaatct ggcttgcaaa tgaaactggt tgggggattt 7860
   tttatgggct ggtattgctc gtatggtacg gcgcacttcc attgcttgat gcgatgtttg 7920
   gtgaggactt taataatccg cctgaagaag tggtgccgaa actagagaag gagcggtact 7980
   atcgagtttt gacatatcta acagttccta tgcattacgc tgcattaatt gtgtcagcat 8040
   ggtgggtcgg aactcagcca atgtcttggc ttgaaattgg tgcgcttgcc ttgtcactgg 8100
   gtatcgtgaa cggactagcg ctcaatacag gacacgaact cggtcacaag aaggagactt 8160
   ttgatcgttg gatggccaaa attgtgttgg ctgtcgtagg gtacggtcac ttctttattg 8220
   agcataataa gggtcatcac cgtgatgtcg ctacaccgat ggatcctgca acatcccgga 8280
   tgggagaaag catttataag ttttcaatcc gtgagatccc aggagcattt attcgtgctt 8340
   gggggcttga ggaacaacgc ctttcgcgcc gtggccaaag cgtttggagt ttcgataatg 8400
   aaatcctcca accaatgatc atcacagtta ttctttacgc cgttctcctt gccttgtttg 8460
   gacctaagat gctggtgttc ctgccgattc aaatggcttt cggttggtgg cagctgacca 8520
   gtgcgaacta tattgaacat tacggcttgc tccgtcaaaa aatggaggac ggtcgatatg 8580
   agcatcaaaa gccgcaccat tcttggaata gtaatcacat cgtctctaat ctagtgctgt 8640
   tccaccttca gcggcactcg gatcaccacg cgcatccaac acgttcttat cagtcacttc 8700
   gggattttcc cggcctgccg gctcttccga cgggttaccc tggtgcattt ttgatggcga 8760
   tgattcctca gtggtttaga tcagttatgg atcccaaggt agtagattgg gctggtggtg 8820
   accttaataa gatccaaatt gatgattcga tgcgagaaac ctatttgaaa aaatttggca 8880
   ctagtagtgc tggtcatagt tcgagtacct ctgcggtagc atcgtagtta tgtgagcacg 8940
   cagagcccgg cggtcgatat ttacaataag tgcttcaatt ttatgtgcgg cgttgaaagc 9000
   tctcacaaag agtgcacttc gctaaagtgc tgagggttga ttgcctctct gtaattgctt 9060
   tgaaggcgac ctgctccgat agttacactc tgatgaagtt gtcggagcag cgactaacgc 9120
   tgagttaata ggagagtggg agaatgtcaa ggtaccagtg tccagattgt cagtatatct 9180
   atgatgaaaa taagggggag ccgcacgaag gtttccaccc gaacaccagc tggaatgata 9240
   tccccaaaga ttgggcatgc ccggactgcg cagttcgaga caaggtggac tttatctttc 9300
   tcgcggattc tccctcgaaa gaaacacagc taggggtgaa tagtcagctt gccaactcgg 9360
   aaagtggtat ttcagatgct actccaactg gaatggcagt tttggccgca gaattagtga 9420
   tcccacttaa tcaagaaaat aaaaatgagg gctgtgcggc taagactgaa gttcttgatc 9480
   aggcgagcac cccacaggtt gtaagaaaat cttccacaag gaagaagatg agaaataaat 9540
   aacgcaaatt tgccgcaacg caaaataaca atttgacatg gtgatgagta tggctagcta 9600
   taaatgcccg gattgtaatt atgtttatga tgagagtgcg ggtaatgtgc atgaggggtt 9660
   ttctccaggt acgccttggc accttattcc tgaggattgg tgctgccccg attgcgccgt 9720
   tcgagacaag cttgacttca tgttaattga gagcggcgta ggtgaaaagg gcgtcacctc 9780
   aacccatact tcgccaaatt tatccgaggt tagtggcaca agtttaactg ctgaagcagt 9840
   ggttgcgccg acaagcttag agaaattgcc tagtgccgac gttaaaggcc aagatctata 9900
   taaaactcaa cctccaaggt ctgatgccca aggcgggaaa gcatacttga agtggatatg 9960
   tattacttgt ggccatatat atgatgaggc gttgggcgat gaggccgagg gttttactcc 10020
   aggtactcgc tttgaggata ttcctgatga ctggtgctgt ccggattgcg gggctacgaa 10080
   agaagactat gtgctctacg aggaaaagtg aagattaaaa cttcaagtca ttctaggtaa 10140
   ttcaggacaa aataaaaatg accataccaa ttagcctagc caagttaaac tctagtgccg 10200
   atacccattc agcgcttgtc gacctgtaac gacaacaaaa cgagggtagc acaatgagtt 10260
   tttctaatta taaagtaatc gcgatgccgg tgttggttgc taattttgtt ttgggggcgg 10320
   ccactgcatg ggcgaatgaa aattatccgg cgaaatctgc tggctataat cagggtgact 10380
   gggtcgctag cttcaatttt tctaaggtct atgtgggtga ggagcttggc gatctaaatg 10440
   ttggaggggg ggctttgcca aatgctgatg taagtattgg taatgataca acacttacgt 10500
   ttgatatcgc ctattttgtt agctcaaata tagcggtgga tttttttgtt ggggtgccag 10560
   ctagggctaa atttcaaggt gagaaatcaa tctcctcgct gggaagagtc agtgaagttg 10620
   attacggccc tgcaattctt tcgcttcaat atcattacga tagctttgag cgactttatc 10680
   catatgttgg ggttggtgtt ggtcgggtgc tattttttga taaaaccgac ggtgctttga 10740
   gttcgtttga tattaaggat aaatgggcgc ctgcttttca ggttggcctt agatatgacc 10800
   ttggtaactc atggatgcta aattcagatg tgcgttatat tcctttcaaa acggacgtca 10860
   caggtactct tggcccggtt cctgtttcta ctaaaattga ggttgatcct ttcattctca 10920
   gtcttggtgc gtcatatgtt ttctaagtaa tcaggtctgt cactgtcgca ggtcgacctg 10980
   cagccaagct tctgttttgg cggatgagag aagattttca gcctgataca gattaaatca 11040
   gaacgcagaa gcggtctgat aaaacagaat ttgcctggcg gcagtagcgc ggtggtccca 11100
   cctgacccca tgccgaactc agaagtgaaa cgccgtagcg ccgatggtag tgtggggtct 11160
   ccccatgcga gagtagggaa ctgccaggca tcaaataaaa cgaaaggctc agtcgaaaga 11220
   ctgggccttt cgttttatct gttgtttgtc ggtgaacgct ctcctgagta ggacaaatcc 11280
   gccgggagcg gatttgaacg atgataagct gtcaaacatg agaattacaa cttatatcgt 11340
   atggggctga cttcaggtgc tacatttgaa gagataaatt gcactgaaat ctagaaatat 11400
   tttatctgat taataagatg atcttcttga gatcgttttg gtctgcgcgt aatctcttgc 11460
   tctgaaaacg aaaaaaccgc cttgcagggc ggtttttcga aggttctctg agctaccaac 11520
   tctttgaacc gaggtaactg gcttggagga gcgcagtcac caaaacttgt cctttcagtt 11580
   tagccttaac cggcgcatga cttcaagact aactcctcta aatcaattac cagtggctgc 11640
   tgccagtggt gcttttgcat gtctttccgg gttggactca agacgatagt taccggataa 11700
   ggcgcagcgg tcggactgaa cggggggttc gtgcatacag tccagcttgg agcgaactgc 11760
   ctacccggaa ctgagtgtca ggcgtggaat gagacaaacg cggccataac agcggaatga 11820
   caccggtaaa ccgaaaggca ggaacaggag agcgcacgag ggagccgcca gggggaaacg 11880
   cctggtatct ttatagtcct gtcgggtttc gccaccactg atttgagcgt cagatttcgt 11940
   gatgcttgtc aggggggcgg agcctatgga aaaacggctt tgccgcggcc ctctcacttc 12000
   cctgttaagt atcttcctgg catcttccag gaaatctccg ccccgttcgt aagccatttc 12060
   cgctcgccgc agtcgaacga ccgagcgtag cgagtcagtg agcgaggaag cggaatatat 12120
   cctgtatcac atattctgct gacgcaccgg tgcagccttt tttctcctgc cacatgaagc 12180
   acttcactga caccctcatc agtgccaaca tagtaagcca gtatacactc cgctagcgct 12240
   gatgtccggc ggtgcttttg ccgttacgca ccaccccgtc agtagctgaa caggagggac 12300
   agctgataga aacagaagcc actggagcac ctcaaaaaca ccatcataca ctaaatcagt 12360
   aagttggcag catcacccga cgcactttgc gccgaataaa tacctgtgac ggaagatcac 12420
   ttcgcagaat aaataaatcc tggtgtccct gttgataccg ggaagccctg ggccaacttt 12480
   tggcgaaaat gagacgttga tcggcacgta agaggttcca actttcacca taatgaaata 12540
   agatcactac cgggcgtatt ttttgagtta tcgagatttt caggagctaa ggaagctaaa 12600
   atggagaaaa aaatcactgg atataccacc gttgatatat cccaatggca tcgtaaagaa 12660
   cattttgagg catttcagtc agttgctcaa tgtacctata accagaccgt tcagctggat 12720
   attacggcct ttttaaagac cgtaaagaaa aataagcaca agttttatcc ggcctttatt 12780
   cacattcttg cccgcctgat gaatgctcat ccggaattcc gtatggcaat gaaagacggt 12840
   gagctggtga tatgggatag tgttcaccct tgttacaccg ttttccatga gcaaactgaa 12900
   acgttttcat cgctctggag tgaataccac gacgatttcc ggcagtttct acacatatat 12960
   tcgcaagatg tggcgtgtta cggtgaaaac ctggcctatt tccctaaagg gtttattgag 13020
   aatatgtttt tcgtctcagc caatccctgg gtgagtttca ccagttttga tttaaacgtg 13080
   gccaatatgg acaacttctt cgcccccgtt ttcaccatgg gcaaatatta tacgcaaggc 13140
   gacaaggtgc tgatgccgct ggcgattcag gttcatcatg ccgtctgtga tggcttccat 13200
   gtcggcagaa tgcttaatga attacaacag tactgcgatg agtggcaggg cggggcgtaa 13260
   tttttttaag gcagttattg gtgcccttaa acgcctggtg ctacgcctga ataagtgata 13320
   ataagcggat gaatggcaga aattcgaaag caaattcgac ccggtcgtcg gttcagggca 13380
   gggtcgttaa atagccgctt atgtctattg ctggtttacc ggtttattga ctaccggaag 13440
   cagtgtgacc gtgtgcttct caaatgcctg aggccagttt gctcaggctc tccccgtgga 13500
   ggtaataatt gacgatatga tcatttattc tgcctcccag agcctgataa aaacggttag 13560
   cgcttcgtta atacagatgt aggtgttcca cagggtagcc agcagcatcc tgcgatgcag 13620
   atccggaaca taatggtgca gggcgcttgt ttcggcgtgg gtatggtggc aggccccgtg 13680
   gccgggggac tgttgggcgc tgccggcacc tgtcctacga gttgcatgat aaagaagaca 13740
   gtcataagtg cggcgacgat agtcatgccc cgcgcccacc ggaaggagct accggacagc 13800
   ggtgcggact gttgtaactc agaataagaa atgaggccgc tcatggcgtt gactctcagt 13860
   catagtatcg tggtatcacc ggttggttcc actctctgtt gcgggcaact tcagcagcac 13920
   gtaggggact tccgcgtttc cagactttac gaaacacgga aaccgaagac cattcatgtt 13980
   gttgctcagg tcgcagacgt tttgcagcag cagtcgcttc acgttcgctc gcgtatcggt 14040
   gattcattct gctaaccagt aaggcaaccc cgccagccta gccgggtcct caacgacagg 14100
   agcacgatca tgcgcacccg tggccaggac ccaacgctgc ccgagatgcg ccgcgtgcgg 14160
   ctgctggaga tggcggacgc gatggatatg ttctgccaag ggttggtttg cgcattcaca 14220
   gttctccgca agaattgatt ggctccaatt cttggagtgg tgaatccgtt agcgaggtgc 14280
   cgccggcttc cattcaggtc gaggtggccc ggctccatgc accgcgacgc aacgcgggga 14340
   ggcagacaag gtatagggcg gcgcctacaa tccatgccaa cccgttccat gtgctcgccg 14400
   agg 14403
<210> 14
   <211> 5664
   <212> DNA
   <213> Artificial Sequence
<220>
   <223> vector
<400> 14
   ccctttcgtc ttcgaataaa tacctgtgac ggaagatcac ttcgcagaat aaataaatcc      60
   tggtgtccct gttgataccg ggaagccctg ggccaacttt tggcgaaaat gagacgttga     120
   tcggcacgta agaggttcca actttcacca taatgaaata agatcactac cgggcgtatt     180
   ttttgagtta tcgagatttt caggagctaa ggaagctaaa atggagaaaa aaatcactgg      240
   atataccacc gttgatatat cccaatggca tcgtaaagaa cattttgagg catttcagtc      300
   agttgctcaa tgtacctata accagaccgt tcagctggat attacggcct ttttaaagac      360
   cgtaaagaaa aataagcaca agttttatcc ggcctttatt cacattcttg cccgcctgat      420
   gaatgctcat ccggaattcc gtatggcaat gaaagacggt gagctggtga tatgggatag      480
   tgttcaccct tgttacaccg ttttccatga gcaaactgaa acgttttcat cgctctggag      540
   tgaataccac gacgatttcc ggcagtttct acacatatat tcgcaagatg tggcgtgtta      600
   cggtgaaaac ctggcctatt tccctaaagg gtttattgag aatatgtttt tcgtctcagc      660
   caatccctgg gtgagtttca ccagttttga tttaaacgtg gccaatatgg acaacttctt      720
   cgcccccgtt ttcaccatgg gcaaatatta tacgcaaggc gacaaggtgc tgatgccgct      780
   ggcgattcag gttcatcatg ccgtttgtga tggcttccat gtcggcagaa tgcttaatga      840
   attacaacag tactgcgatg agtggcaggg cggggcgtaa tttttttaag gcagttattg      900
   gtgcccttaa acgcctggtg ctacgcctga ataagtgata ataagcggat gaatggcaga      960
   aattcgaaag caaattcgac ccggtcgtcg gttcagggca gggtcgttaa atagccgctt     1020
   atgtctattg ctggtttatc ggtacccggg gatcgcggcc gcggaccgga tcctctagag     1080
   cggccgcgat cctctagagt cgaccggtgg cgaatgggac gcgccctgta gcggcgcatt     1140
   aagcgcggcg ggtgtggtgg ttacgcgcag cgtgaccgct acacttgcca gcgccctagc     1200
   gcccgctcct ttcgctttct tcccttcctt tctcgccacg ttcgccggct ttccccgtca     1260
   agctctaaat cgggggctcc ctttagggtt ccgatttagt gctttacggc acctcgaccc     1320
   caaaaaactt gattagggtg atggttcacg tagtgggcca tcgccctgat agacggtttt     1380
   tcgccctttg acgttggagt ccacgttctt taatagtgga ctcttgttcc aaactggaac     1440
   aacactcaac cctatctcgg tctattcttt tgatttataa gggattttgc cgatttcggc     1500
   ctattggtta aaaaatgagc tgatttaaca aaaatttaac gcgaatttta acaaaatatt     1560
   aacgcttaca atttaggtgg cacttttcgg ggaaatgtgc gcggaacccc tatttgttta     1620
   tttttctaaa tacattcaaa tatgtatccg ctcatcagat cctttttaac ccatcacata     1680
   tacctgccgt tcactattat ttagtgaaat gagatattat gatattttct gaattgtgat     1740
   taaaaaggca actttatgcc catgcaacag aaactataaa aaatacagag aatgaaaaga     1800
   aacagataga ttttttagtt ctttaggccc gtagtctgca aatcctttta tgattttcta   1860
   tcaaacaaaa gaggaaaata gaccagttgc aatccaaacg agagtctaat agaatgaggt     1920
   cgaaaagtaa atcgcgcggg tttgttactg ataaagcagg caagacctaa aatgtgtaaa     1980
   gggcaaagtg tatactttgg cgtcacccct tacatatttt aggtcttttt ttattgtgcg     2040
   taactaactt gccatcttca aacaggaggg ctggaagaag cagaccgcta acacagtaca 2100
   taaaaaagga gacatgaacg atgaacatca aaaagtttgc aaaacaagca acagtattaa 2160
   cctttactac cgcactgctg gcaggaggcg caactcaagc gtttgcgaaa gaaacgaacc 2220
   aaaagccata taaggaaaca tacggcattt cccatattac acgccatgat atgctgcaaa 2280
   tccctgaaca gcaaaaaaat gaaaaatatc aagttcctga ttcgtccaca attaaaaata 2340
   tctcttctgc aaaaggcctg gacgtttggg acagctggcc attacaaaac gctgacggca 2400
   ctgtcgcaaa ctatcacggc taccacatcg tctttgcatt agccggagat cctaaaaatg 2460
   cggatgacac atcgatttac atgttctatc aaaaagtcgg cgaaacttct attgacagct 2520
   ggaaaaacgc tggccgcgtc tttaaagaca gcgacaaatt cgatgcaaat gattctatcc 2580
   taaaagacca aacacaagaa tggtcaggtt cagccacatt tacatctgac ggaaaaatcc 2640
   gtttattcta cactgatttc tccggtaaac attacggcaa acaaacactg acaactgcac 2700
   aagttaacgt atcagcatca gacagctctt tgaacatcaa cggtgtagag gattataaat 2760
   caatctttga cggtgacgga aaaacgtatc aaaatgtaca gcagttcatc gatgaaggca 2820
   actacagctc aggcgacaac catacgctga gagatcctca ctacgtagaa gataaaggcc 2880
   acaaatactt agtatttgaa gcaaacactg gaactgaaga tggctaccaa ggcgaagaat 2940
   ctttatttaa caaagcatac tatggcaaaa gcacatcatt cttccgtcaa gaaagtcaaa 3000
   aacttctgca aagcgataaa aaacgcacgg ctgagttagc aaacggcgct ctcggtatga 3060
   ttgagctaaa cgatgattac acactgaaaa aagtgatgaa accgctgatt gcatctaaca 3120
   cagtaacaga tgaaattgaa cgcgcgaacg tctttaaaat gaacggcaaa tggtacctgt 3180
   tcactgactc ccgcggatca aaaatgacga ttgacggcat tacgtctaac gatatttaca 3240
   tgcttggtta tgtttctaat tctttaactg gcccatacaa gccgctgaac aaaactggcc 3300
   ttgtgttaaa aatggatctt gatcctaacg atgtaacctt tacttactca cacttcgctg 3360
   tacctcaagc gaaaggaaac aatgtcgtga ttacaagcta tatgacaaac agaggattct 3420
   acgcagacaa acaatcaacg tttgcgccaa gcttcctgct gaacatcaaa ggcaagaaaa 3480
   catctgttgt caaagacagc atccttgaac aaggacaatt aacagttaac aaataaaaac 3540
   gcaaaagaaa atgccgatcc ggtttattga ctaccggaag cagtgtgacc gtgtgcttct 3600
   caaatgcctc aggctgtcta tgtgtgactg ttgagctgta acaagttgtc tcaggtgttc 3660
   aatttcatgt tctagttgct ttgttttact ggtttcacct gttctattag gtgttacatg 3720
   ctgttcatct gttacattgt cgatctgttc atggtgaaca gctttaaatg caccaaaaac 3780
   tcgtaaaagc tctgatgtat ctatcttttt tacaccgttt tcatctgtgc atatggacag 3840
   ttttcccttt gatatctaac ggtgaacagt tgttctactt ttgtttgtta gtcttgatgc 3900
   ttcactgata gatacaagag ccataagaac ctcagatcct tccgtattta gccagtatgt 3960
   tctctagtgt ggttcgttgt ttttgcgtga gccatgagaa cgaaccattg agatcatgct 4020
   tactttgcat gtcactcaaa aattttgcct caaaactggt gagctgaatt tttgcagtta 4080
   aagcatcgtg tagtgttttt cttagtccgt tacgtaggta ggaatctgat gtaatggttg 4140
   ttggtatttt gtcaccattc atttttatct ggttgttctc aagttcggtt acgagatcca 4200
   tttgtctatc tagttcaact tggaaaatca acgtatcagt cgggcggcct cgcttatcaa 4260
   ccaccaattt catattgctg taagtgttta aatctttact tattggtttc aaaacccatt 4320
   ggttaagcct tttaaactca tggtagttat tttcaagcat taacatgaac ttaaattcat 4380
   caaggctaat ctctatattt gccttgtgag ttttcttttg tgttagttct tttaataacc 4440
   actcataaat cctcatagag tatttgtttt caaaagactt aacatgttcc agattatatt 4500
   ttatgaattt ttttaactgg aaaagataag gcaatatctc ttcactaaaa actaattcta 4560
   atttttcgct tgagaacttg gcatagtttg tccactggaa aatctcaaag cctttaacca 4620
   aaggattcct gatttccaca gttctcgtca tcagctctct ggttgcttta gctaatacac 4680
   cataagcatt ttccctactg atgttcatca tctgagcgta ttggttataa gtgaacgata 4740
   ccgtccgttc tttccttgta gggttttcaa tcgtggggtt gagtagtgcc acacagcata 4800
   aaattagctt ggtttcatgc tccgttaagt catagcgact aatcgctagt tcatttgctt 4860
   tgaaaacaac taattcagac atacatctca attggtctag gtgattttaa tcactatacc 4920
   aattgagatg ggctagtcaa tgataattac tagtcctttt cctttgagtt gtgggtatct 4980
   gtaaattctg ctagaccttt gctggaaaac ttgtaaattc tgctagaccc tctgtaaatt 5040
   ccgctagacc tttgtgtgtt ttttttgttt atattcaagt ggttataatt tatagaataa 5100
   agaaagaata aaaaaagata aaaagaatag atcccagccc tgtgtataac tcactacttt 5160
   agtcagttcc gcagtattac aaaaggatgt cgcaaacgct gtttgctcct ctacaaaaca 5220
   gaccttaaaa ccctaaaggc ttaagtagca ccctcgcaag ctcgggcaaa tcgctgaata 5280
   ttccttttgt ctccgaccat caggcacctg agtcgctgtc tttttcgtga cattcagttc 5340
   gctgcgctca cggctctggc agtgaatggg ggtaaatggc actacaggcg ccttttatgg 5400
   attcatgcaa ggaaactacc cataatacaa gaaaagcccg tcacgggctt ctcagggcgt 5460
   tttatggcgg gtctgctatg tggtgctatc tgactttttg ctgttcagca gttcctgccc 5520
   tctgattttc cagtctgacc acttcggatt atcccgtgac aggtcattca gactggctaa 5580
   tgcacccagt aaggcagcgg tatcatcaac aggcttaccc gtcttactgt cggatcgacg 5640
   ctctccctta tgcgactcct gcat 5664

## Beschreibung

Die Erfindung betrifft ein Verfahren zur Umsetzung eines Carbonsäureesters der Formel (I)

R¹-A-COOR² (I),

wobei R¹ aus der Gruppe ausgewählt ist, die aus Wasserstoff, -CH₂OH, -CHO, -COOR³, - CH₂SH, -CH₂OR³ und -CH₂NH₂ besteht,
wobei R² aus der Gruppe ausgewählt ist, die aus Alkyl, bevorzugt Methyl, Ethyl und Propyl, besteht,
wobei R³ aus der Gruppe ausgewählt ist, die aus Wasserstoff und Alkyl, bevorzugt Methyl, Ethyl und Propyl, besteht
und wobei es sich bei A um einen nicht substituierten, linearen, Alkylen- oder Alkenylenrest mit wenigstens 4, bevorzugter 6, noch bevorzugter 8 Kohlenstoffatomen handelt,
mittels einer Zelle, umfassend den Schritt
a) Kontaktieren der Zelle mit dem Carbonsäureester in einer wässrigen Lösung, wobei es sich bei der Zelle um eine rekombinante Zelle handelt, bei der die Aktivität eines Polypeptides umfassend SEQ ID NO 2 oder eine Variante davon, wobei die Variante im Wesentlichen die gleiche enzymatische Aktivität des Polypeptides umfassend SEQ ID NO 2 aufweist und die Variante eine Homologie mit SEQ ID NO 2 von mindestens 90% aufweist, gegenüber dem Wildtyp der Zelle verringert ist.

Die Biotechnologie beschäftigt sich mit der Herstellung u. a. von Feinchemikalien unter Einsatz von verschiedenen Organismen, die interessante Synthesefähigkeiten besitzen. Gegenüber herkömmlichen chemischen Verfahren weisen biotechnologische Verfahren eine Reihe von Vorteilen auf. Zunächst verzichten sie in der Regel komplett auf gesundheitsgefährende Stoffe wie schwermetallbasierte Katalysatoren und Reaktionsbedingungen mit extremen pH-, Druck-und Temperaturwerten. Weiterhin kommt man beim Aufbau der für das biotechnologische Verfahren erforderlichen Infrastruktur vielfach mit geringeren Investitionen und Sicherheitsmaßnahmen aus. Die Selektivität und Spezifität von biotechnologisch relevanten Enzymen übersteigt die von chemischen Katalysatoren oft erheblich, so dass die Bildung unerwünschter und vom Produkt häufig schlecht abtrennbarer Nebenprodukte vermindert werden kann, die bei einer Synthese unter Verwendung synthetisch-organischer Verfahren notwendigerweise entstehen würden. Schließlich akzeptieren biotechnologisch relevante Organismen als Edukte in vielen Fällen Verbindungen wie komplexe Kohlenhydrate, die aus nachwachsenden Rohstoffen gewonnen werden können. Die Abhängigkeit eines produzierenden Unternehmens von fossilen Rohstoffen wie Erdöl und Erdgas kann somit verringert werden.

Die Etablierung biotechnologischer Verfahren ist jedoch mit erheblichen Schwierigkeiten verbunden, die dazu führen, dass heutzutage nur sehr wenige Stoffe im Industriemaßstab biotechnologisch hergestellt werden. Das Hauptproblem besteht darin, dass eine Zelle mit einer gewünschten Syntheseaktivität nicht nur das eine, für diese Syntheseaktivität verantwortliche Enzym aufweist, sondern vielmehr tausende Enzyme, die in der gleichen Zelle koexistieren und mit einander um Substrate konkurrieren oder gar komplett gegenläufige Reaktionen katalysieren. So sind allein im Genom von *Escherichia coli ca.* 80 mit bioinformatischen Verfahren als Hydrolasen identifizierte Polypeptide kodiert, d. h. Enzyme, die bestimmte Bindungen unter Verbrauch eines Wassermoleküls spalten. Unter welchen Bedingungen ein Enzym von der Zelle hergestellt werden und welche Reaktionen mit welchen Substraten es katalysiert, ist tatsächlich nur in wenigen Fällen erschöpfend geklärt. Die gezielte Auswahl eines Enzyms zur Katalyse einer bestimmten Reaktion ist damit in vielen Fällen nicht möglich.

Dementsprechend besteht beim Einsatz von Zellen als Biokatalysatoren statt chemischer oder isolierter biologischer Katalysatoren auch immer die Gefahr, dass ein von einem mit einer erwünschten Aktivität ausgestatteten Enzym hergestelltes Produkt oder Zwischenprodukt oder bereits das ursprüngliche Edukt von einem anderen Enzym in ein unerwünschtes Nebenprodukt umgewandelt wird. Ob dies geschieht und welches der zahlreichen Enzyme in diesem Fall die unerwünschte Aktivität aufweist, lässt sich trotz technischer Fortschritte auf dem Gebiet der Bioinformatik nicht vorhersehen.

Gerade bei chemisch reaktiven Stoffen, die industriell als reaktionsfähige Edukte für die Herstellung komplexerer Produkte begehrt sind, ist es nicht unwahrscheinlich, dass sie im Inneren der Zelle mit essentiellen Bestandteilen des Organismus reagieren und somit toxisch wirken. Ist das der Fall, so ist die Wachstums- und Synthesefähigkeit des Organismus bis hin zum Absterben der Zelle beeinträchtigt, ohne dass der Entwickler die Toxizität unmittelbar erkennen könnte. Welcher Organismus welche Konzentration an einem chemisch reaktiven Stoff verträgt, ist ebenfalls nicht vorhersehbar.

Bei Verfahren mit mehreren, jeweils von einem Enzym katalysierten Reaktionen erschwert die Komplexität des Systems die Suche nach Ausbeute oder Reinheit limitierenden Faktoren. Ist die Ausbeute an Produkt zu niedrig, so kann dies daran liegen, dass eines der Enzyme in einer zu niedrigen Konzentration vorhanden ist, ohne dass bekannt wäre, um welches der in Frage kommenden Enzyme es sich dabei handelt, d. h. das Edukt wird im vorgesehenen Zeitrahmen oder vor dem Abbau durch konkurrierende Enzyme aufgrund unzureichender Synthesekapazität nicht umgesetzt. Alternativ ist es durchaus möglich, dass ein Enzym zwar nachweisbar in der Zelle in Form eines Polypeptids vorhanden ist, aber gerade in dieser Zelle nicht die für die Aktivität essentielle Faltung aufweist oder ein bis dato unbekannter, für die Aktivität aber essentieller Cofaktor fehlt. Gleichermaßen kann, wie bereits erwähnt, das Stoffwechselprodukt für die Zelle toxisch sein oder abgebaut werden.

Der Fachmann, der ein biotechnologisches Verfahren etablieren oder verbessern möchte, sieht sich also mit zahlreichen möglichen Ansatzpunkten konfrontiert, erhält aus dem Stand der Technik in den meisten Fällen aber keine konkrete und umsetzbare Anweisung, an welchem dieser Ansatzpunkte er ansetzten muss, um zum Ziel zu gelangen.

Carbonsäureester stellen eine Gruppe industriell stark nachgefragter Verbindungen dar, die entweder selbst oder in Form von weiterverarbeiteten Produkten als Pharmazeutika, Kosmetika, Kunststoffe und dergleichen Verwendung finden.

Häufig wird für eine Verarbeitung jedoch nicht nur eine zunächst in ein Vorprodukt einzuführende Esterfunktion benötigt, sondern es muss am Ester eine weitere Derivatisierung durchgeführt werden, ohne dass die Esterfunktion dabei oder in der Folge hydrolysiert wird. Letzteres ist nicht trivial zu bewerkstelligen, da viele Carbonsäureester insbesondere in wässrigen Lösungen und bei vom Neutralpunkt stark abweichenden pH-Werten selbst in Abwesenheit derartige Reaktionen katalysierender Enzyme schon zur Hydrolyse neigen.

Eine wichtige Möglichkeit, Carbonsäureester weiter zu derivatisieren, besteht in der Oxidation darin enthaltener Alkylketten. Dabei entsteht zunächst ein Alkohol, der entweder als solcher eingesetzt oder zum Aldehyd oder Keton weiteroxidiert werden kann. Das Aldehyd oder Keton kann entweder reduktiv aminiert werden oder zur Carbonsäure weiteroxidiert werden, die bei Bedarf ihrerseits wieder verestert werden kann.

Diese vielfältigen Umsetzungsmöglichkeiten, von denen viele durch endogene, d. h. natürlich in einem Organismus vorhandene Enzyme katalysiert werden, zeigen, dass das Problem der ungesteuerten Nebenproduktbildung oder Verstoffwechselung bei der Umsetzung von Carbonsäureestern mittels biotechnologischer Verfahren besonders schwer wiegt.

Ein Beispiel für einen industriell stark nachgefragten Carbonsäureester, der herkömmlich ausgehend von im Erdöl enthaltenen Kohlenwasserstoffen hergestellt wird, ist 12-Aminolaurinsäuremethylester (ALSME). ALSME ist ein wichtiges Ausgangsprodukt bei der Herstellung von Polymeren, beispielsweise zur Herstellung von Leitungssystemen auf Nylonbasis. Bislang wird ALSME in einem Prozess mit niedriger Ausbeute ausgehend von fossilen Rohstoffen hergestellt.

Ein vielversprechender neuer Weg zur biotechnologischen Herstellung von ALS bzw. ALSME ist in der WO 2009/077461 beschrieben.

Es wird ein Carbonsäureester der Formel (I) mittels einer Zelle in einem Verfahren umgesetzt, umfassend den Schritt
a) Kontaktieren der Zelle mit dem Carbonsäureester in einer wässrigen Lösung.

Dabei wird Laurinsäuremethylester in einem ersten Schritt von einer Monooxygenase oxidiert, und das entstehende Aldehyd wird mittels einer Transaminase zum ALSME umgesetzt. Ein Nachteil dieses Verfahrens besteht darin, dass Nebenprodukte, beispielsweise die Dicarbonsäure, entstehen, die vom dem erwünschten Produkt ALSME nur unter Schwierigkeiten abgetrennt werden können. Das vermindert die Ausbeute und erschwert das Recycling von hydrophoben Lösungsmitteln und hydrophoben flüssigen Kationenaustauschern, die gemäß der WO 2012/110124 zur Abtrennung des Produktes aus der wässrigen Reaktionsmischung verwendet werden können, auf Kosten der Effizienz bei der Ressourcennutzung.

Die WO 2007/139871 offenbart ein Verfahren zur Umsetzung des Carbonsäureesters Dimethylbutyryl-S-Methyl-Mercaptopropionat (DMB-S-MMP) mittels einer Zelle, umfassend den Schritt
a) Kontaktieren der Zelle mit dem Carbonsäureester in einer wässrigen Lösung,
   wobei es sich bei der Zelle um eine rekombinante Zelle handelt, bei der die Aktivität eines Polypeptides umfassend SEQ ID NO 2 gegenüber dem Wildtyp der Zelle verringert ist.

Vor diesem Hintergrund besteht die der Erfindung zu Grunde liegende Aufgabe darin, ein mit Hinblick auf Ausbeute, Kohlenstoff- und/oder Stickstoffbilanz und/oder Reinheit möglichst effizientes biotechnologisches Verfahren zur Umsetzung von Carbonsäureestern bereitzustellen.

Eine weitere der Erfindung zu Grunde liegende Aufgabe besteht darin, ein mit Hinblick auf Ausbeute, Kohlenstoff- und/oder Stickstoffbilanz, Wiederverwendbarkeit verwendeter Agenzien und/oder Reinheit des Produktes möglichst effizientes biotechnologisches Verfahren zur Umsetzung von Carbonsäureestern zu aminierten Carbonsäureestern bereitzustellen. In diesem Zusammenhang wird unter einer effizienten Kohlenstoff- und/oder Stickstoffbilanz verstanden, dass sich ein möglichst hoher Anteil des zur Umsetzung eines Carbonsäureesters in Form von geeigneten Substraten an eine Zelle verfütterten Kohlenstoffs und/oder Stickstoffs im erwünschten Endprodukt wiederfindet, anstatt beispielsweise zu anderen Produkten als den erwünschten umgesetzt zu werden.

Eine weitere der Erfindung zu Grunde liegende Aufgabe besteht darin, die Aufarbeitbarkeit einer mehrphasigen Reaktionsmischung aus der Umsetzung eines Carbonsäureesters zu verbessern, besonders mit Hinblick auf die Wiederverwendbarkeit zur Aufarbeitung verwendeter hydrophober Lösungsmittel und flüssiger Kationenaustauscher, sowie mit Hinblick auf die Phasenbildung und -trennung bei einem biphasischen System umfassend eine wässrige Phase, in der die Umsetzung des Carbonsäureesters abläuft, und eine organische Phase mit organischen Lösungsmitteln und/oder flüssigen Kationenaustauschern.

Diese und weitere Aufgaben werden durch den Gegenstand der beigefügten unabhängigen Ansprüche gelöst, wobei sich Ausführungsformen aus den Unteransprüchen ergeben. In einem ersten Aspekt wird das der Erfindung zu Grunde liegende Problem gelöst durch ein Verfahren zur Umsetzung eines Carbonsäureesters der Formel (I)

R¹ - A - COOR² (I),

wobei R¹ aus der Gruppe ausgewählt ist, die aus Wasserstoff, -CH₂OH, -CHO, -COOR³, - CH₂SH, -CH₂OR³ und -CH₂NH₂ besteht,
wobei R² aus der Gruppe ausgewählt ist, die aus Alkyl, bevorzugt Methyl, Ethyl und Propyl, besteht,
wobei R³ aus der Gruppe ausgewählt ist, die aus Wasserstoff und Alkyl, bevorzugt Methyl, Ethyl und Propyl, besteht
und wobei es sich bei A um einen nicht substituierten, linearen, Alkylen- oder Alkenylenrest mit wenigstens 4, bevorzugter 6, noch bevorzugter 8 Kohlenstoffatomen handelt,
mittels einer Zelle, umfassend den Schritt
a) Kontaktieren der Zelle mit dem Carbonsäureester in einer wässrigen Lösung, wobei es sich bei der Zelle um eine rekombinante Zelle handelt, bei der die Aktivität eines Polypeptides umfassend SEQ ID NO 2 oder eine Variante davon, wobei die Variante im Wesentlichen die gleiche enzymatische Aktivität des Polypeptides umfassend SEQ ID NO 2 aufweist und die Variante eine Homologie mit SEQ ID NO 2 von mindestens 90% aufweist, gegenüber dem Wildtyp der Zelle verringert ist.

In einem zweiten Aspekt wird das der Erfindung zu Grunde liegende Problem gelöst durch eine Verwendung eines Knockouts eines für ein Polypeptid umfassend SEQ ID NO 2 oder eine Variante davon kodierenden Gens als Teil der genetischen Ausstattung einer rekombinanten Zelle zur Erhöhung der Produktion eines Carbonsäureesters der Formel (I)

R¹ - A - COOR² (I),

wobei R¹ aus der Gruppe ausgewählt ist, die aus Wasserstoff, -CH₂OH, -CHO, -COOR³, - CH₂SH, -CH₂OR³ und -CH₂NH₂ besteht,
wobei R² aus der Gruppe ausgewählt ist, die aus Alkyl, bevorzugt Methyl, Ethyl und Propyl, besteht,
wobei R³ aus der Gruppe ausgewählt ist, die aus Wasserstoff und Alkyl, bevorzugt Methyl, Ethyl und Propyl, besteht
und wobei es sich bei A um einen nicht substituierten, linearen, Alkylen- oder Alkenylenrest mit wenigstens 4, bevorzugter 6, noch bevorzugter 8 Kohlenstoffatomen handelt,
und wobei die Variante im Wesentlichen die gleiche enzymatische Aktivität des Polypeptides umfassend SEQ ID NO 2 aufweist und die Variante eine Homologie mit SEQ ID NO 2 von mindestens 90% aufweist.

In einem dritten Aspekt wird das der Erfindung zu Grunde liegende Problem gelöst durch die Verwendung einer rekombinanten Zelle, bei der die Aktivität eines Polypeptides mit der SEQ NO 2 oder einer Variante davon, wobei die Variante im Wesentlichen die gleiche enzymatische Aktivität des Polypeptides umfassend SEQ ID NO 2 aufweist, und die Variante eine Homologie mit SEQ ID NO 2 von mindestens 90% aufweist, gegenüber dem Wildtyp der Zelle verringert ist, zur Umsetzung eines Carbonsäureesters der Formel (I)

R¹ - A - COOR² (I),

wobei R¹ aus der Gruppe ausgewählt ist, die aus Wasserstoff, -CH₂OH, -CHO, -COOR³, - CH₂SH, -CH₂OR³ und -CH₂NH₂ besteht,
wobei R² aus der Gruppe ausgewählt ist, die aus Alkyl, bevorzugt Methyl, Ethyl und Propyl, besteht,
wobei R³ aus der Gruppe ausgewählt ist, die aus Wasserstoff und Alkyl, bevorzugt Methyl, Ethyl und Propyl, besteht
und wobei es sich bei A um einen nicht substituierten, linearen, Alkylen- oder Alkenylenrest mit wenigstens 4, bevorzugter 6, noch bevorzugter 8 Kohlenstoffatomen handelt.

In einer Ausführungsform des ersten, zweiten oder dritten Aspekts wird das Problem gelöst durch ein Verfahren oder eine Verwendung, wobei es sich bei A um einen gesättigten Alkylenrest handelt, bevorzugt um einen Alkylenrest der Formel -(CH₂)ₙ, wobei n wenigstens 4 ist.

In einer weiteren Ausführungsform des ersten Aspekts wird das Problem gelöst durch ein Verfahren, wobei R¹ aus der Gruppe ausgewählt ist, die aus Wasserstoff, -CH₂OH, -CHO und -CH₂NH₂ besteht.

In einer bevorzugten Ausführungsform des ersten bis dritten Aspekts wird das Problem gelöst durch ein Verfahren oder eine Verwendung, wobei die Zelle weiterhin eine Transaminase exprimiert.

In einer bevorzugten Ausführungsform des ersten bis dritten Aspekts wird das Problem gelöst durch ein Verfahren oder eine Verwendung, wobei die Zelle weiterhin eine Alanindehydrogenase exprimiert.

In einer bevorzugten Ausführungsform des ersten bis dritten Aspekts wird das Problem gelöst durch ein Verfahren oder eine Verwendung, wobei die Zelle weiterhin ein Protein aus der AlkL-Familie aufweist.

In einer bevorzugten Ausführungsform des ersten bis dritten Aspekts wird das Problem gelöst durch ein Verfahren oder eine Verwendung, wobei die Zelle eine ihrem Wildtyp gegenüber verringerte Aktivität wenigstens eines Enzyms aufweist, das eine der Reaktionen der β-Oxidation von Fettsäuren katalysiert, wobei es sich bevorzugt um ein Enzym aus der Gruppe handelt, die Fettsäure-CoA-Ligase, Acyl-CoA-Dehydrogenase, 2,4-Dienoyl-CoA-Reduktase, Enoyl-CoA-Hydratase and 3-Ketoacyl-CoA-Thiolase, einen Fettsäureimporter oder Varianten davon, wobei die Varianten im Wesentlichen die gleiche enzymatische Aktivität der Fettsäure-CoA-Ligase, der Acyl-CoA-Dehydrogenase, der 2,4-Dienoyl-CoA-Reduktase, der Enoyl-CoA-Hydratase, der 3-Ketoacyl-CoA-Thiolase oder des Fettsäureimporters aufweisen, umfasst, besonders bevorzugt um FadL oder eine Variante davon, wobei die Variante im Wesentlichen die gleiche enzymatische Aktivität des FadL aufweist.

In einer bevorzugten Ausführungsform des ersten bis dritten Aspekts wird das Problem gelöst durch ein Verfahren oder eine Verwendung, wobei die Zelle wenigstens ein Enzym aus der Gruppe bestehend aus Alkanhydroxylase, Alkoholdehydrogenase, Transaminase, Alanindehydrogenase und Protein aus der AlkL-Familie in rekombinanter Form aufweist und/oder überexprimiert.

In einer bevorzugten Ausführungsform des ersten bis dritten Aspekts wird das Problem gelöst durch ein Verfahren oder eine Verwendung, wobei die Aktivität eines Polypeptides umfassend SEQ ID NO 2 oder eine Variante davon gegenüber dem Wildtyp der Zelle durch Knock out eines für ein Polypeptid umfassend SEQ ID NO 2 oder eine Variante davon kodierenden Gens verringert ist.

Die Erfindung beruht auf der überraschenden Erkenntnis der Erfinder, dass eine rekombinante Zelle, bei der die Aktivität eines Polypeptides umfassend SEQ ID NO 2 besonders oder einer Variante davon gegenüber dem Wildtyp der Zelle verringert ist, dahingehend für die Umsetzung von Carbonsäureestern geeignet ist, dass die Ausbeute, Kohlenstoff- und oder Stickstoffbilanz und Reinheit der daraus hervorgehenden Produkte überraschend höher ist als bei einer Zelle, die mit Hinblick auf das Polypeptid umfassend SEQ ID NO 2 die gleiche Aktivität wie der Wildtyp der Zelle aufweist. Dies trifft beispielsweise auf Reaktionen zu, bei denen der Carbonsäureester unter Verwendung von wenigstens einer Alkanhydroxylase und optional weiteren Enzymen oxidiert wird.

Eine weitere überraschende Erkenntnis der Erfinder ist, dass eine rekombinante Zelle, bei der die Aktivität eines Polypeptides umfassend SEQ ID NO 2 oder einer Variante davon gegenüber dem Wildtyp der Zelle verringert ist, besonders dahingehend für die Umsetzung von Carbonsäureestern geeignet ist, dass zur Aufarbeitung des Reaktionsproduktes aus der Umsetzung des Carbonsäureesters verwendete organische Lösungsmittel und flüssige Kationenaustauscher besonders effizient und/oder häufig durch Recycling für eine erneute Verwendung wiedergewonnen werden können, und dass die Abtrennung der wässrigen Reaktionsmischung von einer hydrophoben Lösung umfassend organisches Lösungsmittel und/oder flüssigen Kationenaustauscher verbessert ist.

Die Erfindung betrifft ein Verfahren zur Umsetzung eines Carbonsäureesters mittels einer Zelle, wobei es sich bei der Umsetzung um eine jegliche chemische Reaktion handeln kann, die von dem interessierenden Carbonsäureester als Edukt Gebrauch macht, und bei der die Hydrolyse oder vorzeitige Hydrolyse des Carbonsäureesters oder einer daraus hervorgehenden Verbindung die Ausbeute, Kohlenstoff- und/oder Stickstoffbilanz und/oder Reinheit des daraus herzustellenden Produktes beeinträchtigen könnte. Besonders geeignet ist das erfindungsgemäße Verfahren für Umsetzungen, bei denen eine andere chemische Funktion als die Carbonsäureestergruppe umgesetzt wird, beispielsweise eine endständige Alkylgruppe, und bei denen es gilt, die Carbonsäureestergruppe zu erhalten. Erfindungsgemäß können jedoch auch Reaktionen wie Umesterungen der Carbonsäureestergruppe durchgeführt werden, bei denen es gilt, eine vorzeitige und unspezifische Reaktion der Carbonsäureestergruppe zu vermeiden. Ebenso ist die erfindungsgemäße Lehre für Reaktionen geeignet, bei denen eine die Carbonsäureestergruppe enthaltende Verbindung nicht selbst das umzusetzende Edukt ist, sondern lediglich ihre Anwesenheit und Stabilität über einen längeren Zeitraum erforderlich ist, beispielsweise für den Fall, dass es sich um einen Induktor oder Aktivator eines Enzyms mit einer für das Verfahren essentiellen Aktivität handelt.

Essentiell für die Ausführung der Erfindung ist, dass die in einem erfindungsgemäßen Verfahren verwendete Zelle eine rekombinante Zelle ist, bei der die Aktivität eines Polypeptides umfassend SEQ ID NO 2 oder eine Variante davon gegenüber dem Wildtyp der Zelle verringert ist. Diese Sequenz kodiert für BioH, ein Enzym, das für seine Fähigkeit bekannt ist, als Teil der Biosynthese von Biotin mit dem weiteren Enzym BioC Alanin und/oder Acetat in Pimeloyol-CoA zu überführen (Barker, D. F., and Campbell, A. M. (1980) J. Bacteriol. 143, 789-800). BioH wird in Escherichia coli von einer Nukleotidsequenz umfassend SEQ ID NO 1 kodiert. In einer bevorzugten Ausführungsform ist die Aktivität des Polypeptides umfassend SEQ ID NO 2 durch Knockout, beispielsweise teilweise Deletion, oder andere Maßnahmen zur Verringerung der Expression einer Nukleotidsequenz umfassend SEQ ID NO 1 oder eine Variante davon verringert.

Mit der Entwicklung moderner genetischer, mikrobiologischer und molekularbiologischer Methoden stehen dem Fachmann zahlreiche Werkzeuge zur Verfügung, mit denen er die Aktivität von in lebenden Zellen vorhandenen Polypeptiden routinemäßig messen und beeinflussen kann. Zur Bestimmung der Aktivität eines Enzyms, die in Form einer Suspension, eines Pellets vorliegt oder in prozessierter Form einer Zellkultur entnommen sein kann, können enzymatische Standardtests verwendet und ausgewertet werden, wie es in Lehrbüchern, beispielsweise Cornish-Bowden, 1995, beschrieben ist. Ein Assay zur Bestimmung der Aktivität des Polypeptides umfassend SEQ ID NO 1 oder eine Variante davon ist in X. Xie et al. (2007) Metabolic Engineering 9; 379-386 beschrieben.

Auch routinemäßig anwendbare Verfahren zur Verringerung der Aktivität eines Enzyms in einer Zelle, beispielsweise durch ungerichtete Mutagenese von Zellen durch Exposition gegenüber radioaktiver Strahlung gefolgt von Anreicherung oder Screening der Mutanten, durch ortsgerichtete Einführung von Punktmutationen oder durch Unterbrechung des Leserasters oder Deletion eines Teils des Leserasters eines chromosomal in eine Zelle integrierten für ein aktives Enzym kodierendes Gen sind im Stand der Technik beschrieben, beispielsweise in Maniatis *et al* (1989) oder in Fuchs & Schlegl (2007), und für den Fachmann routinemäßig durchführbar. Auch eine Aktivitätsverringerung auf der Basis von RNA-Interferenz (Tuschl, 2001) oder unter Verwendung von spezifischen Inhibitoren ist möglich. Die Formulierung "wobei die Zelle eine ihrem Wildtyp gegenüber verringerte Aktivität" eines Polypeptids aufweist, bedeutet, wie hierin verwendet, dass die Aktivität des Polypeptids in der veränderten Zelle gegenüber der Aktivität des gleichen Enzyms in einer Wildtyp-Zelle verringert ist. Beispielsweise beträgt die relative Verringerung 5, 10, 20, 40, 50, 75, 90, 95, 99 oder mehr Prozent der Aktivität. Oder es ist keine Aktivität des Enzyms gegenüber dem Hintergrund mehr nachweisbar.

Beispielsweise offenbart ist die Verringerung der Aktivität des Polypeptides umfassend SEQ ID NO 2 oder eine Variante davon durch einen Knock out. Unter dem Begriff "Knock out", wie hierin verwendet, wird eine jegliche Maßnahme verstanden, die die Aktivität des Polypeptides umfassend SEQ ID NO 2 oder eine Variante davon dauerhaft und irreversibel, insbesondere auch bei Nachkommen entsprechender Zellen, verringert, z.B. durch Unterbrechung des Leserasters der für SEQ ID NO 2 oder eine Variante davon kodierenden Sequenz, durch Deletion wenigstens eines Teils der für SEQ ID NO 2 kodierenden Sequenz oder einer Variante davon, die einen Verlust der Enzymaktivität der kodierten Polypeptids bedingt, aber das Leseraster nicht unterbricht, oder durch Mutation einer für die Expression essentiellen Nukleotidsequenz, beispielsweise eines Promotors, einer Ribosomenbindestelle oder dergleichen. Maßnahmen zum Herstellen von Zellen mit verringerten Aktivitäten spezifischer Polypeptide sind dem Fachmann zugängliche Routineverfahren und im Stand der Technik hinlänglich beschrieben, beispielsweise in Kamionka et. al. (2005) Appl Environ Microbiol. 2005 February; 71(2): 728-733, Geng et. al. (2009), Journal of Biomedicine and Biotechnology Volume 2009, Article ID 646380, doi:10.1155/2009/646380, and Murphy (2009) Methods Mol Biol. 2011;765:27-42. Geeignet sind auch kommerziell erhältliche Kits, beispielsweise das TargeTron™ Gene Knockout System von Sigma Aldrich.

Die Erfindung betrifft Umsetzung eines Carbonsäureesters der Formel (I)

R¹ - A - COOR² (I),

wobei R¹ aus der Gruppe ausgewählt ist, die Wasserstoff, -CH₂OH, -CHO, -COOR³, -CH₂SH, -CH₂OR³ und -CH₂NH₂ umfasst, wobei R² aus der Gruppe ausgewählt ist, die Alkyl, bevorzugt Methyl, Ethyl und Propyl, umfasst, wobei R³ aus der Gruppe ausgewählt ist, die Wasserstoff und Alkyl, bevorzugt Methyl, Ethyl und Propyl, umfasst, und wobei es sich bei A um einen nicht substituierten, linearen Alkylen- oder Alkenylenrest mit wenigstens 4, bevorzugter 6, noch bevorzugter 8 Kohlenstoffatomen handelt. Bei dem Alkylenrest A kann es sich, unter der Voraussetzung, dass er wenigstens vier Kohlenstoffatome umfasst, um einen beliebigen linearen Alkylenrest handelt. Beispielsweise handelt es sich bei A um eine Alkylenkette der Formel -(CH₂)ₙ-, wobei n 4, 5, 6, 7, 8, 9, 10, 11, 12, 13, 14, 15, 16, 17, 18, 19, 20, 21, 22, 23, 24, 25, 26, 27, 28, 29 oder 30 sein kann. Beispielsweise stellt A einen Alkylenrest der Formel - (CH₂)ₙ- dar, wobei n 4 bis 24, z.B. 4 bis 22 oder 4 bis 10 ist, R₁ ist Wasserstoff, -CH₂OH, -CHO, oder-COOH, z.B. Wasserstoff, und R² ist Methyl oder Ethyl.

Im Zusammenhang mit Edukten der erfindungsgemäß umgesetzten Carbonsäureester sowie sämtlichen anderen hierin beschriebenen Verbindungen gilt, dass eine mit strukturellen Merkmalen, beispielsweise einer chemischen Formel, bezeichnete Verbindung gleichermaßen protonierte als auch deprotonierte oder andere dissoziierte Verbindungen bezeichnet. Beispielsweise wird unter dem Begriff "Acetat" gleichermaßen die protonierte Form, d. h. Essigsäure, als auch die dissoziierte Form, d.h. CH₃-COO⁻ verstanden.

Bei der erfindungsgemäß einzusetzenden Zelle handelt es sich z.B. um einen Ganzzellkatalysator in Form einer stoffwechselaktiven Zelle, die eine zur Umsetzung des Carbonsäureesters erforderliche enzymatische Aktivität aufweist, z.B. durch Expression eines rekombinanten Enzyms.

Mit Hinblick auf die Wahl des Organismus unterliegt die erfindungsgemäß benutzbare Zelle keinen Einschränkungen, sofern sie kultivierbar, stabil und Verfahren zur Abschwächung von Enzymaktivitäten, beispielsweise Knock outs, zugänglich ist. So kann es sich gleichermaßen um eine prokaryontische oder eukaryontische Zelle handeln. Im Falle einer eukaryontischen Zelle kann es sich um unizelluläre Eukaryonten handeln, z.B. um Hefen wie *Saccharomyces cerevisiae, Candida tropicalis, Candida albicans* und *Pichia pastoris.* Im Falle von prokaryontischen Zellen kann es sich beispielsweise um ein Bakterium handeln, das aus der Gruppe ausgewählt ist, die *Magnetococcus, Mariprofundus, Acetobacter, Acetobacterium, Acidiphilium, Afipia, Ahrensia, Asticcacaulis, Aurantimonas, Azorhizobium, Azospirillum, Bacillus, Bartonella, tribocorum, Beijerinckia, Bradyrhizobium, Brevundimonas, subvibrioides, Brucella, Caulobacter, Chelativorans, Citreicella, Citromicrobium, Clostridium, Corynebacterium, Dinoroseobacter, Erythrobacter, Fulvimarina, Gluconacetobacter, Granulibacter, Hirschia, Hoeflea, Hyphomicrobium, Hyphomonas, Ketogulonicigenium, Labrenzia, Loktanella, Magnetospirillum, Maricaulis, Maritimibacter, Mesorhizobium, Methylobacterium, Methylocystis, Methylosinus, Nitrobacter, Novosphingobium, Oceanibulbus, Oceanicaulis, Oceanicola, Ochrobactrum, Octadecabacter, Oligotropha, Paracoccus, Parvibaculum, Parvularcula, Pelagibaca, Phaeobacter, Phenylobacterium, Polymorphum, Pseudovibrio, Rhodobacter, Rhodomicrobium, Rhodopseudomonas, Rhodospirillum, Roseibium, Roseobacter, Roseomonas, Roseovarius, Ruegeria, Sagittula, Silicibacter, Sphingobium, Sphingomonas, Sphingopyxis, Starkeya, Sulfitobacter, Thalassiobium, Xanthobacter, Zymomonas, Agrobacterium, Rhizobium, Sinorhizobium, Anaplasma, Ehrlichia, Neorickettsia, Orientia, Rickettsia, Wolbachia, Bordetella, Burkholderia, Cupriavidus, Taiwanensis, Lautropia, Limnobacter, Polynucleobacter, Ralstonia, Chromobacterium, Eikenella, corrodens, Basfia, Kingella, Laribacter, Lutiella, Neisseria, Simonsiella, Achromobacter, Acidovorax, Alicycliphilus, Aromatoleum, Azoarcus, Comamonas, Dechloromonas, Delftia, Gallionella, Herbaspirillum, Herminiimonas, Hylemonella, Janthinobacterium, Leptothrix, Methylibium, Methylobacillus, Methylophilales, Methyloversatilis, Methylovorus, Nitrosomonas, Nitrosospira, Oxalobacter, Parasutterella, Polaromonas, Polaromonas, Pusillimonas, Rhodoferax, Rubrivivax, Sideroxydans, Sutterella, wadsworthensis, Taylorella, Thauera, Thiobacillus, Thiomonas, Variovorax, Verminephrobacter, Anaeromyxobacter, Bdellovibrio, bacteriovorus, Bilophila, Desulfarculus, Desulfatibacillum, Desulfobacca, Desulfobacterium, Desulfobulbus, Desulfococcus, Desulfohalobium, Desulfitobacterium, Desulfomicrobium, Desulfonatronospira, Desulfotalea, Desulfovibrio, Desulfuromonas, Geobacter, Haliangium, Hippea, Lawsonia, Myxococcus, Pelobacter, Plesiocystis, Sorangium, Stigmatella, Syntrophobacter, Syntrophus, Arcobacter, Caminibacter, Campylobacter, Helicobacter, Nitratifractor, Nitratiruptor, Sulfuricurvum, Sulfurimonas, Sulfurospirillum, Sulfurovum, Wolinella, Buchnera, Blochmannia, Hamiltonella, Regiella, Riesia, Citrobacter, Cronobacter, Dickeya, Edwardsiella, Enterobacter, Erwinia, Escherichia, Klebsiella, Pantoea, Pectobacterium, Proteus, Providencia, Rahnella, Salmonella, Serratia, Shigella, Sodalis, Wigglesworthia, Glossina, Xenorhabdus, Yersinia, Acidithiobacillus, Acinetobacter, Aeromonas, Alcanivorax, Alkalilimnicola, Allochromatium, Alteromonadales, Alteromonas, Baumannia, Beggiatoa, Bermanella, Carsonella, Ruthia, Vesicomyosocius, Cardiobacterium, Chromohalobacter, Colwellia, Congregibacter, Coxiella, Dichelobacter, Endoriftia, Enhydrobacter, Ferrimonas, Francisella, Glaciecola, Hahella, Halomonas, Halorhodospira, Halothiobacillus, Idiomarina, Kangiella, Legionella, Marinobacter, Marinomonas, Methylobacter, Methylococcus, Methylomicrobium, Methylophaga, Moraxella, Moritella, Neptuniibacter, Nitrococcus, Pseudoalteromonas, Psychrobacter, Psychromonas, Reinekea, Rickettsiella, Saccharophagus, Shewanella, Succinatimonas, Teredinibacter, Thioalkalimicrobium, Thioalkalivibrio, Thiomicrospira, Tolumonas, Vibrionales, Actinobacillus, Aggregatibacter, Gallibacterium, Haemophilus, Histophilus, Mannheimia, Pasteurella, Azotobacter, Cellvibrio, Pseudomonas, Aliivibrio, Grimontia, Photobacterium, Photobacterium, Vibrio, Pseudoxanthomonas, Stenotrophomonas, Xanthomonas, Xylella, Borrelia, Brachyspira, Leptospira, Spirochaeta, Treponema, Hodgkinia, Puniceispirillum, Liberibacter, Pelagibacter, Odyssella, Accumulibacter,* insbesondere B. *subtilis, B. megaterium, C. glutamicum, E. coli, Pseudomonas sp., Pseudomonas fluorescens, Pseudomonas putida, Pseudomonas stutzeri" Acinetobacter* sp., *Burkholderia* sp., *Burkholderia thailandensis,* Cyanobakterien, *Klebsiella* sp., *Klebsiella oxytoca, Salmonella sp., Rhizobium sp.* und *Rhizobium meliloti,* umfasst. Beispielsweise handelt es sich um ein Enterobakterium, z.B. um *Escherichia coli.*

Das erfindungsgemäße Verfahren erfordert, dass die Zelle mit dem Carbonsäureester in einer wässrigen Lösung kontaktiert wird. Beispielsweise wird unter dem Begriff "Kontaktieren", wie hierin verwendet, verstanden, dass die Zelle in unmittelbaren Kontakt mit dem jeweiligen Agens, beispielsweise dem Carbonsäureester oder einem flüssigen Kationenaustauscher gelangt, z.B. ohne dass physikalische Barrieren wie poröse Membranen oder dergleichen zwischengeschaltet sind. Das Kontaktieren geschieht im einfachsten Fall dadurch, dass das Agens, beispielsweise der Carbonsäureester oder der flüssige Kationenaustauscher, zu einer wässrigen Lösung gegeben wird, in der sich die Zelle befindet.

Als wässrige Lösung können sämtliche Lösungen auf Wasserbasis verwendet werden, die zur Erhaltung oder Kultivierung der Zelle und/oder ihrer für die Umsetzung des Carbonsäureesters erforderlichen Aktivität geeignet sind. Darunter fallen gleichermaßen Kulturmedien für Mikroorganismen, darunter Vollmedien wie LB-Medien, Minimalmedien wie M9-Medien sowie Selektivmedien, beispielsweise solche, die eine hohe Salzkonzentration enthalten und daher nur das Wachstum halophiler oder zumindest halotoleranter Organismen ermöglichen.

Beispielsweise ist dies ein Minimalmedium, das möglichst wenige, vom Produkt der Umsetzung des Carbonsäureesters leicht abtrennbare Bestandteile enthält, um die Aufarbeitung des Produkts zu erleichtern.

Sofern das Produkt der vorgesehenen Umsetzung ausreichende Hydrophobizität und eine geeignete Ladung aufweist, bietet es sich an, es in einem Schritt b) durch Kontaktieren der wässrigen Lösung mit einer hydrophoben organischen Lösung umfassend einen Kationenaustauscher zu extrahieren. Geeignete Vorgehensweisen sind in der internationalen Patentanmeldung WO 2012/110124 oder in der europäischen Patentanmeldung EP12181153.3 beschrieben. Kurz gesagt kann die wässrige Lösung während oder nach der Umsetzung mit einer hydrophoben Lösung umfassend einen Fettsäureester als Lösungsmittel und eine Fettsäure, bevorzugt eine ungesättigte Fettsäure, kontaktiert werden.

Bei der Einstellung der Temperatur und der Bedingungen bei Schritt a) sind die Ansprüche der Zelle, der Umsetzungsreaktion und benötigter Enzyme zu beachten. Die Temperaturansprüche verschiedener biotechnologisch bedeutsamer Zellen können mikrobiologischen und molekularbiologischen Lehrbüchern entnommen werden, z. B. Fuchs/Schlegl, Allgemeine Mikrobiologie, 2008, oder durch Wachstumsversuche im Rahmen von Routinearbeiten ermittelt werden. In einer bevorzugten Ausführungsform liegt der pH-Wert des wässrigen Kulturmediums zum Zeitpunkt des Kontaktierens zwischen 4 bis 9, z.B. zwischen 4,5 bis 8,5, oder zwischen 6,5 und 7,5. Beispielsweise liegt die Temperatur zwischen 0 und 45 °C, z.B. zwischen 15 und 40 °C, oder zwischen 20 und 37 °C.

Bevorzugt wird für das erfindungsgemäße Verfahren eine Zelle verwendet, die eine rekombinante Alkanhydroxylase exprimiert, wobei die Aktivität eines Polypeptides umfassend SEQ ID NO 2 oder eine Variante davon gegenüber dem Wildtyp der Zelle verringert ist. Beispielsweise handelt es sich bei der Alkanhydroxylase um eine Cytochrom P450-Monooxygenase der CYP153-Familie. Beispielsweise wird unter dem Begriff "Cytochrom P450-Monooxygenase der CYP153-Familie" eine cytosolische Oxidase verstanden, die Teil eines 3 Komponenten-Systems ist, das weiterhin ein Ferredoxin und eine Ferredoxin-Reduktase umfasst, mit einer Alkan-Bindestelle und der Fähigkeit, Alkane zu hydroxylieren. Beispielsweise handelt es sich um ein Enzym, das zu wenigstens 80, z.B. oder 95 oder 99 Prozent Sequenzidentität zur Cytochrom P450-Monooxygenase der CYP153-Familie aus *Alcanivorax borkumensis* SK2 (Datenbankcode YP_691921) aufweist oder um ein Enzym, das eine Polypeptidsequenz umfasst, die wenigstens 80, z.B. 90, oder 95 oder 99 Prozent Sequenzidentität zur Cytochrom P450-Monooxygenase der CYP153-Familie aus *Alcanivorax borkumensis* SK2 (Datenbankcode YP_691921) aufweist und darüber hinaus Alkanhydroxylase-Aktivität aufweist. Die genannten Datenbankcodes beziehen sich dabei, wie durchgehend in dieser Anmeldung, auf die NCB (National Center for Biotechnology Information, Bethesda, USA)I-Datenbanken, genauer gesagt die am 8. November 2012 online verfügbare Version. Beispielsweise ist unter dem Begriff "Alkanhydroxylase-Aktivität", wie hierin verwendet, die Fähigkeit zu verstehen, die Hydroxylierung von Alkanen oder unsubstituierten linearen Alkylresten umfassend wenigstens sechs, z.B. zwölf Kohlenstoffstoffreste zu katalysieren. Beispielsweise wird unter dem Begriff "Cytochrom P450-Monooxygenase der CYP153-Familie" eine nicht membrangebundene Oxidase verstanden, die eine Bindestelle für Alkane, unsubstituierte lineare Alkylreste umfassend wenigstens fünf, z.B. zwölf Kohlenstoffstoffreste oder einfach hydroxylierte Alkane und deren Polypeptidkette das Motiv LL(I/L)(V/I)GGNDTTRN umfasst. Beispielsweise handelt es sich bei einer "Cytochrom P450-Monooxygenase der CYP153-Familie", wie hierin verwendet, um eine Cytochrom P450-Monooxygenase der CYP153-Familie aus *Alcanivorax borkumensis* SK2 (Datenbankcode YP_691921) oder eine Variante, die beispielsweise Alkanhydroxylaseaktivität aufweist.

Bei den erfindungsgemäß verwendeten Enzymen handelt es sich z.B. um rekombinante Enzyme. Beispielsweise wird unter dem Begriff "rekombinant", wie hierin verwendet, verstanden, dass das entsprechende Nukleinsäure-Molekül in der natürlichen Zelle nicht vorkommt und/oder es unter Verwendung von gentechnischen Methoden hergestellt wurde. Man spricht von einem rekombinanten Protein, wenn das entsprechende Polypeptid von einer rekombinanten Nukleinsäure kodiert ist. Beispielsweise wird unter einer rekombinanten Zelle, wie hierin verwendet, eine Zelle verstanden, die wenigstens eine rekombinante Nukleinsäure oder ein rekombinantes Polypeptid aufweist. Dem Fachmann sind zum Herstellen rekombinanter Moleküle oder Zellen geeignete Verfahren bekannt, beispielsweise die in Sambrook/Fritsch/Maniatis (1989): Molecular Cloning: A Laboratory Manual, Cold Spring Harbor Laboratory Press, 2nd edition, beschriebenen. Rekombinante Enzyme werden z.B. überexprimiert, beispielsweise unter Verwendung von pET- oder pGEX-Vektor-Systemen, die dem Fachmann bekannt sind. Zur optimalen Versorgung der Cytochrom P450-Monooxygenase der CYP153-Familie mit Elektronen aus dem Reduktionsmittel, z.B. NADH, kann die Zelle die Monoxygenase zusammen mit funktionell mit ihr wechselwirkender Ferredoxin-Reduktase und funktionell mit ihr wechselwirkendem Ferredoxin exprimieren. Dabei kann es sich um isolierte oder bei der Verwendung eines Ganzzellkatalysators um co-exprimierte Polypeptide oder um N-oder C-terminal mit der Cytochrom P450-Monooxygenase der CYP153-Familie fusionierte Polypeptide handeln. Ob eine Ferredoxin-Reduktase oder ein Ferredoxin mit einer gegebenen Cytochrom P450-Monooxygenase der CYP153-Familie mit einander funktionell wechselwirken, kann der Fachmann leicht dadurch feststellen, ob das Reduktionsmittel in Gegenwart eines Alkansubstrates und der drei Polypeptide oxidiert wird. Alternativ kann der von Scheps, D., Malca, H., Hoffmann, B., Nestl, B. M, und Hauer, B. (2011) Org. Biomol. Chem., 9, 6727 beschriebene Enzymtest verwendet werden, der im Fall funktionell wechselwirkender Polypeptide eine deutliche Erhöhung der Reaktionsgeschwindigkeit zeigt. Beispielsweise stammen die Cytochrom P450-Monooxygenase der CYP153-Familie, das Ferredoxin und die Ferredoxin-Reduktase aus dem gleichen Organismus. Beispielsweise handelt es sich um die Ferredoxin-Reduktase aus *Alcanivorax borkumensis* SK2 (Datenbankcode YP_691923) oder eine Variante davon, das Ferredoxin aus *Alcanivorax borkumensis* SK2 (Datenbankcode YP_691920) oder eine Variante davon und die Cytochrom P450-Monooxygenase der CYP153-Familie aus *Alcanivorax borkumensis* SK2 (Datenbankcode YP_691921) oder eine Variante davon.

In einem weiteren Beispiel handelt es sich bei der Alkanhydroxylase um eine AlkB-Monooxygenase. AlkB stellt eine zunächst aus dem AlkBGT-System aus *Pseudomonas putida* Gpo1 bekannt gewordene Oxidoreduktase dar, die von zwei weiteren Polypeptiden, AlkG und AlkT, abhängig ist. AlkT wird als FAD-abhängige Rubredoxin-Reduktase charakterisiert, die Elektronen aus NADH an AlkG weitergibt. Bei AlkG handelt es sich um ein Rubredoxin, ein eisenhaltiges Redoxprotein, das als direkter Elektronendonor für AlkB fungiert. Beispielsweise wird unter dem Begriff "AlkB-Monooxygenase" ein Polypeptid mit einer Sequenzhomologie von wenigstens 75, 80, 85, 90, 92, 94, 96, 98 oder 99 % zur Sequenz des AlkB von *Pseudomonas putida* Gpo1 (Datenbankcode: CAB54050.1; dieser Datenbankcode stammt wie alle weiteren in der Anmeldung verwendeten aus dem Stand der Technik, nämlich aus der NCBI Datenbank, genauer dem am 15. Oktober 2012 online verfügbaren Release) mit der Fähigkeit, Alkane zu oxidieren, verstanden. Beispielsweise handelt es sich bei der AlkB-Monooxygenase um eine mit den AlkG (CAB54052.1)- und AlkT (CAB54063.1)-Polypeptiden aus *Pseudomonas putida* Gpo1 funktionell zusammenwirkende, Alkane oxidierende Oxidoreduktase. Zur optimalen Versorgung der AlkB-Alkanhydroxylase mit Elektronen kann die Zelle die Monoxygenase zusammen mit funktionell mit ihr wechselwirkenden Hilfsproteinen, z.B. AlkG und/oder AlkT oder jeweils Varianten davon exprimieren, wobei es sich z.B. wiederum um AlkG (CAB54052.1)- und AlkT (CAB54063.1)-Polypeptide aus *Pseudomonas putida* Gpo1 handelt.

Die Fähigkeit der im erfindungsgemäßen Verfahren verwendeten Zelle zur Oxidation von Substraten kann dadurch verstärkt werden, dass die Zelle alternativ oder zusätzlich zur Alkanhydroxylase eine Alkoholdehydrogenase exprimiert.

Beispielsweise wird unter dem Begriff "Alkoholdehydrogenase", wie hierin verwendet, wird ein Enzym verstanden, das einen Aldehyd bzw. Keton zu dem entsprechenden primären bzw. sekundären Alkohol oxidiert. Beispiele umfassend die Alkoholdehydrogenasen von *Ralstonia eutropha* (ACB78191.1), *Lactobacillus brevis* (YP_795183.1), *Lactobacillus kefiri* (ACF95832.1), aus Pferdeleber, von *Paracoccus pantotrophus* (ACB78182.1) und *Sphingobium yanoikuyae* (EU427523.1) sowie die jeweiligen Varianten davon. Bei der Verwendung eines Ganzzellkatalysators kann sich das Problem stellen, dass ein Substrat mit einem intrazellulär lokalisierten Enzym in Kontakt gebracht werden muss, damit es zur erwünschten Reaktion kommt. Im Falle langkettiger Alkane und Derivate davon ist bevorzugt, dass der Ganzzellkatalysator ein Polypeptid der AlkL-Familie aufweist. Beispielsweise handelt es sich bei einem "Polypeptid der AlkL-Familie", wie hierin verwendet, um ein Polypeptid, das über eine Länge von 230 aufeinander abfolgenden Aminosäuren eine wenigstens 80, z.B. oder 90%ige Sequenzidentität zu AlkL aus *Pseudomonas putida* (Datenbankcode CAB69081) oder eine Variante von AlkL aus *Pseudomonas putida* und beispielsweise die Fähigkeit aufweist, den Import von langkettigen Alkanen ins Innere einer Zelle zu unterstützen. In einem weiteren Beispiel handelt es sich bei einem "Polypeptid der AlkL-Familie", wie hierin verwendet, um eine in der äußeren Membran eines Gram-negativen Bakteriums lokalisiertes Polypeptid, welches das Sequenzmotiv DXWAPAXQ(V/A)GXR, aufweist, wobei X eine proteinogene Aminosäure darstellt, und z.B. zusätzlich AlkL aus *Pseudomonas putida* (Datenbankcode CAB69081) oder eine Variante davon ist. Beispielhafte Mitglieder der AlkL-Familie umfassen AlkL aus *Pseudomonas putida* (Datenbankcode CAB69081), *Marinobacter aquaeolei VT8* (Datenbankcode YP_957722), *Oceanicaulis alexandrii* HTCC2633 (Datenbankcode ZP_00953584), *Marinobacter manganoxydans* Mnl7-9 (Datenbankcode ZP_09158756), *Caulobacter* sp. K31 (Datenbankcode YP_001672217), *Pseudomonas oleovorans* (Datenbankcode Q00595) und Varianten davon.

Die Lehre der vorliegenden Erfindung kann nicht nur unter Verwendung von Makromolekülen mit der exakten Aminosäure- oder Nukleinsäuresequenz, auf die hierin Bezug genommen wird, bzw. nicht nur unter Verwendung von einer Zelle mit relativ zum jeweiligen Wildtyp verringerter Aktivität eines Polypeptides mit der exakten Aminosäuresequenz, auf die hierin Bezug genommen wird, ausgeführt werden, sondern auch unter Verwendung einer Variante derartiger Makromoleküle oder von einer Zelle mit einer relativ zum jeweiligen Wildtyp der jeweiligen Zelle verringerten Aktivität einer Variante des Polypeptids, die durch Deletion, Addition oder Substitution einer oder mehr als einer Aminosäuren oder Nukleinsäuren erhalten werden kann.

Erfindungsgemäß bedeutet der Begriff "Variante" einer Nukleinsäuresequenz oder Aminosäuresequenz, im Folgenden gleichbedeutend und austauschbar mit dem Begriff "Homologon" gebraucht, wie hierin verwendet, eine andere Nukleinsäure- oder Aminosäuresequenz, die mit Hinblick auf die entsprechende ursprüngliche Wildtyp-Nukleinsäure- oder -aminosäuresequenz eine Homologie, hier gleichbedeutend mit Identität verwendet, von 90, 92, 94, 96, 98, 99 % oder mehr Prozent aufweist, wobei auch andere als die das katalytisch aktive Zentrum ausbildende Aminosäuren oder für die Struktur oder Faltung essentielle Aminosäuren deletiert oder substituiert sind oder solche lediglich konservativ substituiert sind, beispielsweise ein Glutamat statt einem Aspartat oder ein Leucin statt einem Valin. Der Stand der Technik beschreibt Algorithmen, die verwendet werden können, um das Ausmaß von Homologie von zwei Sequenzen zu berechnen, z. B. Arthur Lesk (2008), Introduction to bioinformatics, 3rd edition. Erfindungsgemäß weist die Variante einer Aminosäure- oder Nukleinsäuresequenz, zusätzlich zur oben genannten Sequenzhomologie, im Wesentlichen die gleiche enzymatische Aktivität des Wildtypmoleküls bzw. des ursprünglichen Moleküls auf. Zum Beispiel weist eine Variante eines als Protease enzymatisch aktiven Polypeptids die gleiche oder im Wesentlichen die gleiche proteolytische Aktivität wie das Polypeptidenzym auf, d.h. die Fähigkeit, die Hydrolyse einer Peptidbindung zu katalysieren. Beispielsweise bedeutet der Begriff "im Wesentlichen die gleiche enzymatische Aktivität" eine Aktivität mit Hinblick auf die Substrate des Wildtyp-Polypeptids, die deutlich über der Hintergrundaktivität liegt oder/und sich um weniger als 3, z.B. 2, oder eine Größenordnung von den K_{M}- und/oder k_{cat}- Werten unterscheidet, die das Wildtyppolypeptid mit Hinblick auf die gleichen Substrate aufweist. Beispielsweise umfasst der Begriff "Variante" einer Nukleinsäure- oder Aminosäuresequenz wenigstens einen aktiven Teil/oder Fragment der Nukleinsäure- bzw. Aminosäuresequenz. Beispielsweise bedeutet der Begriff "aktiver Teil", wie hierin verwendet, eine Aminosäuresequenz oder eine Nukleinsäuresequenz, die eine geringere als die volle Länge der Aminosäuresequenz aufweist bzw. für eine geringere als die volle Länge der Aminosäuresequenz kodiert, wobei die Aminosäuresequenz oder die kodierte Aminosäuresequenz mit geringerer Länge als der Wildtyp-Aminosäuresequenz im Wesentlichen die gleiche enzymatische Aktivität wie das Wildtyppolypeptid oder eine Variante davon aufweist, beispielsweise als Protease. Beispielsweise umfasst der Begriff "Variante" einer Nukleinsäure eine Nukleinsäure, deren komplementärer Strang, z.B. unter stringenten Bedingungen, an die Wildtyp-Nukleinsäure bindet. Die Stringenz der Hybridisierungsreaktion ist für den Fachmann leicht bestimmbar und hängt im Allgemeinen von der Länge der Sonde, den Temperaturen beim Waschen und der Salzkonzentration ab. Im Allgemeinen benötigen längere Sonden höhere Temperaturen zum Hybridisieren, wohingegen kürzere Proben mit geringen Temperaturen auskommen. Ob Hybridisierung stattfindet, hängt im Allgemeinen von der Fähigkeit der denaturierten DNA ab, an komplementäre Stränge zu annellieren, die in ihrer Umgebung vorhanden sind, und zwar unterhalb der Schmelztemperatur. Die Stringenz von Hybridisierungsreaktion und entsprechende Bedingungen sind ausführlicher in F M Ausubel (1995), Current Protocols in Molecular Biology. John Wiley & Sons, Inc. beschrieben. Anleitungen zur Identifizierung von DNA-Sequenzen mittels Hybridisierung findet der Fachmann unter anderem im Handbuch "The DIG System Users Guide for Filter Hybridization" der Firma Boehringer Mannheim GmbH (Mannheim, Deutschland, 1993) und bei Liebl et al. (International Journal of Systematic Bacteriology 41: 255-260 (1991)). Die Hybridisierung findet beispielsweise unter stringenten Bedingungen statt, das heißt, es werden nur Hybride gebildet, bei denen Sonde und Zielsequenz, d. h. die mit der Sonde behandelten Polynukleotide, mindestens 70% identisch sind. Es ist bekannt, dass die Stringenz der Hybridisierung einschließlich der Waschschritte durch Variieren der Pufferzusammensetzung, der Temperatur und der Salzkonzentration beeinflusst bzw. bestimmt wird. Die Hybridisierungsreaktion wird im Allgemeinen bei relativ niedriger Stringenz im Vergleich zu den Waschschritten durchgeführt (Hybaid Hybridisation Guide, Hybaid Limited, Teddington, UK, 1996). Für die Hybridisierungsreaktion kann beispielsweise ein Puffer entsprechend 5x SSC-Puffer bei einer Temperatur von ca. 50°C - 68°C eingesetzt werden. Dabei können Sonden auch mit Polynukleotiden hybridisieren, die weniger als 70% Identität zur Sequenz der Sonde aufweisen. Solche Hybride sind weniger stabil und werden durch Waschen unter stringenten Bedingungen entfernt. Dies kann beispielsweise durch Senken der Salzkonzentration auf 2x SSC und gegebenenfalls nachfolgend 0,5x SSC (The DIG System User's Guide for Filter Hybridisation, Boehringer Mannheim, Mannheim, Deutschland, 1995) erreicht werden, wobei eine Temperatur von ca. 50°C - 68°C, ca. 52°C - 68°C, ca. 54°C - 68°C, ca. 56°C - 68°C, ca. 58°C - 68°C, ca. 60°C - 68°C, ca. 62°C - 68°C, ca. 64°C - 68°C, ca. 66°C - 68°C eingestellt wird. Temperaturbereiche können von ca. 64°C - 68°C oder ca. 66°C - 68°C sein. Es ist gegebenenfalls möglich die Salzkonzentration bis auf eine Konzentration entsprechend 0,2 x SSC oder 0,1 x SSC zu senken. Durch schrittweise Erhöhung der Hybridisierungstemperatur in Schritten von ca. 1 - 2°C von 50°C auf 68°C können Polynukleotidfragmente isoliert werden, die beispielsweise mindestens 70% oder mindestens 80% oder mindestens 90%, mindestens 91%, mindestens 92%, mindestens 93%, mindestens 94%, mindestens 95%, mindestens 96%, mindestens 97%, mindestens 98% oder mindestens 99% Identität zur Sequenz des eingesetzten Nukleinsäuremoleküls. Weitere Anleitungen zur Hybridisierung sind in Form sogenannter Kits am Markt erhältlich (z.B. DIG Easy Hyb von der Firma Roche Diagnostics GmbH, Mannheim, Deutschland, Catalog No. 1603558). Beispielsweise umfasst der Begriff "Variante" einer Nukleinsäure, wie hierin verwendet, eine beliebige Nukleinsäuresequenz, die für die gleiche Aminosäuresequenz wie die ursprüngliche Nukleinsäure oder eine Variante dieser Aminosäuresequenz im Rahmen der Degeneriertheit des genetischen Codes kodiert.

In einer bevorzugten Ausführungsform weist die erfindungsgemäß verwendete Zelle eine ihrem Wildtyp gegenüber verringerte Aktivität wenigstens eines Enzyms aufweist, das eine der Reaktionen der β-Oxidation von Fettsäuren katalysiert, wobei es sich bevorzugt um ein Enzym aus der Gruppe handelt, die Fettsäure-CoA-Ligase, Acyl-CoA-Dehydrogenase, 2,4-Dienoyl-CoA-Reduktase, Enoyl-CoA-Hydratase and 3-Ketoacyl-CoA-Thiolase, einen Fettsäureimporter oder Varianten davon umfasst, besonders bevorzugt um FadL oder eine Variante davon. Die β-Oxidation von Fettsäuren ist ein weit verbreiteter Stoffwechselweg, der es prokaryontischen und eukaryontischen Organismen gleichermaßen erlaubt, Fettsäuren zu oxidieren und die darin enthaltene chemische Energie dem Stoffwechsel verfügbar zu machen. Im weiteren Sinn beginnt sie mit der Aufnahme einer Fettsäure in die Zelle, im Falle von *E. coli* durch den Transporter FadL, der sie durch die äußere bzw. innere Membran der Gram-negativen Bakterienzelle schleust und das FadD -Genprodukt, das die Fettsäure in Form des CoA-Esters ins Cytosol freisetzt. Dort wird die Fettsäure, sofern die Bedingungen es erfordern, zunächst an der β-Position des CoA-Fettsäureesters durch eine Acyl-CoA-Dehydrogenase, im Falle von *E*. *coli* FadE, oxidiert. Ein ähnliches Molekül kann alternativ auch aus einer doppelt ungesättigten Fettsäure durch Reduktion mittels einer 2,4-dienoyl-CoA-Reduktase, bei *E. coli* FadH, gebildet werden. Ein multifunktionelles Enzym, die Enoyl-CoA-Hydratase/3-Hydroxyacyl-CoA-Dehydrogenase, bei *E*. *coli* FadB, katalysiert anschließend die Hydratisierung unter Bildung des sekundären Alkohols und dessen anschließende Oxidation zum Keton. Im letzten Schritt katalysiert eine 3-Ketoacyl-CoA-Thiolase, im Falle von *E*. *coli* FadA, die Spaltung des Ketoacyl-CoA mit dem Ergebnis, dass Acetyl-CoA und ein im Vergleich zum Ausgangsmolekül um zwei Kohlenstoffatome verkürzter CoA-Ester der Fettsäure freigesetzt werden. Sofern es sich nicht ebenfalls um Acetyl-CoA handelt, kann letzterer erneut in den β-Oxidationszyklus eingespeist und unter Oxidation verkürzt werden. An der Regulation der β-Oxidation von Fettsäuren ist auch FadR beteiligt, ein Regulator des Fad-Operons, der die für den Abbau von Fettsäuren erforderlichen Gene umfasst, ohne dass FadR eine Reaktion der β-Oxidation katalysieren würde. Beispielsweise wird unter dem Begriff "Enzym, das eine der Reaktionen der β-Oxidation von Fettsäuren katalysiert" ein jegliches Enzym verstanden, das direkt mit dem Fettsäuresubstrat oder einem auf dem Weg zum Acetyl-CoA daraus entstehenden Molekül wechselwirkt, z.B. es als Substrat erkennt, und seine Umwandlung zu einem auf diesem Abbauweg näher am Acetyl-CoA liegenden Stoffwechselprodukt katalysiert, z.B. einschließlich des Fettsäureimporters, der die Aufnahme der Fettsäure in die Zelle bewerkstelligt. Beispielsweise zählt zu diesen Enzymen nach der vorangegangenen Definition die Acyl-CoA-Dehydrogenase, da sie mit dem Fettsäure-CoA-Ester wechselwirkt und dessen Umwandlung zum Enyol-CoA katalysiert, das auf dem Stoffwechselweg der β-Oxidation näher am Acetyl-CoA liegt als der Fettsäure-CoA-Ester. Beispielsweise wird unter dem Begriff "Enzym, das eine der Reaktionen der β-Oxidation von Fettsäuren katalysiert", wie hierin verwendet, jedes Enzym aus der Gruppe verstanden, die die Genprodukte FadA, FadB, FadD, FadL und FadE aus *E. coli* und/oder deren Varianten oder Homologa aus anderen Organismen umfasst. Die Genprodukte FadA, FadB, FadD, FadL und FadE aus *E. coli* ebenso wie Varianten und Homologa aus zahlreichen weiteren biotechnologisch nutzbaren Organismen und ihre Nukleinsäure- und Polypeptidssequenzen sind im Stand der Technik beschrieben, beispielsweise FadA unter Zugangsnummer AP009048.1, FadB unter Zugangsnummer BAE77457.1, FadD unter Zugangsnummer BAA15609.1, FadE unter Zugangsnummer BAA77891.2 und FadL unter Zugangsnummer BAA 16205.1.

In einer weiteren bevorzugten Ausführungsform exprimiert die erfindungsgemäße oder im erfindungsgemäßen Verfahren verwendete Zelle eine Transaminase. Beispielsweise wird unter dem Begriff "Transaminase", wie hierin verwendet, ein Enzym verstanden, das die Übertragung von α-Aminogruppen von einem Donor-, z.B. einer Aminosäure, auf ein Akzeptormolekül, bevorzugt eine α-Ketocarbonsäure, katalysiert. Beispielsweise kann eine Transaminase aus der Gruppe umfassend 3HMU_A, AAD41041.1, AAK15486.1, ABE03917.1, ADR60699.1, ADR61066.1, ADR62525.1, AEL07495.1, CAZ86955.1, EFW82310.1, EFW87681.1, EGC99983.1, EGD03176.1, EGE58369.1, EGH06681.1, EGH08331.1, EGH24301.1, EGH32343.1, EGH46412.1, EGH55033.1, EGH62152.1, EGH67339.1, EGH70821.1, EGH71404.1, EGH78772.1, EGH85312.1, EGH97105.1, EGP57596.1, NP_102850.1, NP_106560.1, NP_248912.1, NP_248990.1, NP_354026.2, NP_421926.1, NP_637699.1, NP_642792.1, NP_744329.1, NP_744732.1, NP_747283.1, NP_795039.1, NP_901695.1 (, XP_002943905.1, YP_001021095.1, YP_001059677.1, YP_001061726.1, YP_001066961.1, YP_001074671.1, YP_001120907.1, YP_001140117.1, YP_001170616.1, YP_001185848.1, YP_001188121.1, YP_001233688.1, YP_001268866.1, YP_001270391.1, YP_001345703.1, YP_001412573.1, YP_001417624.1, YP_001526058.1, YP_001579295.1, YP_001581170.1, YP_001668026.1, YP_001669478.1, YP_001671460.1, YP_001685569.1, YP_001747156.1, YP_001749732.1, YP_001765463.1, YP_001766294.1, YP_00 1790770.1, YP_001808775.1, YP_001809596.1, YP_001859758.1, YP_001888405.1, YP_001903233.1, YP_001977571.1, YP_002229759.1, YP_002231363.1, YP_002280472.1, YP_002297678.1, YP_002543874.1, YP_002549011.1, YP_002796201.1, YP_002801960.1, YP_002875335.1, YP_002897523.1, YP_002912290.1, YP_002974935.1, YP_003060891.1, YP_003264235.1, YP_003552364.1, YP_003578319.1, YP_003591946.1, YP_003607814.1, YP_003641922.1, YP_003674025.1, YP_003692877.1, YP_003755112.1, YP_003896973.1, YP_003907026.1, YP_003912421.1, YP_004086766.1, YP_004142571.1, YP_004147141.1, YP_004228105.1, YP_004278247.1, YP_004305252.1, YP_004356916.1, YP_004361407.1, YP_004378186.1, YP_004379856.1, YP_004390782.1, YP_004472442.1, YP_004590892.1, YP_004612414.1, YP_004676537.1, YP_004693233.1, YP_004701580.1, YP_004701637.1, YP_004704442.1, YP_108931.1, YP_110490.1, YP_168667.1, YP_237931.1, YP_260624.1, YP_262985.1, YP_271307.1, YP_276987.1, YP_334171.1, YP_337172.1, YP_350660.1, YP_351134.1, YP_364386.1, YP_366340.1, YP_369710.1, YP_370582.1, YP_426342.1, YP_440141.1, YP_442361.1, YP_468848.1, YP_521636.1, YP_554363.1, YP_608454.1, YP_610700.1, YP_614980.1, YP_622254.1, YP_625753.1, YP_680590.1, YP_751687.1, YP_767071.1, YP_774090.1, YP_774932.1, YP_788372.1, YP_858562.1, YP_928515.1, YP_983084.1, YP_995622.1, ZP_00948889.1, ZP_00954344.1, ZP_00959736.1, ZP_00998881.1, ZP_01011725.1, ZP_01037109.1, ZP_01058030.1, ZP_01076707.1, ZP_01103959.1, ZP_01167926.1, ZP_01224713.1, ZP_01442907.1, ZP_01446892.1, ZP_01550953.1, ZP_01625518.1, ZP_01745731.1, ZP_01750280.1, ZP_01754305.1, ZP_01763880.1, ZP_01769626.1, ZP_01865961.1, ZP_01881393.1, ZP_01901558.1, ZP_02145337.1, ZP_02151268.1, ZP_02152332.1, ZP_02167267.1, ZP_02190082.1, ZP_02242934.1, ZP_02360937.1, ZP_02367056.1, ZP_02385477.1, ZP_02456487.1, ZP_02883670.1, ZP_03263915.1, ZP_03263990.1, ZP_03400081.1, ZP_03452573.1, ZP_03456092.1, ZP_03517291.1, ZP_03529055.1, ZP_03571515.1, ZP_03572809.1, ZP_03587785.1, ZP_03588560.1, ZP_03697266.1, ZP_03697962.1, ZP_04521092.1, ZP_04590693.1, ZP_04890914.1, ZP_04891982.1, ZP_04893793.1, ZP_04902131.1, ZP_04905327.1, ZP_04941068.1, ZP_04944536.1, ZP_04945255.1, ZP_04959332.1, ZP_04964181.1, ZP_05053721.1, ZP_05063588.1, ZP_05073059.1, ZP_05077806.1, ZP_05082750.1, ZP_05091128.1, ZP_05095488.1, ZP_05101701.1, ZP_05116783.1, ZP_05121836.1, ZP_05127756.1, ZP_05637806.1, ZP_05742087.1, ZP_05783548.1, ZP_05786246.1, ZP_05843149.1, ZP_05945960.1, ZP_06459045.1, ZP_06487195.1, ZP_06492453.1, ZP_06493162.1, ZP_06703644.1, ZP_06731146.1, ZP_06839371.1, ZP_07007312.1, ZP_07266194.1, ZP_07374050.1, ZP_07662787.1, ZP_07778196.1, ZP_07797983.1, ZP_08099459.1, ZP_08138203.1, ZP_08141719.1, ZP_08142973.1, ZP_08177102.1, ZP_08185821.1, ZP_08186468.1, ZP_08208888.1, ZP_08266590.1, ZP_08402041.1, ZP_08406891.1, ZP_08522175.1, ZP_08527488.1, ZP_08631252.1, ZP_08636687 verwendet werden.

In einer weiteren bevorzugten Ausführungsform exprimiert die im erfindungsgemäßen Verfahren verwendete Zelle eine Alanindehydrogenase. Beispielsweise wird unter dem Begriff "Alanindehydrogenase", wie hierein verwendet, ein Enzym verstanden, das die Umwandlung von L-Alanin unter Verbrauch von Wasser und NAD⁺ zu Pyruvat, Ammoniak und NADH katalysiert. Beispielsweise können die Alanindehydrogenasen aus der Gruppe umfassend die Alanindehydrogenase aus *Bacillus subtilis* (Datenbankcode L20916), *Rhizobium leguminosarum* (Datenbankcode CP001622), *Vibrio proteolyticus* (Datenbankcode AF070716), *Mycobacterium tuberculosis* (Datenbankcode X63069), *Enterobacter aerogenes* (Datenbankcode AB013821), EGR93259.1, YP_003654745.1, YP_003651439.1, YP_003637111.1, YP_003631815.1, YP_001327051.1, YP_001262560.1, YP_886996.1, YP_882850.1, YP_704410.1, YP_703508.1, ZP_08624689.1, YP_001230376.1, P17557.1, P17556.1, CCB94892.1, CCB73698.1, YP_001168635.1, YP_004668736.1, YP_004569425.1, YP_003513168.1, YP_004561169.1, ZP_08554945.1, YP_400777.1, ZP_08311476.1, ZP_08310170.1, ZP_08267322.1, ZP_08263846.1, ZP_07898723.1, YP_149301.1, YP_148605.1, YP_004340432.1, EFT09946.1, EFS80513.1, EFS51332.1, EFS42459.1, YP_003060895.1, YP_003059033.1, ZP_03305373.1, YP_847214.1, YP_004095847.1, YP_003338282.1, YP_003337256.1, YP_355846.1, YP_253131.1, ZP_08197563.1, ZP_08196283.1, ADW06447.1, YP_734091.1, NP_372233.1, NP_102173.1, ZP_08170259.1, EGD36706.1, EGD32748.1, ZP_08155540.1, YP_004142849.1, YP_002417649.1, YP_001301040.1, YP_002992892.1, YP_081348.1, YP_080482.1, YP_002476349.1, ZP_08115025.1, ZP_08114403.1, YP_003552869.1, YP_002358112.1, YP_575010.1, YP_477594.1, YP_474564.1, YP_130399.1, YP_129373.1, YP_123314.1, NP_810467.1, NP_646469.1, NP_626044.1, NP_391071.1, ZP_08086822.1, ZP_08084776.1, ZP_08083119.1, ZP_08020768.1, ZP_08013590.1, ZP_08011832.1, YP_003783744.1, YP_002781576.1, YP_002780533.1, ZP_02195873.1, NP_797482.1, ZP_07645051.1, ZP_07643260.1, ZP_06611917.1, AAT40119.1, ZP_07864946.1, YP_004068409.1, YP_002796203.1, YP_002774420.1, YP_003600348.1, YP_003599946.1, YP_003565624.1, YP_003565223.1, YP_335198.1, YP_423850.1, YP_155059.1, ZP_07843538.1, ZP_07841226.1, ZP_06928932.1, ZP_05692073.1, ZP_05687006.1, ZP_04867480.1, YP_775531.1, CBE70214.1, ZP_07721182.1, ZP_04302850.1, ZP_04298961.1, ZP_04287684.1, ZP_04277177.1, ZP_04248389.1, ZP_04235899.1, ZP_02159718.1, ZP_02152178.1, YP_003974610.1, YP_003546595.1,YP_002317127.1, ZP_07313778.1, ZP_07302778.1, ZP_07298850.1, CBK69442.1, YP_003413835.1, YP_003595089.1, ZP_06807811.1, YP_003582455.1, YP_003464731.1, YP_003496397.1, YP_003421918.1, CBL07274.1, CBK64956.1, YP_003508515.1, AAL87460.1, AAC23579.1, AAC23578.1, AAC23577.1, ACU78652.1, YP_003471439.1, YP_003452777.1, ZP_06384971.1, ACY25368.1, ABC26869.1, AAP44334.1, EEZ80018.1, ZP_05110458.1, 1PJB_A, ZP_04717201.1, ZP_04689103.1, CAO90307.1, CAM75354.1, CAA44791.1, BAA77513.1, EGR96638.1, EGL90046.1, YP_004510847.1, ZP_08450330.1, YP_003387804.1, YP_003058152.1, EFS74272.1, EFS67128.1, ZP_06844564.1, YP_826658.1, YP_001195249.1, YP_003095978.1, YP_469292.1, YP_004442054.1, YP_004461174.1, YP_004055616.1, YP_003576656.1, YP_003094537.1, YP_001295973.1, AEE71143.1, YP_004447480.1, YP_003761844.1, YP_040853.1, YP_003154888.1, YP_003142045.1, YP_002280953.1, NP_371963.1, NP_422368.1, EGC98966.1, EGC76398.1, YP_004263661.1, YP_004252039.1, YP_679036.1, YP_499973.1, ZP_08054972.1, ZP_08053009.1, ZP_04067276.1, ZP_03968868.1, ZP_03963857.1, ZP_03933079.1, ZP_03497046.1, ZP_06668924.1, ZP_06667106.1, ZP_06324464.1, ZP_06196777.1, ZP_05114159.1, ZP_05083968.1, ZP_05070370.1, ZP_05030022.1, ZP_04673064.1, ZP_03517011.1, ZP_03505783.1, XP_001310698.1, ABK27691.1 oder CAB59281.2 verwendet werden. Für den Fall, dass die Zelle eine Alanindehydrogenase exprimiert, ist es vorteilhaft, eine anorganische Stickstoffquelle, bevorzugt ein Ammoniumsalz wie Ammoniumchlorid oder Ammoniumsulfat, in ausreichender Menge zur wässrigen Lösung zu geben. Ein Verfahren zur Erhöhung der Alaninkonzentration ist in der EP12162846.5 beschrieben.

Ein Aspekt der vorliegenden Erfindung sieht die Verwendung eines Knockouts eines für ein Polypeptid umfassend SEQ ID NO 2 oder eine Variante davon kodierenden Gens als Teil der genetischen Ausstattung einer rekombinanten Zelle zur Erhöhung der Produktion eines Carbonsäureesters der Formel (I) vor. Dies bedeutet, dass der Knock out eines für ein Polypeptid umfassend SEQ ID NO 2 oder eine Variante davon kodierenden Gens als Merkmal der Zelle mit dem Zweck vorgenommen wurde, die Ausbeute, Kohlenstoff- und/oder Stickstoffbilanz und/oder Reinheit des Produktes der Umsetzung des Carbonsäureesters der Formel (I) zu erhöhen.

Es ist eine Zelle offenbart, bei der es sich um eine *E*. *coli*-Zelle handelt, die einen Knockout des Polypeptides umfassend SEQ ID NO 2 oder einer Variante sowie des Polypeptides FadL im Genom der Zelle aufweist, wobei die Zelle weiterhin eine Alkanhydroxylase, bevorzugt AlkB aus *Pseudomonas putida,* eine Transaminase, bevorzugt die Transaminase von *Chromobacterium violaceum* ATCC 12472, eine Alanindehydrogenase, z.B. die Alanindehydrogenase aus *Bacillus subtilis,* und AlkL aus *Pseudomonas putida* exprimiert. Diese Zelle wird beispielsweise in einer wässrigen Lösung mit einem Fettsäureester, z.B. Laurinsäuremethylester, kontaktiert.

### Abbildungen:

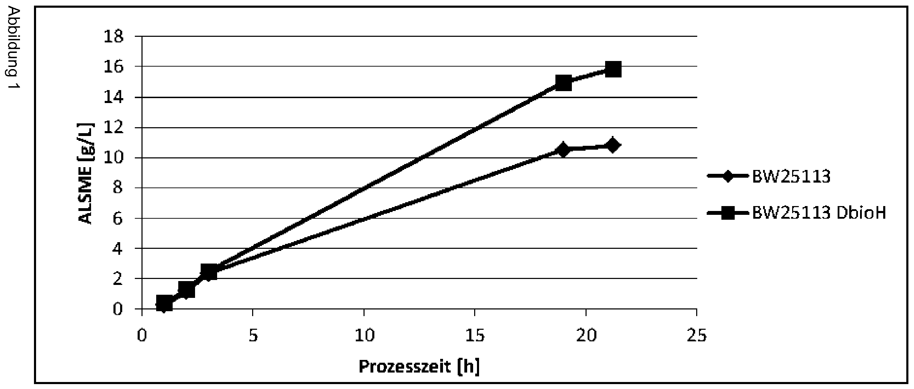

Abbildung 1 zeigt die omega-Aminolaurinsäure-Produktivität verschiedener Stämme

### Beispiel 1 (nicht erfindungsgemäß)

### Inaktivierung von bioH in E. coli W3110 und BW25113

Zum gezielten *Knockout* des *bioH*-Genes (b3412, SEQ ID 1) mit dem Grundvektor pKO3_E933 wurde ein neues Plasmid benötigt. Basis ist der Vektor pKO3_E933 (SEQ ID 14), eingesetzt wurden 500 bp *upstream* (SEQ ID 3) bzw. 500 bp *downstream* des *bioH-Gens* (SEQ ID 4), welche von einer *Psp*XI-Schnittstelle (CCTCGAGG) separiert vorliegen. Das fertig gestellte Plasmid trägt die interne Bezeichnung AHp-LL-42 (SEQ ID 5). Zur Fertigstellung wurden folgende Oligonukleotide verwendet:
o-LL-314, SEQ ID 6 5'-CCGGGGATCGCGGCCCGGCTTCGCTATCCCATTGGCAGT-3'
o-LL-315, SEQ ID 7 5'-CCTCTGCTTCAACGCCCTCGAGGCATCCGCTATTGTTCTCTTTTGACTTACAAGGATG-3'
o-LL-316, SEQ ID 8 5'-GCGTTGAAGCAGAGGGTGTAGGTG-3'
o-LL-317, SEQ ID 9 5'-TAGAGGATCGCGGCCCAAACTGGCAAGGCAGCTTTATGC-3'

Die 500bp-Bereiche wurden mit obigen Primern aus vorliegender chromosomaler DNA von *E*. *coli* W3110 mittels Polymerase-Kettenreaktion (PCR) zur Verfügung gestellt. Verwendet wurden o-LL-314 + o-LL-315 für den *upstream*-Bereich (SEQ ID 3) und o-LL-316 + o-LL-317 für den *downstream*-Bereich (SEQ ID 4). Folgende Parameter wurden für die PCR angewandt:
Initiale Denaturierung: 98 °C, 10 s
30 x Denaturierung: 98 °C, 10 s
30 x Annealing: 57,9/58,8/59,7/60,6/61,4/62,3/63,2/64,1 °C (Temperaturgradient)
30 x Elongation: 72 °C, 20 s
Finale Elongation: 72 °C, 4 min

Für die Vervielfachung wurde der 2x Phusion HF Master Mix von New England Biolabs (NEB, M0531S) nach Herstellerangaben verwendet. Die PCR-Produkte wurden abhängig vom Reinheitsgrad direkt säulengereinigt (*QiaQuick PCR Purification Kit,* Qiagen, Hilden) oder über ein Agarosegel gereinigt und extrahiert (*QiaQuick Gel Extraction Kit,* Qiagen, Hilden). Die Durchführung der PCR, der Agarose-Gel-Elektrophorese, Ethidiumbromidfärbung der DNA und Bestimmung der PCR-Fragmentgrößen erfolgte in der für den Fachmann bekannten Art und Weise. In beiden Fällen konnten PCR-Fragmente der erwarteten Größe bereitgestellt werden.

Die aufgereinigten PCR-Produkte wurden in den mit *Notl* -geschnittenen pKO3_E933-Vektor (SEQ ID 14) mittels Rekombination unter Verwendung des In-Fusion HD Cloning Kit nach Anleitung des Herstellers (Clontech Laboratories Inc., Mountain View, CA, USA) kloniert. Die Transformation chemisch kompetenter *E.coli* DH10β (New England Biolabs, Frankfurt) erfolgte nach der für den Fachmann bekannten Art und Weise. Die korrekte Insertion der Zielsequenzen wurde durch Restriktionsanalyse überprüft und die Authentizität der eingebrachten Sequenzen durch DNA-Sequenzierung bestätigt. Der entstandene Vektor wurde mit AHp-LL-42 (SEQ ID 5) bezeichnet.

Die Konstruktion des Stammes *E.coli* W3110 *ΔbioH* erfolgte mit Hilfe des Vektors AHp-LL-42 (SEQ ID 5) mit dem Fachmann bekannten Methoden (siehe Link AJ, Phillips D, Church GM. J.Bacteriol. 1997. 179(20).). Die in den Experimenten verwendeten Stämme BW25113 sowie BW25113 ΔbioH (JW3375) wurden kommerziell als Teil der Keio-Collection erworben (siehe Construction of Escherichia coli K-12 in-frame, single-gene knockout mutants: the Keio collection, Tomoya Baba, Takeshi Ara, Miki Hasegawa, Yuki Takai, Yoshiko Okumura, Miki Baba, Kirill A Datsenko, Masaru Tomita, Barry L Wanner and Hirotada Mori, Molecular Systems Biology (2006), 2006 EMBO and Nature Publishing Group). Der konstruierte Stamm wurde unter folgender Bezeichnung weitergeführt:

| Stamm | Beschreibung |
|---|---|
| AHs-LL-56 | E. coli W3110 Δ*bioH*, Klon 1.1 |

Die DNA-Sequenz von *bioH* nach Deletion in W3110 ist in SEQ ID 10 wiedergegeben.

### Beispiel 2

### Produktion von Aminolaurinsäuremethylester durch E. coli W3110 mit Deletion im Gen bioH durch Einsatz von Expressionsvektoren für die Gene ald aus Bacillus subtilis und Cv_2025 aus Chromobacterium violaceum in Kombination mit einem Expressionsvektor für die Gene alkB, alkG, alkT und alkL aus dem alk-Operon von Pseudomonas putida.

Zur Erzeugung der *E*. *coli*-Stämme mit Expressionsvektoren für die Gene *ald* aus *Bacillus subtilis* (kodierend für eine Alanin-Dehydrogenase, codon-optimiert für *E. coli*), *Cv_2025* aus *Chromobacterium violaceum* (kodierend für eine Transaminase, codon-optimiert für *E. coli*) in Kombination mit einem Expressionsvektor für die Gene *alkB, alkG, alkT* (kodierend für eine Alkan-Monooxygenase, ein Rubredoxin und eine Rubredoxin-Reduktase) und *alkL* (kodierend für ein Membran-/Transportprotein) aus dem alk-Operon von *Pseudomonas putida* wurden elektrokompetente Zellen von *E. coli* W3110 Δ*bioH* sowie der entsprechende Kontrollstamm *E*. *coli* W3110 hergestellt. Dies geschah in einer dem Fachmann bekannten Art und Weise. Die Stämme wurden wie in Beispiel 1 beschrieben hergestellt. Diese wurden mit den Plasmiden pBT10_alkL (SEQ ID 11 bzw. WO/2011/131420 und die dort aufgeführte Seq ID NR: 8) und pJ294[alaDH_Bs(co)TA_Cv(co)] (SEQ ID 12 bzw. Beispiel 1 der WO/2013/024114 und die dort aufgeführte SEQ ID Nr. 17) transformiert und auf LB-Agar-Platten mit Kanamycin (50 µg/ml) und Ampicillin (100 µg/ml) ausplattiert. Transformanten wurden durch Plasmidpräparation und analytische Restriktionsanalyse bezüglich der Anwesenheit der korrekten Plasmide überprüft. Auf diese Weise wurden folgende Stämme konstruiert:

| Stammhintergrund | Vorhandene Plasmide |
|---|---|
| W3110 | pBT10_alkL pJ294[alaDH_Bs(co)TA_Cv(co)] |
| W3110 Δ*bioH* | pBT10_alkL pJ294[alaDH_Bs(co)TA_Cv(co)] |

Die Stämme wurden einer *fed-batch*-Fermentation unterzogen, um die Fähigkeit zur Produktion von Hydroxy-Laurinsäuremethylester, Oxo-Laurinsäuremethylester, Carboxy-Laurinsäuremethylester und Amino-Laurinsäuremethylester aus Laurinsäuremethylester zu analysieren. Dies wurde mit einem 8-fach Parallelfermentationssystem der Firma DASGIP durchgeführt.

Für die Fermentation wurden 1L-Reaktoren verwendet. Die pH-Sonden wurden mittels einer Zwei-Punkt-Kalibration mit Maßlösungen von pH 4,0 und pH 7,0 kalibriert. Die Reaktoren wurden mit 300 mL Trinkwasser befüllt und 20 min bei 121°C autoklaviert, um Sterilität zu gewährleisten. Anschließend wurden die pO2-Sonden über Nacht (mindestens 6 h lang) am DASGIP-System polarisiert. Am nächsten Morgen wurde das Wasser unter der Clean Bench entnommen und durch 300 mL Hochzelldichtemedium mit 100 mg/L Ampicillin, 50 mg/L Kanamycin und 5 mg/L Tetrazyklin ersetzt. Im Folgenden wurden die pO2-Sonden mit einer Einpunkt-Kalibration (Rührer: 400 rpm / Begasung: 10 sL/h Luft) kalibriert und die Feed-, Korrekturmittel- und Induktionsmittelstrecken mittels Clean-in-Place gereinigt. Dazu wurden die Schläuche mit 70% Ethanol, anschließend mit 1 M NaOH, dann mit sterilem VE-Wasser gespült und zuletzt mit den jeweiligen Medien befüllt.

Die ALS und ALSME produzierenden *E*. *coli*-Stämme wurden zuerst aus den jeweiligen Cryokulturen in LB-Medium (25 mL in einem 100 mL Schikanekolben) mit 100 mg/L Ampicillin über Nacht bei 37°C und 200 rpm ca. 18 h lang angezogen. Anschließend wurden je 2 mL der Kulturen in Hochzelldichtemedium (Glucose 15 g/L (30 mL / L einer separat autoklavierten 500 g/L Stammlösung mit 1% MgSO₄*7H₂O und 2,2% NH₄Cl), (NH₄)₂SO4 1,76 g/L, K₂HPO₄ 19,08 g/L, KH₂PO₄ 12,5 g/L, Hefeextrakt 6,66 g/L, Trinatriumcitrat-Dihydrat 2,24 g/L, Ammoniumeisencitrat-Lösung 17 mL/L einer separat autoklavierten 1% igen Stammlösung, Spurenelementlösung 5 mL/L separat autoklavierten Stammlösung (HCl (37 %) 36,50 g/L, MnCl₂*4H₂O 1,91 g/L, ZnSO₄*7H₂O 1,87 g/L, Ethylendiamintetraessigsäure-Dihydrat 0,84 g/L, H₃BO₃ 0,30 g/L. Na₂MoO₄*2H₂O 0,25 g/L, CaCl₂*2H₂O 4,70 g/L, FeSO₄*7H₂O 17,80 g/L, CuCl₂*2H₂O 0,15 g/L)) (Je Stamm 25mL in einem 100 mL Schikanekolben) mit 100 mg/L Ampicillin, 50 mg/L Kanamycin und 5 mg/L Tetrazyklin überimpft und bei 37°C / 200 rpm weitere 5,5 h lang inkubiert.

Die Reaktoren wurden mit einer optischen Dichte von 0,1 angeimpft indem ein entsprechendes Volumen der Vorkultur in einer 5 mL Spritze (unter sterilen Bedingungen) aufgezogen wurde und die Reaktoren mittels Kanüle über ein mit 70% Ethanol überschichtetes Septum beimpft wurde.

Folgendes Standardprogramm wurde verwendet:

| DO-Regler | | pH-Regler | |
|---|---|---|---|
| Preset | 0% | Preset | 0 ml/h |
| P | 0,1 | P | 5 |
| Ti | 300 s | Ti | 200 s |
| Min | 0% | Min | 0 mlL/h |
| Max | 100% | Max | 40 mL/h |

| N (Rotation) | von | bis | XO2 (Gasmischung) | von | bis | F (Gasfluss) | von | bis |
|---|---|---|---|---|---|---|---|---|
| Wachstum und Biotransformation | 0% | 30% | Wachstum und Biotransformation | 0% | 100% | Wachstum und Biotransformation | 15% | 80% |
| | 400 rpm | 1500 rpm | | 21 % | 21% | | 6 sL/h | 72 sL/h |

| Script | |
|---|---|
| Trigger scharf | 31% DO (1/60h) |
| Induktion IPTG | 2 h nach Feedstart |
| Feedtrigger | 50% DO |
| Feedrate | 3 [mL/h] |

Das durchgeführte Experiment lässt sich in zwei Phasen gliedern, die Anzucht, bei der die Zellen eine bestimmte optische Dichte erreichen sollen, und die nachfolgende Biotransformation, in der nach Zugabe des Substrates Laurinsäuremethylester eine Umsetzung zu Aminolaurinsäureester von in der Expression gebildeten Enzyme stattfinden soll. Die pH-Werte wurden einseitig mit Ammoniak (12,5 %) auf pH 6,8 geregelt. Während Anzucht und Biotransformation wurde der gelöste Sauerstoff (DO, dissolved oxygen) in der Kultur über Rührerdrehzahl und Begasungsrate bei 30% geregelt. Die Fermentation wurde als Fed-Batch durchgeführt, wobei der Feedstart, 5 g/Lh Glucosefeed (500 g/L Glucose mit 1% MgSO₄*7H₂O und 2,2% NH₄Cl), über einen DO-Peak getriggert wurde. Mit Feedstart wurde auch die Temperatur von vorher 37°C auf 30°C gesenkt. Die Expression der Transaminase, Alanindehydrogenase und Fettsäurereduktase wurde 2 h nach Feedstart durch die automatische Zugabe von IPTG (1 mM) induziert. Die Induktion der alk-Gene erfolgte durch die manuelle Zugabe von DCPK (0,025 % v/v) 10 h nach Feedstart. Vor Start der Biotransformation wurde die optische Dichte der Kulturbrühen bestimmt.

Der Start der Biotransformationsphase erfolgte 14 h nach Feedstart. Dazu wurden 150 mL eines Gemisches aus Laurinsäuremethylesther und dem lonentauscher Ölsäure (techn. 90%) als Batch zu der Fermentationsbrühe zugegeben. Um einen Aminogruppendonor für die Transaminase zur Verfügung zu stellen, wurde eine halbe Stunde vor Biotransformationsstart 5 mL einer 3M Ammoniumsulfatlösung zu der Fermentationsbrühe zugegeben. Zur Probennahme wurden 2 mL Fermentationsbrühe aus dem Kessel entnommen und ein Teil davon 1/20 in einem Aceton-HCl-Gemisch (c(HCl) = 0,1 mol/L) verdünnt und extrahiert. Proben wurden bei 1 h, 2 h, 3 h, 4 h, 5 h, 7.5 h, 10.5 h, 19.5 h und 21 h nach Start der Biotransformation von allen Reaktoren genommen. Die Umsatzraten für Sauerstoff (OTR = oxygen transfer rate) und Kohlenstoff (CTR = carbon transfer rate) wurden während der Fermentation über die Abgasanalytik an den DASGIP-Systemen bestimmt. Die Fermentation wurde 21 h nach Start der Biotransformation beendet. Der Rührer, die Begasung, die Temperaturregelung und pH-Regelung wurden ausgestellt und die Kessel 5-10 Minuten ruhig stehen gelassen.

Zur Quantifizierung von DDS (C12-Di-Carbonsäure), DDSME (C12-Di-Carbonsäure-Metylester), LS (Laurinsäure), LSME (Laurinsäure-Methylester), HLS (omega-Hydroxy-Laurinsäure), HLSME(omega-Hydroxy-Laurinsäure-Methylester), OLS (omega-Oxo-Laurinsäure), OLSME OLS (omega-Oxo-Laurinsäure-Methylester), ALS (omega-Amino-Laurinsäure)und ALSME (omega-Amino-Laurinsäure-Methylester) in Fermentationsproben wurden während der Kultivierung Proben entnommen. Diese Proben wurden für die Analytik vorbereitet. (siehe LC-ESI/MS²-basierte Quantifizierung von Produkten).

### LC-ESI/MS²-basierte Quantifizierung von Produkten

Die Quantifizierung von ALS, ALSME, DDS, DDSME, LS, LSME, HLS, HLSME, OLS und OLSME in Fermentationsproben erfolgte mittels LC-ESI/MS² anhand einer externen Kalibrierung für alle Analyten (0,1 - 50 mg/L) und unter Verwendung der internen Standards Aminoundekansäure (AUD für HLS, DDS, OLS, HLSME, OLSME), d4-ALSME (für ALSME), ¹³C-DDSME (für DDSME), d3-LS (für LS) und d3-LSME (für LSME).

Dabei kommen folgende Geräte zum Einsatz:
- HPLC Anlage 1260 (Agilent; Böblingen) mit Autosampler (G1367E), binärer Pumpe (G1312B) und Säulenofen (G1316A)
- Massenspektrometer TripelQuad 6410 (Agilent; Böblingen) mit ESI-Quelle
- HPLC-Säule: Kinetex C18, 100 x 2,1 mm, Partikelgröße: 2,6 µm, Porengröße 100 Å (Phenomenex; Aschaffenburg)
- Vorsäule: KrudKatcher Ultra HPLC In-Line Filter; 0,5 µm Filtertiefe und 0,004 mm Innendurchmesser (Phenomenex; Aschaffenburg)

Die Proben wurden vorbereitet, indem 1900 µL Lösungsmittel (80% (v/v) ACN, 20% bidest. H₂O (v/v), + 0.1% Ameisensäure) und 100 µL Probe in ein 2-mL-Reaktionsgefäß pipettiert wurden. Das Gemisch wurde ca. 10 Sekunden gevortext und anschließend bei ca. 13000 rpm für 5 min zentrifugiert. Der klare Überstand wurde mit einer Pipette entnommen und nach entsprechender Verdünnung mit Diluent (80% (v/v) ACN, 20% bidest. H₂O (v/v), + 0.1% Ameisensäure) analysiert. Zu je 900 µL Probe wurden 100 µL ISTD hinzupipettiert (10 µL bei einem Probenvolumen von 90 µL).

Die HPLC-Trennung erfolgte mit oben genannter Säule bzw. Vorsäule. Das Injektionsvolumen beträgt 0,7 µL, die Säulentemperatur 50°C, die Flussrate 0,6 mL/min. Die mobile Phase besteht aus Eluent A (0,1 %ige (v/v) wässrige Ameisensäure) und Eluent B (Acetonitril mit 0,1 % (v/v) Ameisensäure). Folgendes Gradientenprofil wurde genutzt:

| **Zeit [min]** | **Eluent A [%]** | **Eluent B [%]** |
|---|---|---|
| 0 | 77 | 23 |
| 0.3 | 77 | 23 |
| 0.4 | 40 | 60 |
| 2.5 | 40 | 60 |
| 2.6 | 2 | 98 |
| 5.5 | 2 | 98 |
| 5.6 | 77 | 23 |
| 9 | 77 | 23 |

Die ESI-MS²-Analyse erfolgte im positiven Modus mit folgenden Parametern der ESI-Quelle:
- Gastemperatur 280°C
- Gasfluss 11 L/min
- Nebulizerdruck 50 psi
- Kapillarspannung 4000 V

Die Detektion und Quantifizierung der Verbindungen ALS, ALSME, DDS, DDSME, HLS, HLSME, OLS, OLSME erfolgte mit den folgenden MRM Parametern, wobei jeweils ein Produkt-Ion als *Qualifier* und eines als *Quantifier* genutzt wurde:

| **Analyt** | **Vorläufer-Ion [m/z]** | **Produkt-Ion [m/z]** | **Verweilzeit [ms]** | **Kollisionsenergie [eV]** |
|---|---|---|---|---|
| DDSME | 245,2 | 167,1 | 25 | 6 |
| DDSME | 245,2 | 149,1 | 50 | 8 |
| HLSME | 231,3 | 181,2 | 15 | 2 |
| HLSME | 231,3 | 163,2 | 25 | 5 |
| DDS | 231,2 | 213,2 | 50 | 0 |
| DDS | 231,2 | 149,1 | 25 | 9 |
| ALSME | 230,3 | 198,1 | 25 | 10 |
| ALSME | 230,3 | 163,2 | 15 | 10 |
| OLSME | 229,2 | 197,2 | 50 | 0 |
| OLSME | 229,2 | 161,1 | 25 | 5 |
| HLS | 217,2 | 181,2 | 35 | 0 |
| HLS | 217,2 | 163,1 | 20 | 4 |
| OLS | 215,2 | 161,2 | 25 | 0 |
| OLS | 215,2 | 95,2 | 60 | 13 |

Die Analyten LS und LSME wurden im SIM Modus detektiert (*m*/*z* 201 und 215).

### Ergebnisse

### Reduzierte Bildung der freien Säuren Aminolaurinsäure, Dodecandisäure und Laurinsäure nach Knockout von bioH in E. coli W3110

Der Stamm mit *bioH-Knockout* zeigte eine deutlich reduzierte Bildung der freien Säuren Aminolaurinsäure, Dodecandisäure und Laurinsäure im Vergleich zum Kontrollstamm mit intaktem *bioH.* Berechnet wurden die Verhältnisse der Absolut-Endtiter von Dodecandisäure (DDS) und Dodecandisäuremethylester (DDSME), Aminolaurinsäure (ALS) und Aminolaurinsäuremethylester (ALSME) sowie Laurinsäure (LS) und Laurinsäuremethylester (LSME) ausgedrückt in Prozent.

| | Endtiter in g/L | | | | | |
|---|---|---|---|---|---|---|
| | ALS | ALSME | DDS | DDSME | LS | LSME |
| W3110 | 0.12 | 11.77 | 5.02 | 7.27 | 0.03 | 1.91 |
| W3110 Δ*bioH* | 0.01 | 11.82 | 0.23 | 2.35 | 0.03 | 12.92 |

| | Verhältnis freie Säure / Methylester in Prozent | | |
|---|---|---|---|
| | ALS/ALSME | DDS/DDSME | LS/LSME |
| W3110 | 0.98 | 69.12 | 1.79 |
| W3110 Δ*bioH* | 0.10 | 9.74 | 0.24 |

| | Verhältnis freie Säure / Methylester in Prozent | | |
|---|---|---|---|
| | ALS/ALSME | DDS/DDSME | LS/LSME |
| W3110 zu W3110 Δ*bioH* | 9.75 | 7.10 | 7.57 |

Der Effekt des *bioH-Knockouts* auf die Bildung von freien Säuren wurde deutlich sichtbar und bewegte sich zwischen einer Reduktion um den Faktor 7,10 (DDS/DDSME) bis hin zu einem Faktor 9,75 (ALS/ALSME).

### Verbessertes Produkt-zu-Nebenprodukt-Verhältnis nach Knockout von bioH in E. coli W3110

Bewertet wurden die nach einer festen Zeit (= Ende der Biotransformation) erreichten Absoluttiter. Hier zeigte sich im Stammhintergrund W3110 ΔbioH ein deutlich verbessertes Verhältnis von Produkten (ALS und ALSME) zu Hauptnebenprodukten (DDS und DDSME) bei gleich bleibendem Endprodukttiter. Das Verhältnis stieg von 49,17 % ALS(ME) zu 82,10 % ALS(ME).

| | Konzentration in g/L | | |
|---|---|---|---|
| | ALS + ALSME | DDS + DDSME | Verhältnis ALS(ME) zu ALS(ME) + DDS(ME) in Prozent |
| W3110 | 11.89 | 12.29 | 49,17 |
| W3110 Δ*bioH* | 11.83 | 2.58 | 82,10 |

### Beispiel 3

### Produktion von Aminolaurinsäuremethylester durch einen E. coli-Stamm mit einer Deletion im Gen bioH durch Einsatz eines Expressionsvektors für die Gene ald aus Bacillus subtilis und Cv_2025 aus Chromobacterium violaceum und den Genen alkB, alkG, alkT und alkL aus dem alk-Operon von Pseudomonas putida.

Zur Erzeugung der *E*. *coli*-Stämme mit einem Expressionsvektor für die Gene *ald* aus *Bacillus subtilis* (kodierend für eine Alanin-Dehydrogenase, codon-optimiert für *E. coli*), *Cv_2025* aus *Chromobacterium violaceum* (kodierend für eine Transaminase, codon-optimiert für *C*. *tropicalis*), *alkB, alkG, alkT* (kodierend für eine Alkan-Monooxygenase, ein Rubredoxin und eine Rubredoxin-Reduktase) und *alkL* (kodierend für ein Membran-/Transportprotein) aus dem alk-Operon von *Pseudomonas putida* wurden elektrokompetente Zellen von *E. coli* BW25113 ΔbioH sowie dem entsprechenden Kontrollstamm *E. coli* BW25113 hergestellt. Dies geschah in einer dem Fachmann bekannten Art und Weise. Die Stämme stammen aus der kommerziell erhältlichen Keio-Collection. Diese wurden mit dem Plasmid pACYC184{MCS2.0}[alkST_BFGL][alaDH_Bs(co) {PspXI} TAcv(ct)] (SEQ ID 13 bzw. Beispiel 1 der WO/2013/024114 und die dort aufgeführte SEQ ID Nr. 17) transformiert und auf LB-Agar-Platten mit Chloramphenicol (50 µg/ml) ausplattiert. Transformanten wurden durch Plasmidpräparation und analytische Restriktionsanalyse bezüglich der Anwesenheit der korrekten Plasmide überprüft. Auf diese Weise wurden folgende Stämme konstruiert:

| Stammhintergrund | Vorhandene Plasmide |
|---|---|
| BW25113 | pACYC184{MCS2.0}[alkST_BFGL][alaDH_Bs(co) {PspXI} TAcv(ct)] |
| BW25113 Δ*bioH* | pACYC184{MCS2.0}[alkST_BFGL][alaDH_Bs(co) {PspXI} TAcv(ct)] |

Die Stämme wurden einer *fed-batch*-Fermentation unterzogen, um die Fähigkeit zur Produktion von Hydroxy-Laurinsäuremethylester, Oxo-Laurinsäuremethylester, Carboxy-Laurinsäuremethylester und Amino-Laurinsäuremethylester aus Laurinsäuremethylester zu analysieren. Dies wurde mit einem 8-fach Parallelfermentationssystem der Firma DASGIP durchgeführt. Die weitere experimentelle Durchführung fand exakt wie in Beispiel 2 beschrieben statt.

### Ergebnisse

### Reduzierte Bildung der freien Säuren Aminolaurinsäure, Dodecandisäure, Laurinsäure und Hydroxylaurinsäure nach Knockout von bioH in E. coli BW25113

Der Stamm mit *bioH-Knockout* zeigte eine deutlich reduzierte Bildung der freien Säuren Aminolaurinsäure, Dodecandisäure, Laurinsäure und Hydroxylaurinsäure im Vergleich zum Kontrollstamm mit intaktem *bioH*, teils unterhalb der Nachweisgrenze. Berechnet wurden die Verhältnisse der Absolut-Endtiter von Dodecandisäure (DDS) und Dodecandisäuremethylester (DDSME), Aminolaurinsäure (ALS) und Aminolaurinsäuremethylester (ALSME), Hydroxylaurinsäure (HLS) und Hydroxylaurinsäuremethylester (HLSME) sowie Laurinsäure (LS) und Laurinsäuremethylester (LSME) ausgedrückt in Prozent.

| | Titer in g/L | | | | | | | |
|---|---|---|---|---|---|---|---|---|
| | ALS | ALSME | DDS | DDSME | HLS | HLSME | LS | LSME |
| BW25113 | 0.16 | 10.80 | 2.75 | 1.40 | 0.72 | 15.01 | 0.48 | 40.77 |
| BW25113 Δ*bioH* | n.n. | 15.86 | 0.23 | 3.95 | n.n. | 12.32 | n.n. | 28.49 |

Einzig mathematisch darstellbares Verhältnis ist DDS zu DDSME:

| | DDS/DDSME in Prozent |
|---|---|
| BW25113 | 196.94 |
| BW25113 Δ*bioH* | 5.87 |

Der *bioH-Knockout* reduzierte die Bildung von DDS um den Faktor 33,5, alle weiteren freien Säuren liegen unterhalb der unteren Nachweisgrenze.

### Verlängerte Oxidationsphase nach Knockout von bioH in E. coli BW25113

Im Vergleich zum Kontrollstamm zeigte sich, dass die initiale Oxidationsleistung im *bioH-Knockout*-Stamm nicht an Steigung verliert, sondern in Bezug auf die ALSME-Bildung über den Prozessverlauf nahezu konstant bleibt.

| | Prozesszeit [h] | ALSME [g/L] |
|---|---|---|
| BW25113 | 1 | 0.26 |
| | 2 | 1.16 |
| | 3 | 2.35 |
| | 19 | 10.51 |
| | 21.25 | 10.80 |
| BW25113 Δ*bioH* | 1 | 0.38 |
| | 2 | 1.27 |
| | 3 | 2.48 |
| | 19 | 14.94 |
| | 21.25 | 15.86 |

Abbildung 1 zeigt: Der *Knockout* von *bioH* bewirkte bezogen auf den Endtiter bei gleich bleibenden Bedingungen eine um 47 % erhöhte Produktivität.

### Gesteigerte Bildungsrate von Aminolaurinsäuremethylester nach Knockout von bioH in E. coli BW25113

Bewertet wurden die nach einer festen Zeit (= Ende der Biotransformation) erreichten Absoluttiter. Hier zeigte sich im *Knockout*-Stamm eine deutlich höhere Produktbildungsrate von 0,75 g ALSME pro Liter und Stunde gegenüber 0,51 g ALSME pro Liter und Stunde im Wildtyp-Stamm.

| | Prozesszeit [h] | ALSME [g/L] |
|---|---|---|
| BW25113 | 21.25 | 10.80 |
| BW25113 Δ*bioH* | 21.25 | 15.86 |

### Verbessertes Produkt-zu-Nebenprodukt-Verhältnis nach Knockout von bioH in E. coli BW25113

Bewertet wurden die nach einer festen Zeit (= Ende der Biotransformation) erreichten Absoluttiter. Hier zeigte sich im *Knockout*-Stammhintergrund ein deutlich verbessertes Verhältnis von Produkt (ALS und ALSME) zum Hauptnebenprodukt (DDS und DDSME).

| | Titer in g/L | | | |
|---|---|---|---|---|
| | ALS | ALSME | DDS | DDSME |
| BW25113 | 0.16 | 10.80 | 2.75 | 1.40 |
| BW25113 Δ*bioH* | 0.00 | 15.86 | 0.23 | 3.95 |

| | ALS + ALSME [g/L] | DDS + DDSME [g/L] |
|---|---|---|
| BW25113 | 10.96 | 4.15 |
| BW25113 Δ*bioH* | 15.86 | 4.18 |

| | ALS(ME)/DDS(ME) |
|---|---|
| BW25113 | 2.64 |
| BW25113 Δ*bioH* | 3.80 |

Das Verhältnis stieg durch *bioH-Knockout* um 43,9 %. Der Produkttiter stieg um 43,9 % bei gleich bleibendem Nebenprodukt-Titer.

### Gesteigerter Glucose-Ausbeutekoeffizient (Y_{P/S}) bei der Produktion von Aminolaurinsäure und Aminolaurinsäuremethylester nach Knockout von bioH in E. coli BW25113

Betrachtet wurden die finalen kumulierten Absoluttiter von Aminolaurinsäure und Aminolaurinsäuremethylester im Vergleich zur eingesetzten Menge an Glucose.

| | ALS + ALSME [g/L] | Glucoseverbrauch [g] | g/L ALS(ME) pro g Glucose |
|---|---|---|---|
| BW25113 | 10,96 | 52,95 | 0.21 |
| BW25113 Δ*bioH* | 15,86 | 52,55 | 0.30 |

Der Glucose-Ausbeutekoeffizient stieg von 0,21 g/L ALS(ME) pro Gramm Glucose um 46 % auf 0,30 g/L ALS(ME) pro Gramm Glucose bedingt durch den *bioH-Knockout.*

## Patentansprüche

1. Verfahren zur Umsetzung eines Carbonsäureesters der Formel (I)
R¹ - A - COOR² (I),
wobei R¹ aus der Gruppe ausgewählt ist, die aus Wasserstoff, -CH₂OH, -CHO, -COOR³, -CH₂SH, - CH₂OR³ und -CH₂NH₂ besteht,
wobei R² aus der Gruppe ausgewählt ist, die aus Alkyl, bevorzugt Methyl, Ethyl und Propyl, besteht,
wobei R³ aus der Gruppe ausgewählt ist, die aus Wasserstoff und Alkyl, bevorzugt Methyl, Ethyl und Propyl, besteht, und wobei es sich bei A um einen nicht substituierten, linearen, Alkylen- oder Alkenylenrest mit wenigstens 4, bevorzugter 6, noch bevorzugter 8 Kohlenstoffatomen handelt,
mittels einer Zelle, umfassend den Schritt
a) Kontaktieren der Zelle mit dem Carbonsäureester in einer wässrigen Lösung,
wobei es sich bei der Zelle um eine rekombinante Zelle handelt, bei der die Aktivität eines Polypeptides umfassend SEQ ID NO 2 oder eine Variante davon, wobei die Variante im Wesentlichen die gleiche enzymatische Aktivität des Polypeptides umfassend SEQ ID NO 2 aufweist und die Variante eine Homologie mit SEQ ID NO 2 von mindestens 90% aufweist, gegenüber dem Wildtyp der Zelle verringert ist.

2. Verfahren nach Anspruch 1, weiter umfassend den Schritt
b) Kontaktieren der wässrigen Lösung mit einer hydrophoben organischen Lösung umfassend einen Kationenaustauscher.

3. Verwendung eines Knockouts eines für ein Polypeptid umfassend SEQ ID NO 2 oder eine Variante davon kodierenden Gens als Teil der genetischen Ausstattung einer rekombinanten Zelle zur Erhöhung der Produktion eines Carbonsäureesters der Formel (I)
R¹ - A - COOR² (I),
wobei R¹ aus der Gruppe ausgewählt ist, die aus Wasserstoff, -CH₂OH, -CHO, -COOR³, -CH₂SH, - CH₂OR³ und -CH₂NH₂ besteht,
wobei R² aus der Gruppe ausgewählt ist, die aus Alkyl, bevorzugt Methyl, Ethyl und Propyl, besteht,
wobei R³ aus der Gruppe ausgewählt ist, die aus Wasserstoff und Alkyl, bevorzugt Methyl, Ethyl und Propyl, besteht,
wobei es sich bei A um einen nicht substituierten, linearen Alkylen- oder Alkenylenrest mit wenigstens 4, bevorzugter 6, noch bevorzugter 8 Kohlenstoffatomen handelt,
und wobei die Variante im Wesentlichen die gleiche enzymatische Aktivität des Polypeptides umfassend SEQ ID NO 2 aufweist und die Variante eine Homologie mit SEQ ID NO 2 von mindestens 90% aufweist,

4. Verwendung einer rekombinanten Zelle, bei der die Aktivität eines Polypeptides umfassend SEQ ID NO 2 oder eine Variante davon, wobei die Variante im Wesentlichen die gleiche enzymatische Aktivität des Polypeptides umfassend SEQ ID NO 2 aufweist, und die Variante eine Homologie mit SEQ ID NO 2 von mindestens 90% aufweist, gegenüber dem Wildtyp der Zelle verringert ist, zur Umsetzung eines Carbonsäureesters der Formel (I)
R¹ - A - COOR² (I),
wobei R¹ aus der Gruppe ausgewählt ist, die aus Wasserstoff, -CH₂OH, -CHO, -COOR³, -CH₂SH, - CH₂OR³ und -CH₂NH₂ besteht,
wobei R² aus der Gruppe ausgewählt ist, die aus Alkyl, bevorzugt Methyl, Ethyl und Propyl, besteht,
wobei R³ aus der Gruppe ausgewählt ist, die aus Wasserstoff und Alkyl, bevorzugt Methyl, Ethyl und Propyl, besteht,
und wobei es sich bei A um einennicht substituierten, linearen, Alkylen- oder Alkenylenrest mit wenigstens 4, bevorzugter 6, noch bevorzugter 8 Kohlenstoffatomen, handelt.

5. Verfahren oder Verwendung nach einem der Ansprüche 1 bis 4, wobei es sich bei A um einen gesättigten Alkylenrest handelt, bevorzugt um einen Alkylenrest der Formel -(CH₂)ₙ-, wobei n wenigstens 4 ist.

6. Verfahren nach einem der Ansprüche 1 bis 2, wobei R¹ aus der Gruppe ausgewählt ist, die aus Wasserstoff, -CH₂OH, -CHO und -CH₂NH₂ besteht.

7. Verfahren oder Verwendung nach einem der Ansprüche 1 bis 6, wobei die Zelle weiterhin eine Transaminase exprimiert.

8. Verfahren oder Verwendung nach einem der Ansprüche 1 bis 7, wobei die Zelle weiterhin eine Alanindehydrogenase exprimiert.

9. Verfahren oder Verwendung nach einem der Ansprüche 1 bis 8, wobei die Zelle weiterhin ein Protein aus der AlkL-Familie aufweist.

10. Verfahren oder Verwendung nach einem der Ansprüche 1 bis 9, wobei die Zelle eine ihrem Wildtyp gegenüber verringerte Aktivität wenigstens eines Enzyms aufweist, das eine der Reaktionen der β-Oxidation von Fettsäuren katalysiert, wobei es sich bevorzugt um ein Enzym aus der Gruppe handelt, die Fettsäure-CoA-Ligase, Acyl-CoA-Dehydrogenase, 2,4-Dienoyl-CoA-Reduktase, Enoyl-CoA-Hydratase , 3-Ketoacyl-CoA-Thiolase, einen Fettsäureimporter oder Varianten davon, wobei die Varianten im Wesentlichen die gleiche enzymatische Aktivität der Fettsäure-CoA-Ligase, der Acyl-CoA-Dehydrogenase, der 2,4-Dienoyl-CoA-Reduktase, der Enoyl-CoA-Hydratase, der 3-Ketoacyl-CoA-Thiolase oder des Fettsäureimporters aufweisen, umfasst, besonders bevorzugt um FadL oder eine Variante davon, wobei die Variante im Wesentlichen die gleiche enzymatische Aktivität des FadL aufweist.

11. Verfahren oder Verwendung nach einem der Ansprüche 1 bis 10, wobei die Zelle wenigstens ein Enzym aus der Gruppe bestehend aus Alkanhydroxylase, Alkoholdehydrogenase, Transaminase, Alanindehydrogenase und ein Protein aus der AlkL-Familie in rekombinanter Form aufweist und/oder überexprimiert.

12. Verfahren oder Verwendung nach einem der Ansprüche 1 bis 11, wobei die Aktivität eines Polypeptides umfassend SEQ ID NO 2 oder eine Variante davon gegenüber dem Wildtyp der Zelle durch Knock out eines für ein Polypeptid umfassend SEQ ID NO 2 oder eine Variante davon kodierenden Gens verringert ist.

## Claims

1. Process for reacting a carboxylic acid ester of the formula (I)
R¹ - A - COOR² (I),
wherein R¹ is selected from the group consisting of hydrogen, -CH₂OH, -CHO, -COOR³, -CH₂SH, -CH₂OR³ and -CH₂NH₂,
wherein R² is selected from the group consisting of alkyl, preferably methyl, ethyl and propyl,
wherein R³ is selected from the group consisting of hydrogen and alkyl, preferably methyl, ethyl and propyl,
and wherein A is an unsubstituted, linear alkylene or alkenylene radical having at least 4, more preferably 6, even more preferably 8, carbons,
by means of a cell, comprising the step of
a) contacting the cell with said carboxylic acid ester in an aqueous solution,
wherein the cell is a recombinant cell which has reduced activity of a polypeptide comprising SEQ ID NO 2 or a variant thereof over the wild-type cell, wherein the variant has essentially the same enzymatic activity of the polypeptide comprising SEQ ID NO 2 and the variant has a homology with SEQ ID NO 2 of at least 90%.

2. Process according to Claim 1, further comprising the step of
b) contacting the aqueous solution with a hydrophobic organic solution comprising a cation exchanger.

3. Use of a knockout of a gene coding for a polypeptide comprising SEQ ID NO 2 or a variant thereof as part of the genetic make-up of a recombinant cell for increasing production of a carboxylic acid ester of the formula (I)
R¹ - A - COOR² (I),
wherein R¹ is selected from the group consisting of hydrogen, -CH₂OH, -CHO, -COOR³, -CH₂SH, -CH₂OR³ and -CH₂NH₂,
wherein R² is selected from the group consisting of alkyl, preferably methyl, ethyl and propyl,
wherein R³ is selected from the group consisting of hydrogen and alkyl, preferably methyl, ethyl and propyl,
wherein A is an unsubstituted, linear alkylene or alkenylene radical having at least 4, more preferably 6, even more preferably 8, carbons,
and wherein the variant has essentially the same enzymatic activity of the polypeptide comprising SEQ ID NO 2 and the variant has a homology with SEQ ID NO 2 of at least 90%.

4. Use of a recombinant cell which has reduced activity of a polypeptide comprising SEQ ID NO 2 or a variant thereof over the wild-type cell, wherein the variant has essentially the same enzymatic activity of the polypeptide comprising SEQ ID NO 2 and the variant has a homology with SEQ ID NO 2 of at least 90%, for reacting a carboxylic acid ester of the formula (I)
R¹ - A - COOR² (I),
wherein R¹ is selected from the group consisting of hydrogen, -CH₂OH, -CHO, -COOR³, -CH₂SH, -CH₂OR³ and -CH₂NH₂,
wherein R² is selected from the group consisting of hydrogen and alkyl, preferably methyl, ethyl and propyl,
wherein R³ is selected from the group consisting of hydrogen and alkyl, preferably methyl, ethyl and propyl,
and wherein A is an unsubstituted, linear alkylene or alkenylene radical having at least 4, more preferably 6, even more preferably 8, carbons.

5. Process or use according to any of Claims 1 to 4, wherein A is a saturated alkylene radical, preferably an alkylene radical of the formula -(CH₂)ₙ-, where n is at least 4.

6. Process according to either of Claims 1 and 2, wherein R¹ is selected from the group consisting of hydrogen, -CH₂OH, -CHO and -CH₂NH₂.

7. Process or use according to any of Claims 1 to 6, wherein the cell furthermore expresses a transaminase.

8. Process or use according to any of Claims 1 to 7, wherein the cell furthermore expresses an alanine dehydrogenase.

9. Process or use according to any of Claims 1 to 8, wherein the cell furthermore has a protein of the AlkL family.

10. Process or use according to any of Claims 1 to 9, wherein the cell has an activity, which is reduced over its wild type, of at least one enzyme catalysing any of the reactions of fatty acid β-oxidation, wherein the enzyme is preferably one from the group comprising fatty acid-CoA ligase, acyl-CoA dehydrogenase, 2,4-dienoyl-CoA reductase, enoyl-CoA hydratase, 3-ketoacyl-CoA thiolase, a fatty acid importer or variants thereof, wherein the variants have essentially the same enzymatic activity of the fatty acid-CoA ligase, the acyl-CoA dehydrogenase, the 2,4-dienolyl-CoA reductase, the enoyl-CoA hydratase, the keto-CoA thiolase or the fatty acid importer, particularly preferably FadL or a variant thereof, wherein the variant has essentially the same enzymatic activity of the FadL.

11. Process or use according to any of Claims 1 to 10, wherein the cell has and/or overexpresses at least one enzyme from the group consisting of alkane hydroxylase, alcohol dehydrogenase, transaminase, alanine dehydrogenase and a protein of the AlkL family in recombinant form.

12. Process or use according to any of Claims 1 to 11, wherein the activity of a polypeptide comprising SEQ ID NO 2 or a variant thereof is reduced over the wild-type cell due to knockout of a gene coding for a polypeptide comprising SEQ ID NO 2 or a variant thereof.

## Revendications

1. Procédé de mise en réaction d'un ester d'acide carboxylique de formule (I)
R¹-A-COOR² (I)
dans laquelle R¹ est choisi dans le groupe constitué par hydrogène, -CH₂OH, -CHO, -COOR³, -CH₂SH, -CH₂OR³ et-CH₂NH₂,
R² est choisi dans le groupe constitué par alkyle, de préférence méthyle, éthyle et propyle,
R³ est choisi dans le groupe constitué par hydrogène et alkyle, de préférence méthyle, éthyle et propyle,
et A est un radical alkylène ou alcénylène linéaire non substitué contenant au moins 4, de préférence 6, de manière encore davantage préférée 8 atomes de carbone, au moyen d'une cellule, comprenant l'étape suivante :
a) la mise en contact de la cellule avec l'ester d'acide carboxylique dans une solution aqueuse,
la cellule étant une cellule recombinante dans laquelle l'activité d'un polypeptide comprenant SEQ ID NO 2 ou d'une variante de celui-ci, la variante présentant essentiellement la même activité enzymatique que le polypeptide comprenant SEQ ID NO 2 et la variante présentant une homologie avec SEQ ID NO 2 d'au moins 90 %, est réduite par rapport au type sauvage de la cellule.

2. Procédé selon la revendication 1, comprenant en outre l'étape suivante :
b) la mise en contact de la solution aqueuse avec une solution organique hydrophobe comprenant un échangeur de cations.

3. Utilisation d'une invalidation d'un gène codant pour un polypeptide comprenant SEQ ID NO 2 ou une variante de celui-ci en tant que partie de l'équipement génétique d'une cellule recombinante pour augmenter la production d'un ester d'acide carboxylique de formule (I)
R¹-A-COOR² (I)
dans laquelle R¹ est choisi dans le groupe constitué par hydrogène, -CH₂OH, -CHO, -COOR³, -CH₂SH, -CH₂OR³ et-CH₂NH₂,
R² est choisi dans le groupe constitué par alkyle, de préférence méthyle, éthyle et propyle,
R³ est choisi dans le groupe constitué par hydrogène et alkyle, de préférence méthyle, éthyle et propyle,
et A est un radical alkylène ou alcénylène linéaire non substitué contenant au moins 4, de préférence 6, de manière encore davantage préférée 8 atomes de carbone, la variante présentant essentiellement la même activité enzymatique que le polypeptide comprenant SEQ ID NO 2 et la variante présentant une homologie avec SEQ ID NO 2 d'au moins 90 %.

4. Utilisation d'une cellule recombinante, dans laquelle l'activité d'un polypeptide comprenant SEQ ID NO 2 ou d'une variante de celui-ci, la variante présentant essentiellement la même activité enzymatique que le polypeptide comprenant SEQ ID NO 2 et la variante présentant une homologie avec SEQ ID NO 2 d'au moins 90 %, est réduite par rapport au type sauvage de la cellule, pour la mise en réaction d'un ester d'acide carboxylique de formule (I)
R¹-A-COOR² (I)
dans laquelle R¹ est choisi dans le groupe constitué par hydrogène, -CH₂OH, -CHO, -COOR³, -CH₂SH, -CH₂OR³ et-CH₂NH₂,
R² est choisi dans le groupe constitué par alkyle, de préférence méthyle, éthyle et propyle,
R³ est choisi dans le groupe constitué par hydrogène et alkyle, de préférence méthyle, éthyle et propyle,
et A est un radical alkylène ou alcénylène linéaire non substitué contenant au moins 4, de préférence 6, de manière encore davantage préférée 8 atomes de carbone.

5. Procédé ou utilisation selon l'une quelconque des revendications 1 à 4, dans lequel ou dans laquelle A est un radical alkylène saturé, de préférence un radical alkylène de formule -(CH₂)ₙ-, n valant au moins 4.

6. Procédé selon l'une quelconque des revendications 1 à 2, dans lequel R¹ est choisi dans le groupe constitué par hydrogène, -CH₂OH, -CHO et -CH₂NH₂.

7. Procédé ou utilisation selon l'une quelconque des revendications 1 à 6, dans lequel ou dans laquelle la cellule exprime en outre une transaminase.

8. Procédé ou utilisation selon l'une quelconque des revendications 1 à 7, dans lequel ou dans laquelle la cellule exprime en outre une alanine-déshydrogénase.

9. Procédé ou utilisation selon l'une quelconque des revendications 1 à 8, dans lequel ou dans laquelle la cellule comprend en outre une protéine de la famille AlkL.

10. Procédé ou utilisation selon l'une quelconque des revendications 1 à 9, dans lequel ou dans laquelle la cellule présente une activité réduite par rapport à son type sauvage d'au moins une enzyme qui catalyse une des réactions de l'oxydation β d'acides gras, celle-ci étant de préférence une enzyme du groupe comprenant l'acide gras-CoA-ligase, l'acyl-CoA-déshydrogénase, la 2,4-diénoyl-CoA-réductase, l'énoyl-CoA-hydratase, la 3-cétoacyl-CoA-thiolase, un importateur d'acides gras ou des variantes de celles-ci, les variantes présentant essentiellement la même activité enzymatique que l'acide gras-CoA-ligase, l'acyl-CoA-déshydrogénase, la 2,4-diénoyl-CoA-réductase, l'énoyl-CoA-hydratase, la 3-cétoacyl-CoA-thiolase ou l'importateur d'acides gras, de manière particulièrement préférée la FadL ou une variante de celle-ci, la variante présentant essentiellement la même activité enzymatique que la FadL.

11. Procédé ou utilisation selon l'une quelconque des revendications 1 à 10, dans lequel ou dans laquelle la cellule comprend et/ou surexprime au moins une enzyme du groupe constitué par une alcane-hydroxylase, une alcool-déshydrogénase, une transaminase, une alanine-déshydrogénase et une protéine de la famille AlkL sous forme recombinante.

12. Procédé ou utilisation selon l'une quelconque des revendications 1 à 11, dans lequel ou dans laquelle l'activité d'un polypeptide comprenant SEQ ID NO 2 ou d'une variante de celui-ci est réduite par rapport au type sauvage de la cellule par invalidation d'un gène codant pour un polypeptide comprenant SEQ ID NO 2 ou une variante de celui-ci.
